# EUROPEAN PATENT SPECIFICATION

(11) **EP 3 423 448 B1**
(45) Date of publication and mention of the grant of the patent: **04.12.2024**
(21) Application number: 17711443.6
(22) Date of filing: 01.03.2017
(51) Int. Cl.: C07D 403/12, C07D 231/06, C07D 401/12, C07D 409/12, C07D 413/12, A61K 31/4155, A61K 31/415

(54) **CANNABINOID RECEPTOR MEDIATING COMPOUNDS**
CANNABINOID-REZEPTORVERMITTELNDE VERBINDUNGEN
COMPOSÉS DE MÉDIATION DES RÉCEPTEURS CANNABINOÏDES

(30) Priority: 04.03.2016 US 201615061829
(43) Date of publication of application: 09.01.2019
(73) Proprietor: The U.S.A. as represented by the Secretary, Department of Health and Human Services, Bethesda, MD 20852-7660 (US)
(72) Inventor: CINAR, Resat, Bethesda MD 20892-9413 (US); KUNOS, George, Rockville MD 20852 (US); IYER, Malliga, R., Bethesda MD 20892-9413 (US); GAHL, William, A., Bethesda MD 20892-1851 (US); GOCHUICO, Bernadette, R., Bethesda MD 20892-1851 (US); MALICDAN, May, Christine, Vergara, Bethesda MD 20814 (US)
(74) Representative: HGF
(86) International application number: PCT/US2017/020250
(87) International publication number: WO 2017/151802

(56) References cited:
- WO-A1-03/026647
- WO-A1-2014/078309
- WO-A1-2016/196646
- LANGE J H M ET AL: "SYNTHESIS, BIOLOGICAL PROPERTIES, AND MOLECULAR MODELING INVESTIGATIONS OF NOVEL 3,4-DIARYLPYRAZOLINES AS POTENT AND SELECTIVE CB1 CANNABINOID RECEPTOR ANTAGONISTS", JOURNAL OF MEDICINAL CHEMISTRY, AMERICAN CHEMICAL SOCIETY, vol. 47, no. 3, 1 January 2004 (2004-01-01), pages 627 - 643, XP001188902, ISSN: 0022-2623, DOI: 10.1021/JM031019Q
- MALLIGA R. IYER ET AL: "Design, Synthesis, and Biological Evaluation of Novel, Non-Brain-Penetrant, Hybrid Cannabinoid CB 1 R Inverse Agonist/Inducible Nitric Oxide Synthase (iNOS) Inhibitors for the Treatment of Liver Fibrosis", JOURNAL OF MEDICINAL CHEMISTRY, vol. 60, no. 3, 13 January 2017 (2017-01-13), pages 1126 - 1141, XP055368197, ISSN: 0022-2623, DOI: 10.1021/acs.jmedchem.6b01504

## Description

### CROSS REFERENCE TO RELATED APPLICATION

This application claims the benefit of U.S. Patent Application No. 15/061,829, filed March 4, 2016.

### BACKGROUND

Endocannabinoids are lipid signaling molecules that act on the same cannabinoid receptors - CB₁ and CB₂ - that recognize and mediate the effects of marijuana. Activation of CB₁ receptors increases appetite, increases the biosynthesis and storage of lipids, inhibits the actions of insulin and leptin, and promotes inflammation and fibrosis, which has led to the development of CB₁ receptor blocking drugs for the treatment of obesity and its metabolic complications, referred to as the metabolic syndrome. The prototype compound rimonabant proved effective in the treatment of the metabolic syndrome, but caused neuropsychiatric side effects, which resulted in its withdrawal from the market and halted further therapeutic development of this class of compounds.

Hermansky-Pudlak Syndrome (HPS) is a rare genetic disorder associated with high susceptibility to develop pulmonary fibrosis (PF). In the absence of currently available FDA-approved therapy for HPSPF, there is an urgent need for identifying new therapeutic targets and treatment strategies.

WO2014/078309A1 and Lange et al (JOURNAL OF MEDICINAL CHEMISTRY, vol. 47, no. 3, 2004) disclose CB Icannabinoid receptor antagonists which share the same basic pharmacophore with the presently claimed compounds. These compounds however are not known to be used in the treatment in pulmonary fibrosis.

### SUMMARY

In one embodiment, provided herein is a compound, or a tautomer thereof, or a pharmaceutically acceptable salt or ester thereof, for use in the treatment of pulmonary fibrosis in a subject, wherein the compound is of the formula: 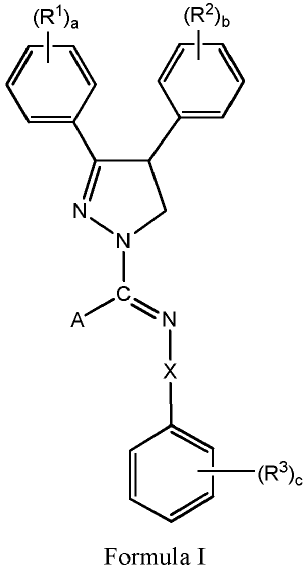 wherein
X is SO₂;
b is zero;
a and c are each independently 0, 1, 2, 3, 4 or 5;
R¹ and R³ are each independently selected from C₁-C₆ alkyl, substituted C₁-C₆ alkyl, halogen, C₁-C₈ alkoxy and substituted C₁-C₈ alkoxy;
A is an amidino-containing moiety having the structure of: 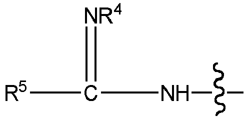
wherein
   R⁴ is H;
   R⁵ is C₁-C₆ alkyl, C₃-C₆ cycloalkyl, -NH₂, -N(C₁-C₆ alkyl)₂, -NH(C₁-C₆ alkyl), phenyl, a monocyclic or bicyclic heteroaryl that includes at least one nitrogen heteroatom, or a monocyclic or bicyclic heterocycloalkyl that includes at least one nitrogen heteroatom; or
   R⁵ is C₁-C₆ alkyl, -N(C₁-C₆ alkyl)₂, -NH(C₁-C₆ alkyl), or -NHC(O)CH₃; or
   R⁵ is an amino of the formula -NR³⁰R³¹, where R³⁰ and R³¹ are, independently, hydrogen or an optionally-substituted C₁-C₆ alkyl, provided that at least one of R³⁰ and R³¹ is optionally substituted C₁-C₆ alkyl; or
   R⁵ is a thiol substituted with a C₁-C₆ alkyl or a substituted C₁-C₆ alkyl;
   wherein substituted C₁-C₆ alkyl is a C₁-C₆ alkyl wherein one of more hydrogen atoms are substituted with halogen, cycloalkyl, C₁-C₈ alkoxy, -NH₂, hydroxyl, aryl, alkenyl with up to 6 carbon atoms or carboxyl; and
   substituted C₁-C₈ alkoxy is C₁-C₈ alkoxy substituted with alkenyl with up to 6 carbon atoms, alkynyl with up to 6 carbon atoms, aryl, aryl-C₁-C₆ alkyl, cycloalkyl, halogenated C₁-C₆ alkyl, or C₁-C₈ alkoxy.

Also provided herein, is a compound or a tautomer thereof, or a pharmaceutically acceptable salt thereof, for use in the treatment of pulmonary fibrosis in a subject, wherein the compound is of the formula: 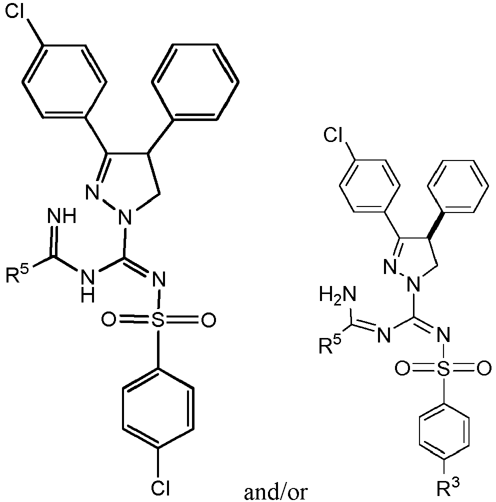 wherein R⁵ is methyl, -S-methyl, -NHC(O)CH₃, NH(Boc), or NH₂; and R³ is CF₃ or Cl.

Also provided herein, is a compound or a tautomer thereof, or a pharmaceutically acceptable salt thereof, for use in the treatment of pulmonary fibrosis in a subject, wherein the compound is of the formula: 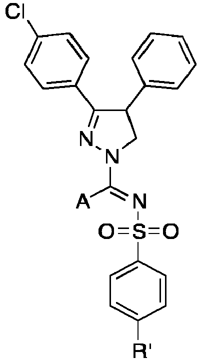 wherein the compound has an A and R' group as indicated in the table below:

| Serial# | A | R' |
|---|---|---|
| 1 | 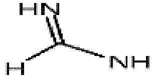 | Cl |
| 2E1 | 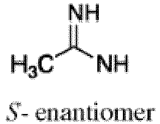 | Cl |
| 2E2 | 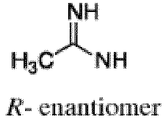 | Cl |
| 3 | 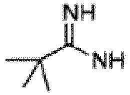 | Cl |
| 3E1 | 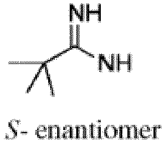 | Cl |
| 4 | 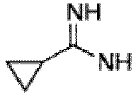 | Cl |
| 5 | 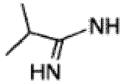 | Cl |
| 6 | 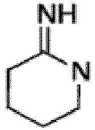 | Cl |
| 7 | 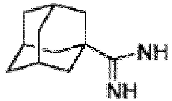 | Cl |
| 8 | 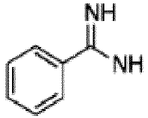 | Cl |
| 9 | 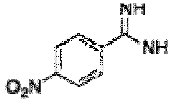 | Cl |
| 10 | 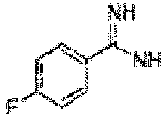 | Cl |
| 10E1 | 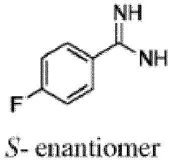 | Cl |
| 10E2 | 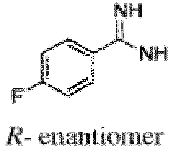 | Cl |
| 11 | 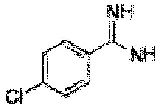 | Cl |
| 11E1 | 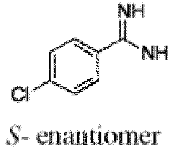 | Cl |
| 11E2 | 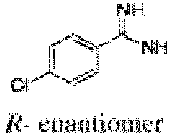 | Cl |
| 12 | 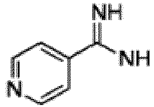 | Cl |
| 13 | 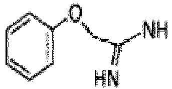 | Cl |
| 14 | 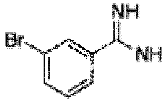 | Cl |
| 15 | 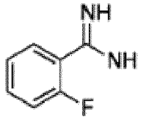 | Cl |
| 16 | 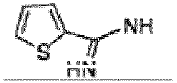 | Cl |
| 17 | 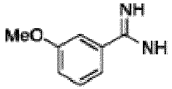 | Cl |
| 18 | 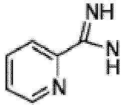 | Cl |
| 19 | 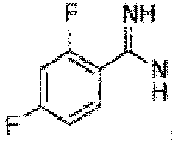 | Cl |
| 20 | 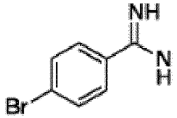 | Cl |
| 21 | 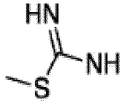 | Cl |
| 22 | 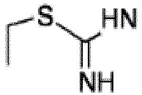 | Cl |
| 23 | 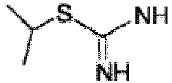 | Cl |
| 24 | 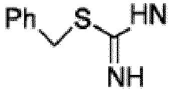 | Cl |
| 25 | 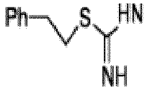 | Cl |
| 26 | 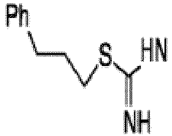 | Cl |
| 27 | 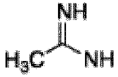 | F |
| 28 | 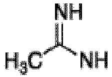 | Br |
| 29 | 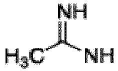 | I |
| 30 | 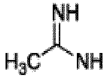 | H |
| 31 | 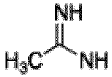 | CH₃ |
| 32 | 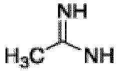 | OCH₃ |
| 33 | 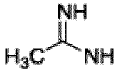 | (C₄H₈)- |
| 34 | 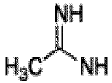 | CF₃ |
| 35 | 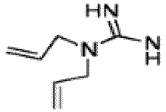 | Cl |
| 36 | 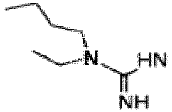 | Cl |
| 37 | 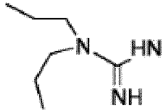 | Cl |
| 38 | 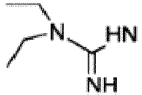 | Cl |
| 39 | 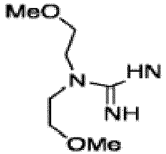 | Cl |
| 40 | 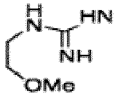 | Cl |
| 41 | 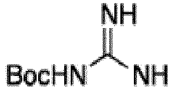 | Cl |
| 42 | 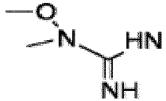 | Cl |
| 43 | 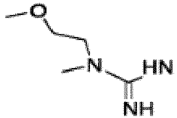 | Cl |
| 44 | 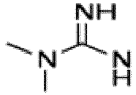 | Cl |
| 45 | 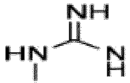 | Cl |
| 46 | 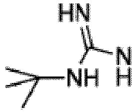 | Cl |
| 47 | 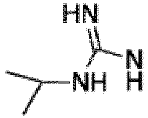 | Cl |
| 48 | 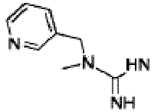 | Cl |
| 49 | 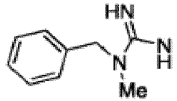 | Cl |
| 50 | 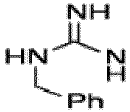 | Cl |
| 51 | 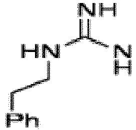 | Cl |
| 52 | 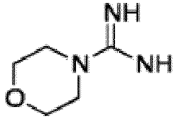 | Cl |
| 53 | 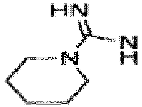 | Cl |
| 54 | 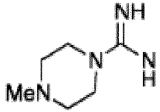 | Cl |
| 55 | 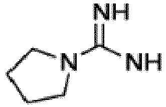 | Cl |
| 56 | 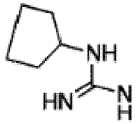 | Cl |
| 57 | 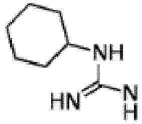 | Cl |
| 58 | 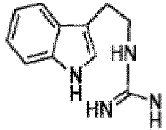 | Cl |
| 59 | 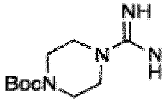 | Cl |
| 60 | 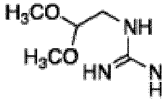 | Cl |
| 61 | 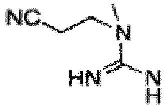 | Cl |
| 62 | 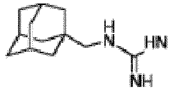 | Cl |
| 63 | 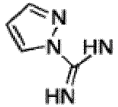 | Cl |
| 64 | 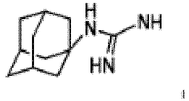 | Cl |
| 66 | 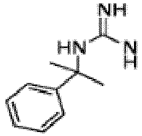 | Cl |
| 67 | 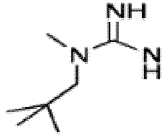 | Cl |
| 68 | 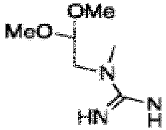 | Cl |
| 69 | 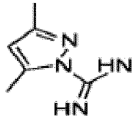 | Cl |
| 70 | 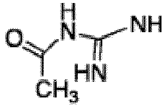 | I |
| 71 | 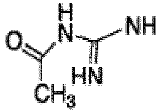 | C₄H₈ |
| 73 | NH₂NH | Cl |

Also disclosed, but not within the literal scope of the claims, is a compound, or a pharmaceutically acceptable salt or ester thereof, having a structure of: 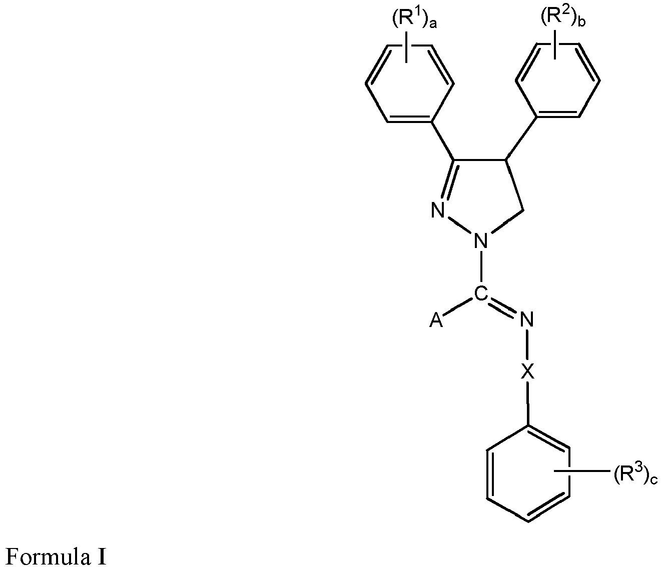 wherein A is an amidino-containing moiety, a hydrazino-containing moiety, 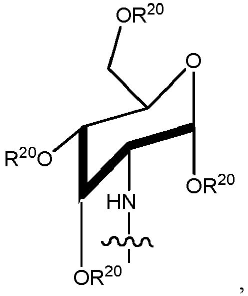 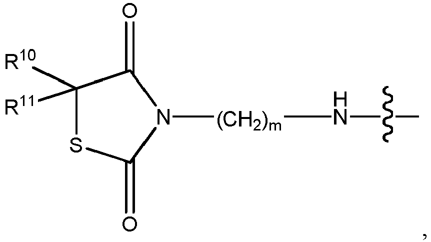 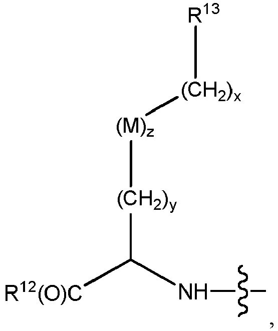 or 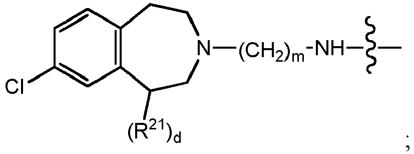
R¹, R², and R³ are each independently selected from optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
X is SO₂ or C=O;
R¹⁰, R¹¹, R¹², R¹³, and R²⁰ are each independently selected from H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
R²¹ is optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
M is S or Se;
a, b, and c are each independently 0, 1, 2, 3, 4 or 5;
m, x, and y are each independently 0, 1, 2, 3, 4, 5 or 6;
d is 0 or 1; and
z is 1 or 2.

Disclosed herein, but not within the literal scope of the claims, is a compound, or a pharmaceutically acceptable salt or ester thereof, comprising (i) a CB₁ receptor mediating scaffold and (ii) a second therapeutic scaffold.

Also disclosed herein, but not within the literal scope of the claims, is a compound, or a pharmaceutically acceptable salt or ester thereof, having a structure of: 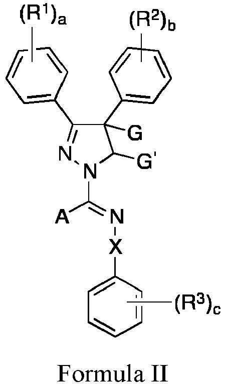 wherein A is an amidino-containing moiety, a hydrazino-containing moiety, an optionally-substituted thiol, 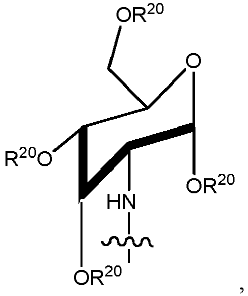 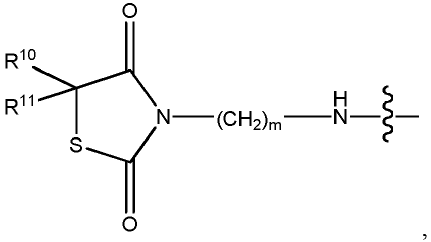 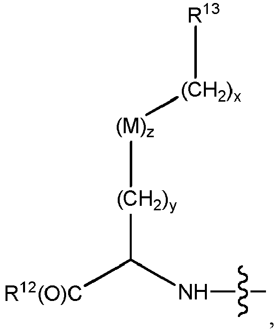 or 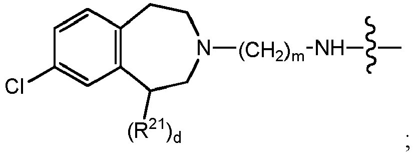
R¹, R², and R³ are each independently selected from optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
G and G' are each independently H, hydroxy, optionally-substituted alkyl, aralkyl, amino, or optionally-substituted thiol;
X is SO₂ or C=O;
R¹⁰, R¹¹, R¹², R¹³, and R²⁰ are each independently selected from H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
R²¹ is optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
M is S or Se;
a, b, and c are each independently 0, 1, 2, 3, 4 or 5;
m, x, and y are each independently 0, 1, 2, 3, 4, 5 or 6;
d is 0 or 1; and
z is 1 or 2.

Disclosed herein, but not within the literal scope of the claims, is a pharmaceutical composition comprising a compound disclosed herein, and at least one pharmaceutically acceptable additive.

Disclosed herein, but not within the literal scope of the claims, is a method for treating obesity, diabetes, non-alcoholic and alcoholic fatty liver disease, or a co-morbidity of obesity such as arteriosclerotic heart disease or gout, in a subject, comprising administering to the subject in need thereof a therapeutically effective amount of a compound disclosed herein.

Disclosed herein, but not within the literal scope of the claims, is a method for treating fibrosis or liver cancer in a subject, comprising administering to the subject in need thereof a therapeutically effective amount of a compound disclosed herein.

Disclosed herein, but not within the literal scope of the claims, is a method of preventing or reversing the deposition of adipose tissue in a subject, comprising administering to the subject in need thereof an effective amount of a compound disclosed herein.

The foregoing will become more apparent from the following detailed description of a several embodiments which proceeds with reference to the accompanying figures.

### BRIEF DESCRIPTION OF THE DRAWINGS

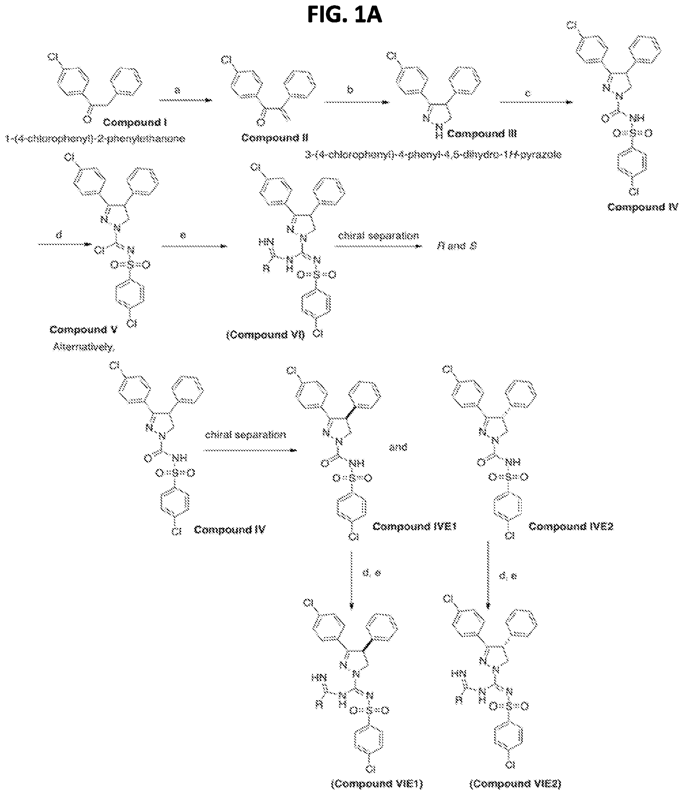

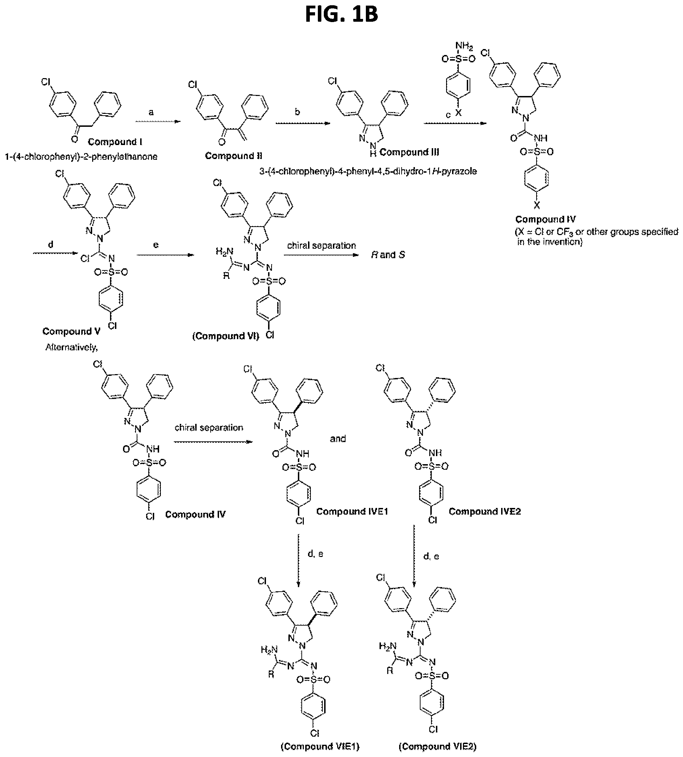

Figs. 1A, 1B, 2 and 3 depict synthesis schemes for compounds disclosed herein. Figs. 1A and 1B show the same scheme but with tautomeric isomers.

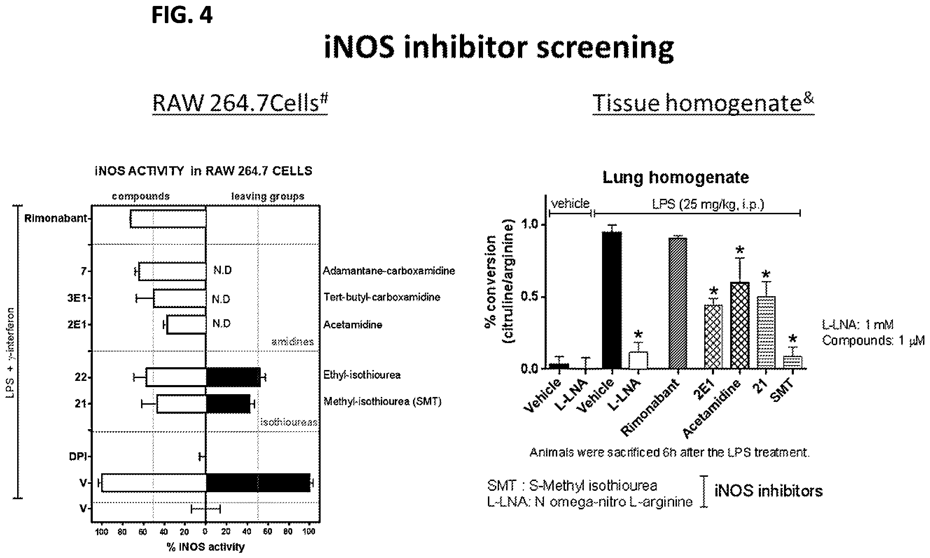

Fig. 4 shows iNOS inhibitory effect of compounds disclosed herein. ^{#}RAW264.7 cells incubated for 24 h in the absence or presence of LPS (50 ng/ml) and γ-interferon (10 ng/ml). Cellular iNOS activity was determined after replacing growth medium with reaction mixtures containing appropriate ligands (100 nM). & Mice were treated in vivo with vehicle or LPS (25 mg/kg, ip) and sacrificed 6 h later. Crude homogenate prepared from lung were incubated with the indicated ligands and iNOS activity was determined using a radioactivity-based assay.

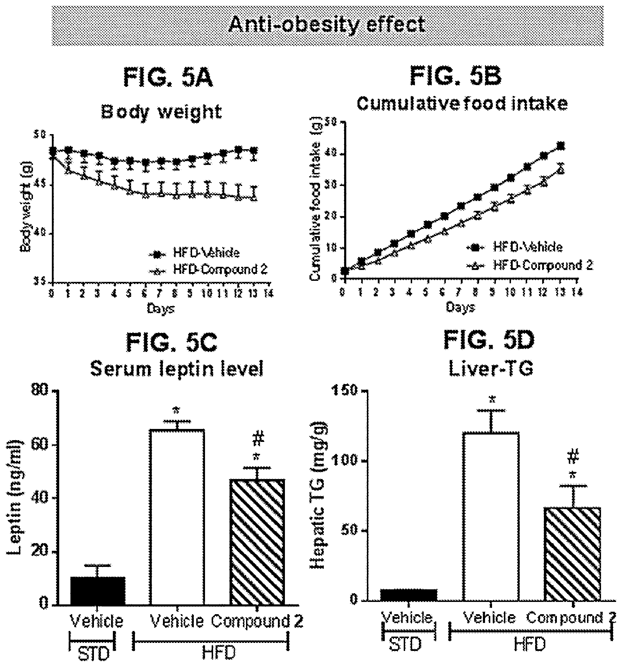

Figs. 5A-5G are graphs showing anti-obesity and anti-diabetic effects for a compound disclosed herein. DIO mice were treated for 14 days with the compound 2 (10 mg/kg/day). Compound 2 treatment reduced body weight (A), food intake (B), hyperleptinemia (C), hepatic TG (D) and abrogated HFD-induced glucose intolerance (E), insulin resistance (F), and hyperinsulinemia (G). Data represent mean ± SEM from 5-6 mice per group. **(P<0.05),* indicate significant difference from (Pettersen et al.) diet control. ^{#}indicates significant treatment effect *(P<0.05)* relative to vehicle-treated HFD group.

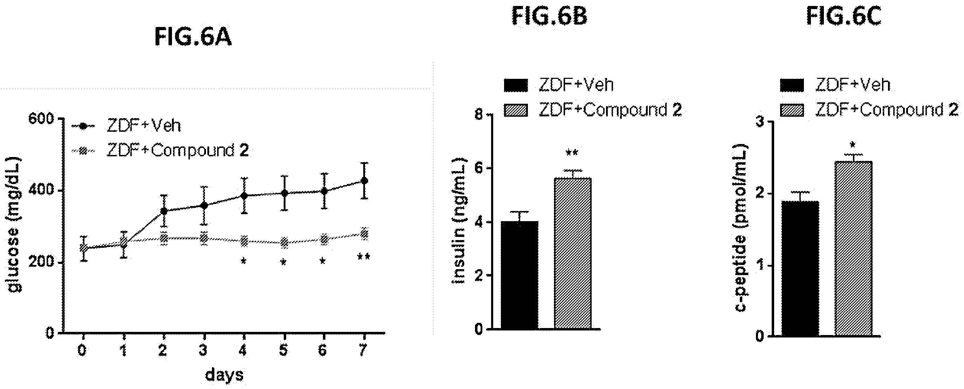

Figs. 6A-6C shows anti-diabetic effect of a compound disclosed herein. ZDF rats were treated with vehicle or compound 2 (10 mg/kg/day) by oral gavage for 7 days. Treatment with compound 2 prevented the progressive increase in blood glucose (A), and parallel decrease in plasma insulin (B), and plasma c-peptide (C). Data represent mean ± SEM from 4-5 mice per group. **(P<0.05),* indicate significant difference from vehicle group.

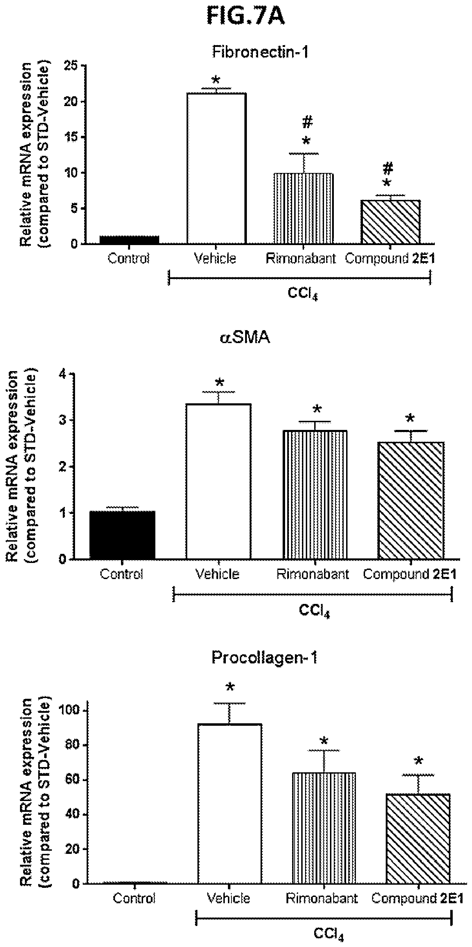

Figs. 7A-7C shows anti-fibrotic effect of a compound disclosed herein. CCl₄-induced liver fibrosis was generated by intraperitoneal injection of CCL₄ (1 ml/kg, diluted 1:10 corn oil), twice weekly for 8 weeks. Mice were also treated with vehicle, rimonabant, or compound 2 at 10 mg/kg/day orally for 4 weeks. Note that compound 2 is more effective than rimonabant in reducing α-SMA, Procollagen-1 and Fibronectin-1 mRNA (A) and in reducing liver fibrosis as assessed by Sirius Red and Masson's trichrome staining (B). CCl₄-induced increase in immunoreactive iNOS was attenuated by compound 2 but not by rimonabant (C). Data represent mean ± SEM from 7-8 mice per group. *P<0.05 relative to control. ^{#}indicates significant treatment effect *(P<0.05)* relative to CCl₄-treated vehicle group.

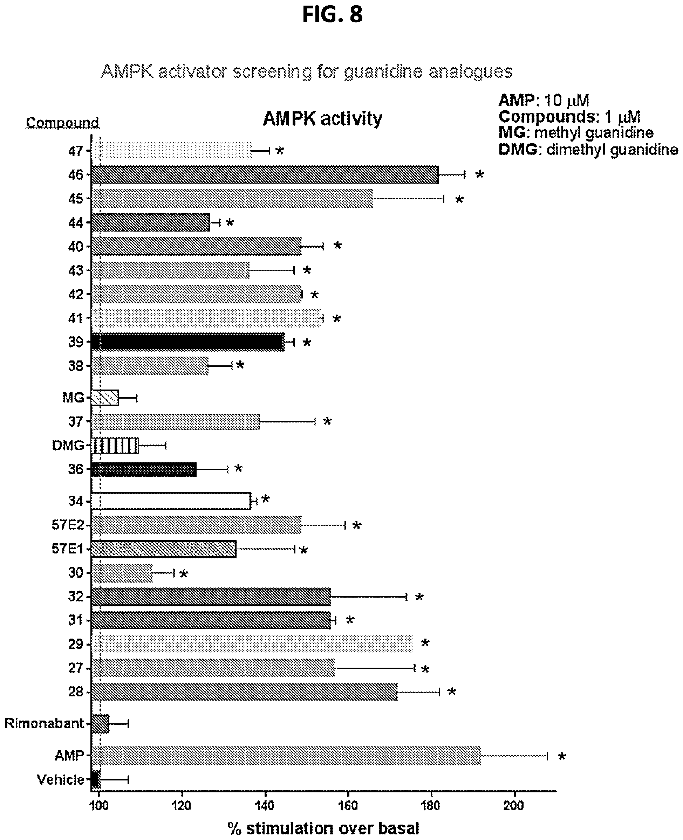

Fig. 8 shows AMPK (AMP-activated protein kinase) activation by compounds disclosed herein. Guanides and biguanides, such as metformin, are effective as antidiabetic agents linked to their AMPK activating properties (Hardie et al., Chem Biol. 2012, 19(1); 1222-1236). Guanidine-containing analogs in certain embodiments were screened for activation of recombinant human AMPK, using an assay kit (Cyclex, Nagona, Japan), as illustrated in Fig. 8. Note that all analogs elicited variable level of AMPK activation, whereas rimonabant even at the high concentration of 1 µM had no effect on AMPK activity.

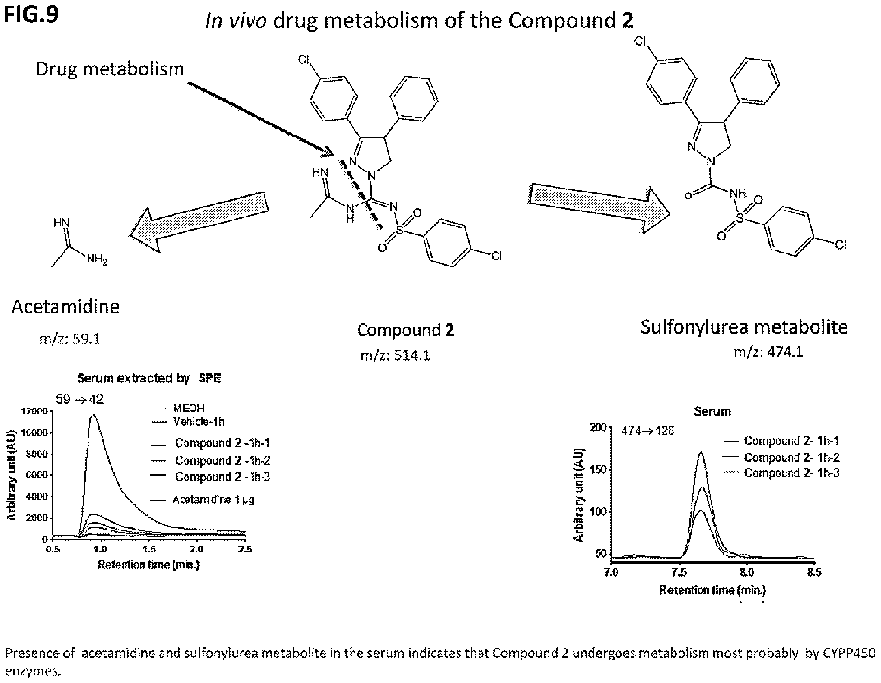

Fig. 9 illustrates the in vivo metabolism of compound 2. A normal mouse was given 10 mg/kg of compound 2 orally and sacrificed 1 h later to analyze the plasma level and chemical structure of the parent compound and its primary metabolites by LC/MS/MS.

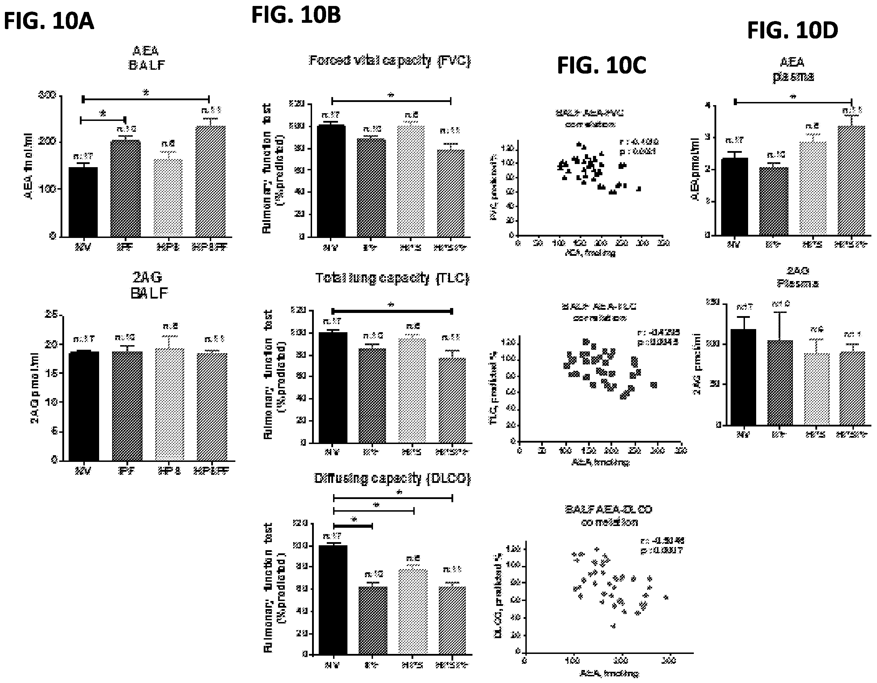

Figs. 10A -10D shows endocannabinoids levels in BALF (FIG. 10A) and plasma samples (FIG. 10D) from normal volunteers (NV), HPS patients without PF (HPS), with PF (HPSPF), and patients with IPF (IPF). Pulmonary function tests in the same group (FIG. 10B) and their correlation with BALF and AEA (Fig. 10C). Correlation was calculated by using Pearson correlation coefficients.

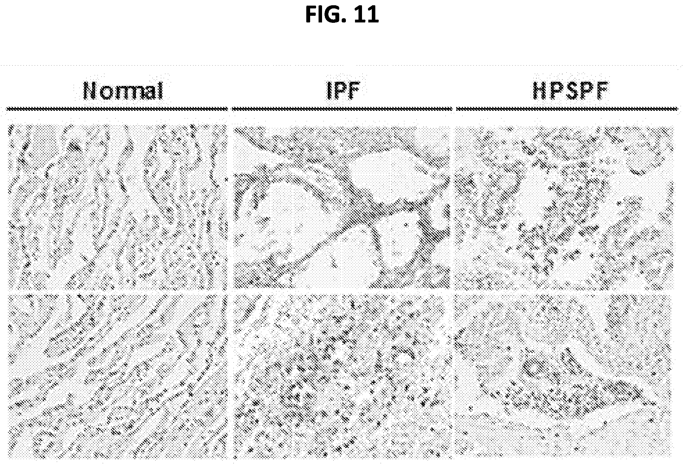

Fig. 11: CB₁R immuno-staining in human lung slides in normal, IPF and HPSPF patient populations. Specimens from at least 5 patients were stained and representative images were selected from each patient group. The person conducting these analyses was blinded to the sample IDs.

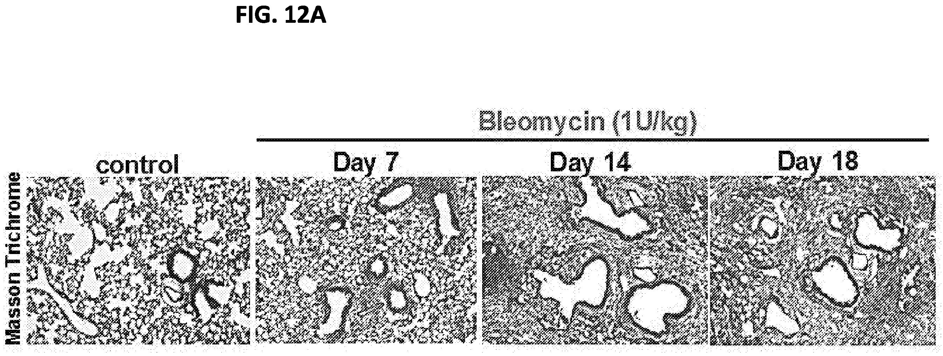

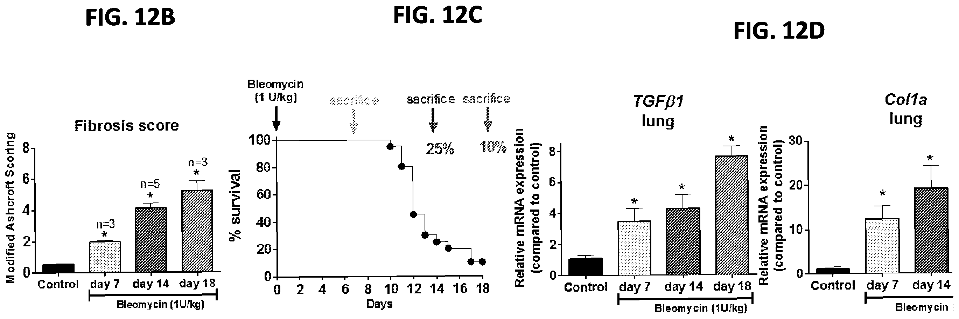

Fig. 12A -12D: Collagen deposition in lung tissue from bleomycin-treated mice assessed by Masson trichrome staining. Fibrosis grade was assessed using Ashcroft scoring. Fig. 12B: Eight fields were randomly selected from each slide and were independently scored by 3 readers blinded to sample ID (0, no fibrosis, 8, severe fibrosis). Fig. 12C: To determine survival time, 25% body weight loss was considered as study termination criteria. Fig. 12d: Gene expression of fibrosis markers in lung tissue.

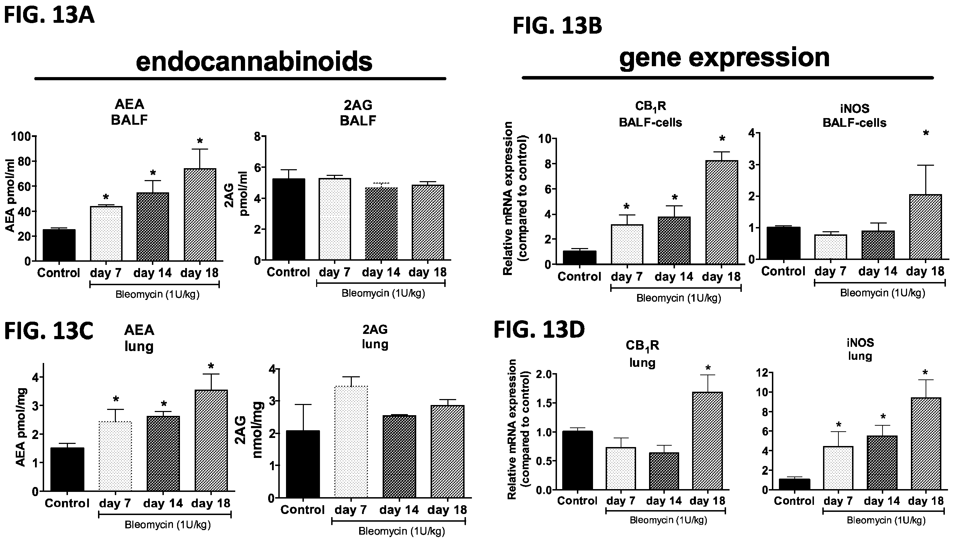

Figs. 13A and 13B show endocannabinoid levels in BALF (Fig. 13A) and lung (Fig. 13B). Gene expression *of cnr1* (CB₁R) and *nos2* (iNOS) in BALF cells (Fig. 13C) and lung (Fig. 13D). BAL was collected by washing the lungs with 1 ml HBSS without calcium and magnesium. BALF was centrifuged at low speed to isolate cells, which were then subjected to gene expression analyses.

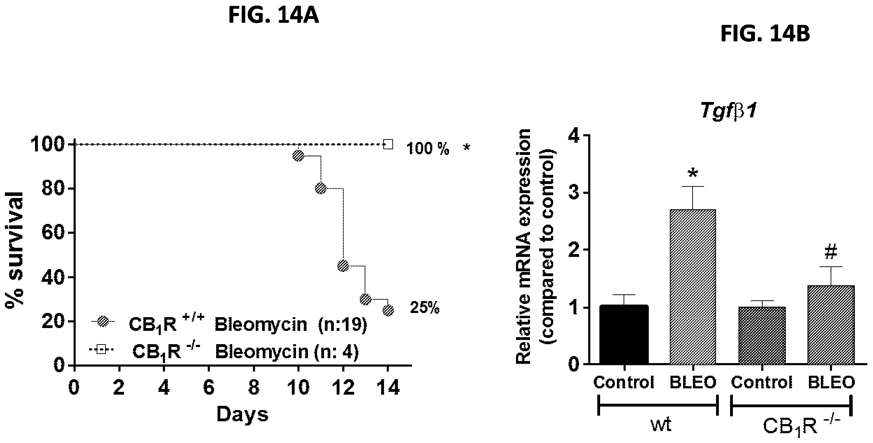

Figs. 14A and 14B: Survival curve was plotted in mice after bleomycin challenge and significant improvement of survival was analyzed by using Log-rank (Mantel-Cox) test between wt and CB₁R^{-/-} mice (Fig. 14A); gene expression of fibrogenic marker *Tgfβ1* (Fig. 14B). * (P<0.05) indicates significant difference from control (C) group in wt. ^{#}*(P<0.05)* indicates significant effect of gene deletion relative to wt in BLEO group.

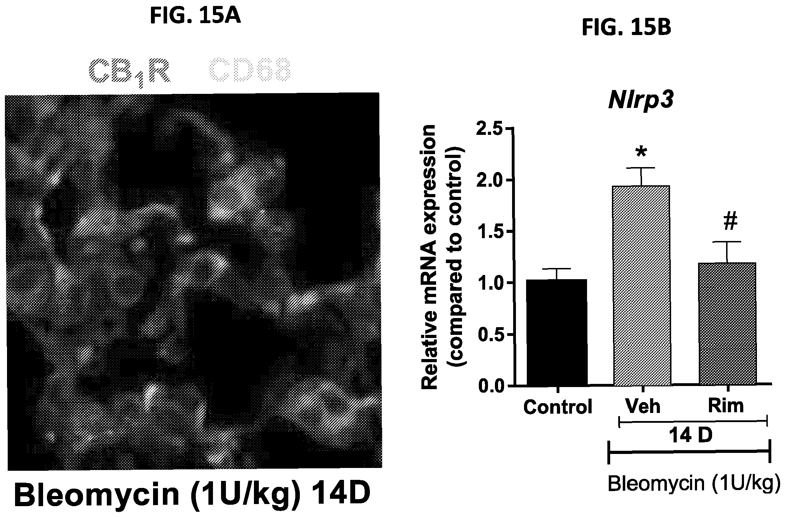

Figs. 15A and 15B: CB₁R/CD68 double immunochemistry and imaging by confocal microscopy in lung tissue from bleomycin-instilled mice (Fig. 15A). *Nlrp3* expression in lung tissue quantified 14 days post-bleomycin or vehicle, following chronic treatment with vehicle or rimonabant (3 mg/kg, PO) (Fig. 15B).

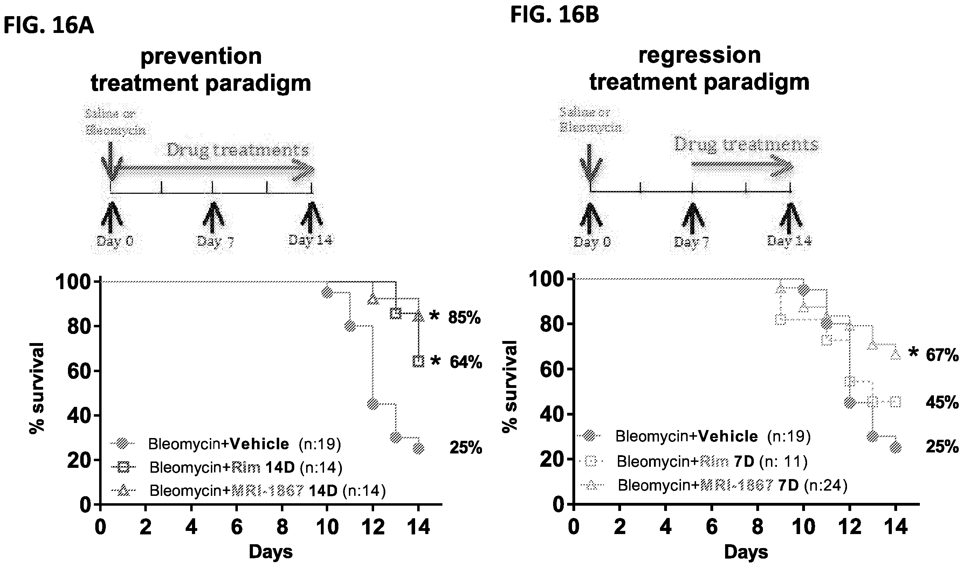

Figs. 16A and 16B: Survival plot for fibrosis prevention (Fig. 16A) and regression (Fig. 16B) treatment paradigms in bleomycin-instilled mice following different treatments.

### DETAILED DESCRIPTION

The present invention is defined by the claims. Any subject matter falling outside the scope of the claims is provided for information purposes only. Any references in the description to methods for treatment of the human or animal body by therapy refer to the compounds and pharmaceutical compositions of the present invention for use in methods for the treatment of the human or animal body by therapy.

### Terminology

The following explanations of terms and methods are provided to better describe the present compounds, compositions and methods, and to guide those of ordinary skill in the art in the practice of the present disclosure. It is also to be understood that the terminology used in the disclosure is for the purpose of describing particular embodiments and examples only and is not intended to be limiting.

"Acyl" refers to a group having the structure -C(O)R, where R may be, for example, optionally substituted alkyl, optionally substituted aryl, or optionally substituted heteroaryl. "Lower acyl" groups are those that contain one to six carbon atoms.

"Acyloxy" refers to a group having the structure -OC(O)R-, where R may be, for example, optionally substituted alkyl, optionally substituted aryl, or optionally substituted heteroaryl. "Lower acyloxy" groups contain one to six carbon atoms.

"Administration" as used herein is inclusive of administration by another person to the subject or self-administration by the subject.

The term "aliphatic" is defined as including alkyl, alkenyl, alkynyl, halogenated alkyl and cycloalkyl groups. A "lower aliphatic" group is a branched or unbranched aliphatic group having from 1 to 10 carbon atoms.

"Alkanediyl," "cycloalkanediyl," "aryldiyl," "alkanearyldiyl" refers to a divalent radical derived from aliphatic, cycloaliphatic, aryl, and alkanearyl hydrocarbons.

"Alkenyl" refers to a cyclic, branched or straight chain group containing only carbon and hydrogen, and contains one or more double bonds that may or may not be conjugated. Alkenyl groups may be unsubstituted or substituted. "Lower alkenyl" groups contain one to six carbon atoms.

The term "alkoxy" refers to a straight, branched or cyclic hydrocarbon configuration and combinations thereof, including from 1 to 20 carbon atoms, preferably from 1 to 8 carbon atoms (referred to as a "lower alkoxy"), more preferably from 1 to 4 carbon atoms, that include an oxygen atom at the point of attachment. An example of an "alkoxy group" is represented by the formula - OR, where R can be an alkyl group, optionally substituted with an alkenyl, alkynyl, aryl, aralkyl, cycloalkyl, halogenated alkyl, alkoxy or heterocycloalkyl group. Suitable alkoxy groups include methoxy, ethoxy, n-propoxy, i-propoxy, n-butoxy, i-butoxy, sec-butoxy, tert-butoxy cyclopropoxy, cyclohexyloxy, and the like.

"Alkoxycarbonyl" refers to an alkoxy substituted carbonyl radical, -C(O)OR, wherein R represents an optionally substituted alkyl, aryl, aralkyl, cycloalkyl, cycloalkylalkyl or similar moiety.

The term "alkyl" refers to a branched or unbranched saturated hydrocarbon group of 1 to 24 carbon atoms, such as methyl, ethyl, n-propyl, isopropyl, n-butyl, isobutyl, t-butyl, pentyl, hexyl, heptyl, octyl, decyl, tetradecyl, hexadecyl, eicosyl, tetracosyl and the like. A "lower alkyl" group is a saturated branched or unbranched hydrocarbon having from 1 to 6 carbon atoms. Preferred alkyl groups have 1 to 4 carbon atoms. Alkyl groups may be "substituted alkyls" wherein one or more hydrogen atoms are substituted with a substituent such as halogen, cycloalkyl, alkoxy, amino, hydroxyl, aryl, alkenyl, or carboxyl. For example, a lower alkyl or (C₁-C₆)alkyl can be methyl, ethyl, propyl, isopropyl, butyl, iso-butyl, sec-butyl, pentyl, 3-pentyl, or hexyl; (C₃-C₆)cycloalkyl can be cyclopropyl, cyclobutyl, cyclopentyl, or cyclohexyl; (C₃-C₆)cycloalkyl(C₁-C₆)alkyl can be cyclopropylmethyl, cyclobutylmethyl, cyclopentylmethyl, cyclohexylmethyl, 2-cyclopropylethyl, 2-cyclobutylethyl, 2-cyclopentylethyl, or 2-cyclohexylethyl; (C₁-C₆)alkoxy can be methoxy, ethoxy, propoxy, isopropoxy, butoxy, iso-butoxy, sec-butoxy, pentoxy, 3-pentoxy, or hexyloxy; (C₂-C₆)alkenyl can be vinyl, allyl, 1-propenyl, 2-propenyl, 1-butenyl, 2-butenyl, 3-butenyl, 1,-pentenyl, 2-pentenyl, 3-pentenyl, 4-pentenyl, 1- hexenyl, 2-hexenyl, 3-hexenyl, 4-hexenyl, or 5-hexenyl; (C₂-C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1- hexynyl, 2-hexynyl, 3-hexynyl, 4-hexynyl, or 5-hexynyl; (C₁-C₆)alkanoyl can be acetyl, propanoyl or butanoyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; hydroxy(C₁-C₆)alkyl can be hydroxymethyl, 1-hydroxyethyl, 2-hydroxyethyl, 1-hydroxypropyl, 2-hydroxypropyl, 3-hydroxypropyl, 1-hydroxybutyl, 4-hydroxybutyl, 1-hydroxypentyl, 5-hydroxypentyl, 1-hydroxyhexyl, or 6-hydroxyhexyl; (C₁-C₆)alkoxycarbonyl can be methoxycarbonyl, ethoxycarbonyl, propoxycarbonyl, isopropoxycarbonyl, butoxycarbonyl, pentoxycarbonyl, or hexyloxycarbonyl; (C₁-C₆)alkylthio can be methylthio, ethylthio, propylthio, isopropylthio, butylthio, isobutylthio, pentylthio, or hexylthio; (C₂-C₆)alkanoyloxy can be acetoxy, propanoyloxy, butanoyloxy, isobutanoyloxy, pentanoyloxy, or hexanoyloxy.

"Alkynyl" refers to a cyclic, branched or straight chain group containing only carbon and hydrogen, and unless otherwise mentioned typically contains one to twelve carbon atoms, and contains one or more triple bonds. Alkynyl groups may be unsubstituted or substituted. "Lower alkynyl" groups are those that contain one to six carbon atoms.

The term "amine" or "amino" refers to a group of the formula -NRR', where R and R' can be, independently, hydrogen or an alkyl, alkenyl, alkynyl, aryl, aralkyl, cycloalkyl, halogenated alkyl, or heterocycloalkyl group. For example, an "alkylamino" or "alkylated amino" refers to - NRR', wherein at least one of R or R' is an alkyl.

The term "aminoalkyl" refers to alkyl groups as defined above where at least one hydrogen atom is replaced with an amino group (e.g, -CH₂-NH₂).

"Aminocarbonyl" alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like. An aminocarbonyl group may be -N(R)-C(O)-R (wherein R is a substituted group or H). A suitable aminocarbonyl group is acetamido.

The term "amide" or "amido" is represented by the formula -C(O)NRR', where R and R' independently can be a hydrogen, alkyl, alkenyl, alkynyl, aryl, aralkyl, cycloalkyl, halogenated alkyl, or heterocycloalkyl group.

An "analog" is a molecule that differs in chemical structure from a parent compound, for example a homolog (differing by an increment in the chemical structure or mass, such as a difference in the length of an alkyl chain or the inclusion of one of more isotopes), a molecular fragment, a structure that differs by one or more functional groups, or a change in ionization. An analog is not necessarily synthesized from the parent compound. A derivative is a molecule derived from the base structure.

An "animal" refers to living multi-cellular vertebrate organisms, a category that includes, for example, mammals and birds. The term mammal includes both human and non-human mammals. Similarly, the term "subject" includes both human and non-human subjects, including birds and non-human mammals, such as non-human primates, companion animals (such as dogs and cats), livestock (such as pigs, sheep, cows), as well as non-domesticated animals, such as the big cats. The term subject applies regardless of the stage in the organism's life-cycle. Thus, the term subject applies to an organism *in utero* or *in ovo,* depending on the organism (that is, whether the organism is a mammal or a bird, such as a domesticated or wild fowl).

The term "aralkyl" refers to an alkyl group wherein an aryl group is substituted for a hydrogen of the alkyl group. An example of an aralkyl group is a benzyl group.

"Aryl" refers to a monovalent unsaturated aromatic carbocyclic group having a single ring (e.g., phenyl) or multiple condensed rings (e.g., naphthyl or anthryl), which can optionally be unsubstituted or substituted. A "heteroaryl group," is defined as an aromatic group that has at least one heteroatom incorporated within the ring of the aromatic group. Examples of heteroatoms include, but are not limited to, nitrogen, oxygen, sulfur, and phosphorous. Heteroaryl includes, but is not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like. The aryl or heteroaryl group can be substituted with one or more groups including, but not limited to, alkyl, alkynyl, alkenyl, aryl, halide, nitro, amino, ester, ketone, aldehyde, hydroxy, carboxylic acid, or alkoxy, or the aryl or heteroaryl group can be unsubstituted.

"Aryloxy" or "heteroaryloxy" refers to a group of the formula -OAr, wherein Ar is an aryl group or a heteroaryl group, respectively.

The term "carboxylate" or "carboxyl" refers to the group -COO⁻ or -COOH. The carboxyl group can form a carboxylic acid. "Substituted carboxyl" refers to -COOR where R is alkyl, alkenyl, alkynyl, aryl, aralkyl, cycloalkyl, halogenated alkyl, or heterocycloalkyl group. For example, a substituted carboxyl group could be a carboxylic acid ester or a salt thereof (e.g., a carboxylate).

The term "co-administration" or "co-administering" refers to administration of a dendrimeric compound disclosed herein with at least one other therapeutic or diagnostic agent within the same general time period, and does not require administration at the same exact moment in time (although co-administration is inclusive of administering at the same exact moment in time). Thus, co-administration may be on the same day or on different days, or in the same week or in different weeks. In certain embodiments, a plurality of therapeutic and/or diagnostic agents may be co-administered by encapsulating the agents within the dendrimeric platform disclosed herein and/or by covalently conjugating the agents to the surface of the dendrimeric platform.

The term "cycloalkyl" refers to a non-aromatic carbon-based ring composed of at least three carbon atoms. Examples of cycloalkyl groups include, but are not limited to, cyclopropyl, cyclobutyl, cyclopentyl, cyclohexyl, and the like. The term "heterocycloalkyl group" is a cycloalkyl group as defined above where at least one of the carbon atoms of the ring is substituted with a heteroatom such as, but not limited to, nitrogen, oxygen, sulfur, or phosphorous.

The term "ester" refers to a carboxyl group-containing moiety having the hydrogen replaced with, for example, a C₁₋₆alkyl group ("carboxylC₁₋₆alkyl" or "alkylester"), an aryl or aralkyl group ("arylester" or "aralkylester") and so on. CO₂C₁₋₃alkyl groups are preferred, such as for example, methylester (CO ₂Me), ethylester (CO₂Et) and propylester (CO₂Pr) and includes reverse esters thereof (e.g. -OCOMe, -OCOEt and -OCOPr).

The terms "halogenated alkyl" or "haloalkyl group" refer to an alkyl group with one or more hydrogen atoms present on these groups substituted with a halogen (F, Cl, Br, I).

The term "hydroxyl" is represented by the formula -OH.

The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

"Inhibiting" refers to inhibiting the full development of a disease or condition. "Inhibiting" also refers to any quantitative or qualitative reduction in biological activity or binding, relative to a control.

"N-heterocyclic" refers to mono or bicyclic rings or ring systems that include at least one nitrogen heteroatom. The rings or ring systems generally include 1 to 9 carbon atoms in addition to the heteroatom(s) and may be saturated, unsaturated or aromatic (including pseudoaromatic). The term "pseudoaromatic" refers to a ring system which is not strictly aromatic, but which is stabilized by means of delocalization of electrons and behaves in a similar manner to aromatic rings. Aromatic includes pseudoaromatic ring systems, such as pyrrolyl rings.

Examples of 5-membered monocyclic N-heterocycles include pyrrolyl, H-pyrrolyl, pyrrolinyl, pyrrolidinyl, oxazolyl, oxadiazolyl, (including 1,2,3 and 1,2,4 oxadiazolyls) isoxazolyl, furazanyl, thiazolyl, isothiazolyl, pyrazolyl, pyrazolinyl, pyrazolidinyl, imidazolyl, imidazolinyl, triazolyl (including 1,2,3 and 1,3,4 triazolyls), tetrazolyl, thiadiazolyl (including 1,2,3 and 1,3,4 thiadiazolyls), and dithiazolyl. Examples of 6-membered monocyclic N-heterocycles include pyridyl, pyrimidinyl, pyridazinyl, pyrazinyl, piperidinyl, morpholinyl, thiomorpholinyl, piperazinyl, and triazinyl. The heterocycles may be optionally substituted with a broad range of substituents, and preferably with C₁₋₆ alkyl, C₁₋₆ alkoxy, C₂₋₆ alkenyl, C₂₋₆ alkynyl, halo, hydroxy, mercapto, trifluoromethyl, amino, cyano or mono or di(C₁₋₆alkyl)amino. The N-heterocyclic group may be fused to a carbocyclic ring such as phenyl, naphthyl, indenyl, azulenyl, fluorenyl, and anthracenyl.

Examples of 8, 9 and 10-membered bicyclic heterocycles include 1H thieno[2,3-c]pyrazolyl, indolyl, isoindolyl, benzoxazolyl, benzothiazolyl, benzisoxazolyl, benzisothiazolyl, benzimidazolyl, indazolyl, isoquinolinyl, quinolinyl, quinoxalinyl, purinyl, cinnolinyl, phthalazinyl, quinazolinyl, quinoxalinyl, benzotriazinyl, and the like. These heterocycles may be optionally substituted, for example with C₁₋₆ alkyl, C₁₋₆ alkoxy, C₂₋₆ alkenyl, C₂₋₆ alkynyl, halo, hydroxy, mercapto, trifluoromethyl, amino, cyano or mono or di(C₁₋₆alkyl)amino. Unless otherwise defined optionally substituted N-heterocyclics includes pyridinium salts and the N-oxide form of suitable ring nitrogens.

The term "subject" includes both human and non-human subjects, including birds and non-human mammals, such as non-human primates, companion animals (such as dogs and cats), livestock (such as pigs, sheep, cows), as well as non-domesticated animals, such as the big cats. The term subject applies regardless of the stage in the organism's life-cycle. Thus, the term subject applies to an organism *in utero* or *in ovo,* depending on the organism (that is, whether the organism is a mammal or a bird, such as a domesticated or wild fowl).

"Substituted" or "substitution" refers to replacement of a hydrogen atom of a molecule or an R-group with one or more additional R-groups. Unless otherwise defined, the term "optionally-substituted" or "optional substituent" as used herein refers to a group which may or may not be further substituted with 1, 2, 3, 4 or more groups, preferably 1, 2 or 3, more preferably 1 or 2 groups. The substituents may be selected, for example, from C₁₋₆alkyl, C₂₋₆alkenyl, C₂₋₆alkynyl, C₃₋₈cycloalkyl, hydroxyl, oxo, C₁₋₆alkoxy, aryloxy, C₁₋₆alkoxyaryl, halo, C₁₋₆alkylhalo (such as CF₃ and CHF₂), C₁₋₆alkoxyhalo (such as OCF₃ and OCHF₂), carboxyl, esters, cyano, nitro, amino, substituted amino, disubstituted amino, acyl, ketones, amides, aminoacyl, substituted amides, disubstituted amides, thiol, alkylthio, thioxo, sulfates, sulfonates, sulfinyl, substituted sulfinyl, sulfonyl, substituted sulfonyl, sulfonylamides, substituted sulfonamides, disubstituted sulfonamides, aryl, arC₁₋₆alkyl, heterocyclyl and heteroaryl wherein each alkyl, alkenyl, alkynyl, cycloalkyl, aryl and heterocyclyl and groups containing them may be further optionally substituted. Optional substituents in the case N-heterocycles may also include but are not limited to C₁₋₆alkyl i.e. *N*C₁₋₃alkyl, more preferably methyl particularly *N*-methyl.

"Sulfinyl" refers to the group -S(=O)H.

The term "substituted sulfinyl" or "sulfoxide" refers to a sulfinyl group having the hydrogen replaced with, for example a C₁₋₆alkyl group ("C₁₋₆alkylsulfinyl" or "C₁₋₆alkylsulfoxide"), an aryl ("arylsulfinyl"), an aralkyl ("aralkyl sulfinyl") and so on. C₁₋₃alkylsulfinyl groups are preferred, such as for example, -SOmethyl, -SOethyl and -SOpropyl.

The term "sulfonyl" refers to the group -SO₂H.

The term "substituted sulfonyl" refers to a sulfonyl group having the hydrogen replaced with, for example a C₁₋₆alkyl group ("sulfonylC_{1\-6}alkyl"), an aryl ("arylsulfonyl"), an aralkyl ("aralkylsulfonyl") and so on. SulfonylC₁₋₃alkyl groups are preferred, such as for example, - SO₂Me, -SO₂Et and -SO₂Pr.

The term "sulfonylamido" or "sulfonamide" refers to the group -SO₂NH₂.

A "therapeutically effective amount" refers to a quantity of a specified agent sufficient to achieve a desired effect in a subject being treated with that agent. For example, a therapeutically amount may be an amount of a FBXO3 inhibitor that is sufficient to inhibit inflammation in a subject. Ideally, a therapeutically effective amount of an agent is an amount sufficient to inhibit or treat the disease or condition without causing a substantial cytotoxic effect in the subject. The therapeutically effective amount of an agent will be dependent on the subject being treated, the severity of the affliction, and the manner of administration of the therapeutic composition.

"Thiol" refers to the group -SH.

The term "substituted thiol" refers to a thiol group having the hydrogen replaced with, for example a C₁₋₆alkyl group ("-S(C₁₋₆alkyl)"), an aryl ("-S(aryl)"), or an aralkyl ("-S(alkyl)(aryl)") and so on.

"Treatment" refers to a therapeutic intervention that ameliorates a sign or symptom of a disease or pathological condition after it has begun to develop, or administering a compound or composition to a subject who does not exhibit signs of a disease or exhibits only early signs for the purpose of decreasing the risk of developing a pathology or condition, or diminishing the severity of a pathology or condition. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. The phrase "treating a disease" refers to inhibiting the full development of a disease, for example, in a subject who is at risk for a disease such as diabetes. "Preventing" a disease or condition refers to prophylactic administering a composition to a subject who does not exhibit signs of a disease or exhibits only early signs for the purpose of decreasing the risk of developing a pathology or condition, or diminishing the severity of a pathology or condition. In certain embodiments disclosed herein, the treatment inhibits food intake or weight gain in a subject. In certain embodiments disclosed herein, the treatment inhibits fibrogenesis or reverses insulin resistance in a subj ect.

"Pharmaceutical compositions" are compositions that include an amount (for example, a unit dosage) of one or more of the disclosed compounds together with one or more non-toxic pharmaceutically acceptable additives, including carriers, diluents, and/or adjuvants, and optionally other biologically active ingredients. Such pharmaceutical compositions can be prepared by standard pharmaceutical formulation techniques such as those disclosed in Remington's Pharmaceutical Sciences, Mack Publishing Co., Easton, PA (19th Edition).

The terms "pharmaceutically acceptable salt or ester" refers to salts or esters prepared by conventional means that include salts, e.g., of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. "Pharmaceutically acceptable salts" of the presently disclosed compounds also include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. These salts may be prepared by standard procedures, for example by reacting the free acid with a suitable organic or inorganic base. Any chemical compound recited in this specification may alternatively be administered as a pharmaceutically acceptable salt thereof. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). When compounds disclosed herein include an acidic function such as a carboxy group, then suitable pharmaceutically acceptable cation pairs for the carboxy group are well known to those skilled in the art and include alkaline, alkaline earth, ammonium, quaternary ammonium cations and the like. Such salts are known to those of skill in the art. For additional examples of "pharmacologically acceptable salts," see Berge et al., J. Pharm. Sci. 66:1 (1977).

"Pharmaceutically acceptable esters" includes those derived from compounds described herein that are modified to include a carboxyl group. An *in vivo* hydrolysable ester is an ester, which is hydrolysed in the human or animal body to produce the parent acid or alcohol. Representative esters thus include carboxylic acid esters in which the non-carbonyl moiety of the carboxylic acid portion of the ester grouping is selected from straight or branched chain alkyl (for example, methyl, n-propyl, t-butyl, or n-butyl), cycloalkyl, alkoxyalkyl (for example, methoxymethyl), aralkyl (for example benzyl), aryloxyalkyl (for example, phenoxymethyl), aryl (for example, phenyl, optionally substituted by, for example, halogen, C.sub. 1-4 alkyl, or C.sub. 1-4 alkoxy) or amino); sulphonate esters, such as alkyl- or aralkylsulphonyl (for example, methanesulphonyl); or amino acid esters (for example, L-valyl or L-isoleucyl). A "pharmaceutically acceptable ester" also includes inorganic esters such as mono-, di-, or triphosphate esters. In such esters, unless otherwise specified, any alkyl moiety present advantageously contains from 1 to 18 carbon atoms, particularly from 1 to 6 carbon atoms, more particularly from 1 to 4 carbon atoms. Any cycloalkyl moiety present in such esters advantageously contains from 3 to 6 carbon atoms. Any aryl moiety present in such esters advantageously comprises a phenyl group, optionally substituted as shown in the definition of carbocycylyl above. Pharmaceutically acceptable esters thus include C₁-C₂₂ fatty acid esters, such as acetyl, t-butyl or long chain straight or branched unsaturated or omega-6 monounsaturated fatty acids such as palmoyl, stearoyl and the like. Alternative aryl or heteroaryl esters include benzoyl, pyridylmethyloyl and the like any of which may be substituted, as defined in carbocyclyl above. Additional pharmaceutically acceptable esters include aliphatic L-amino acid esters such as leucyl, isoleucyl and especially valyl.

For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

The pharmaceutically acceptable acid and base addition salts as mentioned hereinabove are meant to comprise the therapeutically active non-toxic acid and base addition salt forms which the compounds are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which the compounds described herein are able to form. Such solvates are for example hydrates, alcoholates and the like.

The term "quaternary amine" as used hereinbefore defines the quaternary ammonium salts which the compounds are able to form by reaction between a basic nitrogen of a compound and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

Disclosed herein, but not within the literal scope of the claims, are prodrugs of the disclosed compounds. A prodrug is an active or inactive compound that is modified chemically through *in vivo* physiological action, such as hydrolysis, metabolism and the like, into an active compound following administration of the prodrug to a subject. The term "prodrug" as used throughout this text means the pharmacologically acceptable derivatives such as esters, amides and phosphates, such that the resulting *in vivo* biotransformation product of the derivative is the active drug as defined in the compounds described herein. Prodrugs preferably have excellent aqueous solubility, increased bioavailability and are readily metabolized into the active inhibitors *in vivo.* Prodrugs of a compounds described herein may be prepared by modifying functional groups present in the compound in such a way that the modifications are cleaved, either by routine manipulation or *in vivo,* to the parent compound. The suitability and techniques involved in making and using prodrugs are well known by those skilled in the art. F or a general discussion of prodrugs involving esters see Svensson and Tunek, Drug Metabolism Reviews 165 (1988) and Bundgaard, Design of Prodrugs, Elsevier (1985).

The term "prodrug" also is intended to include any covalently bonded carriers that release an active parent drug of the present invention *in vivo* when the prodrug is administered to a subject. Since prodrugs often have enhanced properties relative to the active agent pharmaceutical, such as, solubility and bioavailability, the compounds disclosed herein can be delivered in prodrug form. Thus, also disclosed are prodrugs of the presently disclosed compounds, methods of delivering prodrugs and compositions containing such prodrugs. Prodrugs of the disclosed compounds typically are prepared by modifying one or more functional groups present in the compound in such a way that the modifications are cleaved, either in routine manipulation or *in vivo,* to yield the parent compound. Prodrugs include compounds having a phosphonate and/or amino group functionalized with any group that is cleaved *in vivo* to yield the corresponding amino and/or phosphonate group, respectively. Examples of prodrugs include, without limitation, compounds having an acylated amino group and/or a phosphonate ester or phosphonate amide group. In particular examples, a prodrug is a lower alkyl phosphonate ester, such as an isopropyl phosphonate ester.

Protected derivatives of the disclosed compounds also are contemplated. A variety of suitable protecting groups for use with the disclosed compounds are disclosed in Greene and Wuts, Protective Groups in Organic Synthesis; 3rd Ed.; John Wiley & Sons, New York, 1999.

In general, protecting groups are removed under conditions that will not affect the remaining portion of the molecule. These methods are well known in the art and include acid hydrolysis, hydrogenolysis and the like. One preferred method involves the removal of an ester, such as cleavage of a phosphonate ester using Lewis acidic conditions, such as in TMS-Br mediated ester cleavage to yield the free phosphonate. A second preferred method involves removal of a protecting group, such as removal of a benzyl group by hydrogenolysis utilizing palladium on carbon in a suitable solvent system such as an alcohol, acetic acid, and the like or mixtures thereof. A t-butoxy-based group, including t-butoxy carbonyl protecting groups can be removed utilizing an inorganic or organic acid, such as HCl or trifluoroacetic acid, in a suitable solvent system, such as water, dioxane and/or methylene chloride. Another exemplary protecting group, suitable for protecting amino and hydroxy functions amino is trityl. Other conventional protecting groups are known and suitable protecting groups can be selected by those of skill in the art in consultation with Greene and Wuts, Protective Groups in Organic Synthesis; 3rd Ed.; John Wiley & Sons, New York, 1999. When an amine is deprotected, the resulting salt can readily be neutralized to yield the free amine. Similarly, when an acid moiety, such as a phosphonic acid moiety is unveiled, the compound may be isolated as the acid compound or as a salt thereof.

### Compounds

Disclosed herein are novel peripherally restricted cannabinoid receptor mediating compounds for the treatment of, for example, fibrosis, diabetes, obesity and liver cancer. The cannabinoid receptor may be CB₁ and/or CB₂ receptors. The compounds may be essentially non-selective for CB₁ versus CB₂, or show selectivity for either the CB₁ receptor or the CB₂ receptor. In a preferred embodiment, the cannabinoid receptor mediating compounds are selective of CB₁ receptors.

In certain embodiments, the cannabinoid receptor mediating compounds are cannabinoid receptor inverse agonists, particularly CB₁ inverse agonists. In certain embodiments, the cannabinoid receptor mediating compounds are neutral antagonists. A CB₁ inverse agonist is a drug that on its own produces an effect opposite to that of a CB₁ agonist, and is also able to block the effect of a CB₁ agonist. In contrast, a CB₁ neutral antagonist can only do the latter (i.e. blocking the effect of a CB₁ agonist), but has no effect on its own. CB₁ inverse agonism is usually documented by the ability of a drug to decrease GTPgammaS binding and/or to increase adenylate cyclase activity.

In certain embodiments, the compounds preferentially target CB₁ receptors in peripheral tissue (e.g., adipose tissue, liver, muscle, lung, kidney, macrophages, pancreatic beta cells and gastrointestinal tract), while not interacting with CB₁ receptors in brain tissue. Peripherally-mediated effects are maintained, but CNS side effects are minimal or non-existent.

There is evidence that the metabolic effects of endocannabinoids are mediated, at least in part, by CB₁ receptors in peripheral tissues, whereas the neuropsychiatric side effects are mediated by CB₁ receptor in the brain. This suggests CB₁ receptor blocking drugs with reduced ability to penetrate the brain would cause fewer if any neuropsychiatric side effects while retaining some or most of their metabolic benefits. As to limited metabolic efficacy of CB₁ receptor blocking drugs, this could be improved by the design of dual activity compounds that act on more than one target in the cell to influence the same metabolic process. As an example, such secondary targets could include, but not limited to, the enzyme inducible nitric oxide synthase (iNOS) or adenosine monophosphate kinase (AMPK), as suggested by findings that inhibition of iNOS or activation of AMPK improves insulin resistance, and reduces fibrosis and inflammation (Shinozaki S et al., J. Biol. Chem. 2012, 286(40), 34959-34975; Young RJ et al., Bioorg. Med. Chem Let. 2000, 10(6), 597-600; da Silva Morais A et al., Clin. Sci. 2010, 118(6), 411-420). Certain embodiments disclosed herein are CB₁ blocking compounds that have very low brain penetrance, and give rise to metabolites that either inhibit iNOS or activate AMPK directly. The generation of an iNOS inhibitory metabolite of compound 2 is illustrated in Fig. 9.

In certain embodiments, a peripherally restricted cannabinoid CB₁ receptor mediating compound may be characterized and can be identified from a ratio of maximum concentration in the brain to maximum concentration in plasma which is less than 0.1, as measured in a mouse after intravenous dosing. The preferred peripherally restricted cannabinoid CB₁ receptor mediating compounds have a brain Cₘₐₓ to plasma Cₘₐₓ ratio which is less than 0.05. Especially preferred peripherally restricted cannabinoid receptor mediating compounds have a brain Cₘₐₓ to plasma Cₘₐₓ ratio which is less than 0.025.

Disclosed herein, but not within the literal scope of the claims, are compounds, or pharmaceutically acceptable salts or esters, thereof having a formula of: 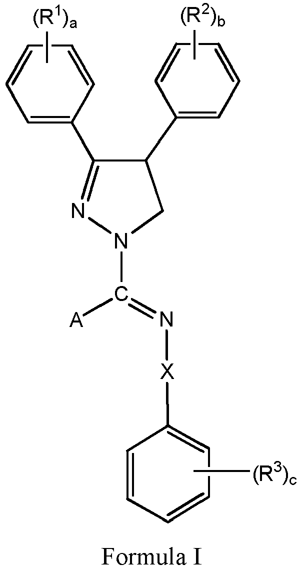 wherein A is an amidino-containing moiety, a hydrazino-containing moiety, 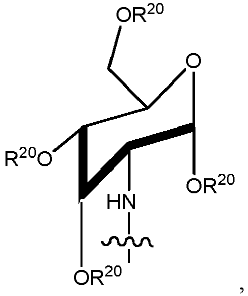 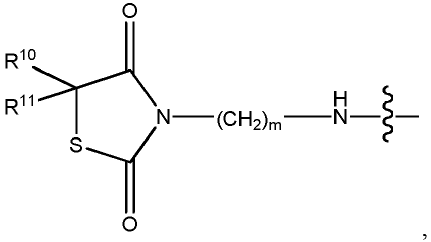 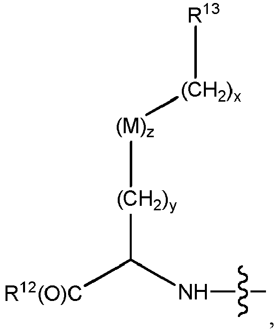 or 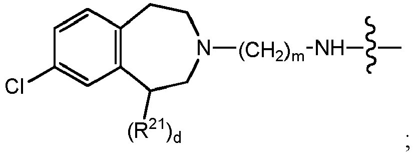
R¹, R², and R³ are each independently selected from optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
X is SO₂ or C=O;
R¹⁰, R¹¹, R¹², R¹³, and R²⁰ are each independently selected from H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
R²¹ is optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
M is S or Se;
a, b, and c are each independently 0, 1, 2, 3, 4 or 5;
m, x, and y are each independently 0, 1, 2, 3, 4, 5 or 6;
d is 0 or 1; and
z is 1 or 2.

Also disclosed herein, but not within the literal scope of the claims, is a compound, or a pharmaceutically acceptable salt or ester thereof, having a structure of: 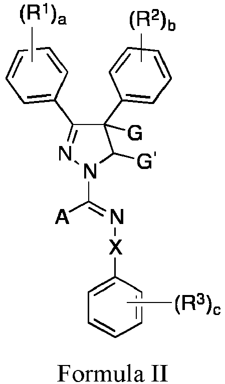 wherein A is an amidino-containing moiety, a hydrazino-containing moiety, an optionally-substituted thiol, 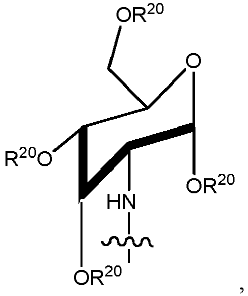 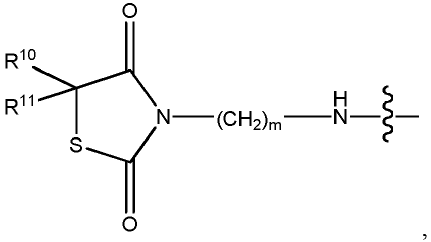 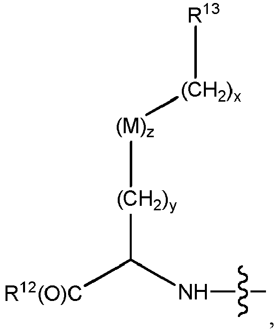 or 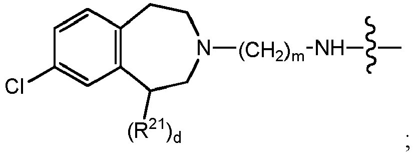
R¹, R², and R³ are each independently selected from optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
G and G' are each independently H, hydroxy, optionally-substituted alkyl, aralkyl, amino, or optionally-substituted thiol;
X is SO₂ or C=O;
R¹⁰, R¹¹, R¹², R¹³, and R²⁰ are each independently selected from H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
R²¹ is optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino;
M is S or Se;
a, b, and c are each independently 0, 1, 2, 3, 4 or 5;
m, x, and y are each independently 0, 1, 2, 3, 4, 5 or 6;
d is 0 or 1; and
z is 1 or 2.

Also disclosed, but not within the literal scope of the claims, A is an amidino-containing moiety having a structure of 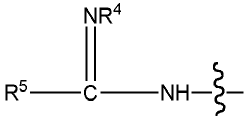 wherein R⁴ is selected from H, optionally-substituted alkyl, optionally-substituted cycloalkyl, optionally-substituted heterocycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino; and R⁵ is selected from optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R⁵ is an optionally-substituted thiol. R⁴ is H, hydroxy, C₁-C₆ alkyl, or acyl (e.g., t-butyloxycarbonyl). R⁵ is C₁-C₆ alkyl, C₃-C₆ cycloalkyl, amino (e.g., -NH₂, -N(alkyl)₂ or -NH(alkyl)), phenyl, heteroaryl, acyl (e.g., t-butyloxycarbonyl) or heterocycloalkyl. R⁴ is H, hydroxy, C₁-C₆ alkyl, or acyl and R⁵ is C₁-C₆ alkyl, C₃-C₆ cycloalkyl, amino (e.g., -N(alkyl)₂ or -NH(alkyl)), phenyl, heteroaryl (e.g., an N-heteroaryl), or heterocycloalkyl (e.g., an N-heterocycloalkyl). In particular embodiments, R⁴ is H.

In particular embodiments, R⁵ is a thiol substituted with a C₁-C₆ alkyl (e.g., -S(C₁-C₆ alkyl)), or a substituted C₁-C₆ alkyl, particularly an aralkyl (e.g, -S(C₁-C₆ alkyl)Ph). In particular embodiments, R⁴ is H and R⁵ is a substituted thiol.

In particular embodiments, R⁵ is an amino of the formula -NR³⁰R³¹, where R³⁰ and R³¹ can be, independently, hydrogen or an optionally-substituted C₁-C₆ alkyl, provided that at least one of R³⁰ and R³¹ is optionally substituted C₁-C₆ alkyl. Illustrative substituted alkyls for R³⁰ and R³¹ include, for example, alkenyl-substituted alkyl, alkoxy-substituted alkyl, aralkyl, heteroaryl-substituted alkyl, cyano-substituted alkyl, cycloalkyl-substituted alkyl, and carboxylate-substituted alkyl. In particular embodiments, R⁴ is H, R³⁰ is H, and R³¹ is an optionally-substituted C₁-C₆ alkyl.

In certain embodiments, the racemic or enantiopure compounds disclosed herein may exist as a 'NH₂' tautomer, or a 'NH' tautomer, or a combination of both. For example, disclosed herein, but not within the literal scope of the claims is: 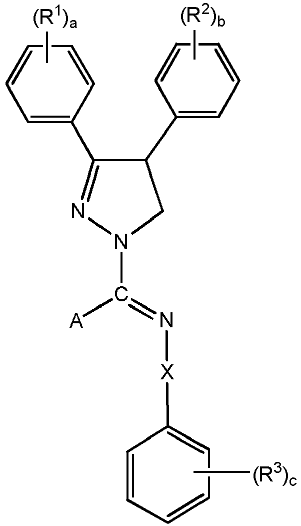 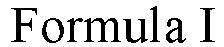 wherein A is 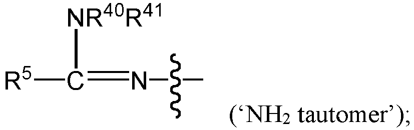 or 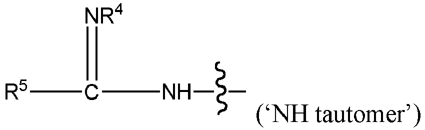
wherein R⁴ is selected from H, optionally-substituted alkyl, optionally-substituted cycloalkyl, optionally-substituted heterocycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino; R⁵ is selected from optionally-substituted alkyl, optionally-substituted cycloalkyl, halogen, cyano, nitro, hydroxy, optionally-substituted alkoxy, amino, optionally-substituted thiol, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino; and
R⁴⁰ and R⁴¹ are each independently selected from H, optionally-substituted alkyl, optionally-substituted cycloalkyl, optionally-substituted heterocycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino.

Also disclosed, R⁴⁰ and R⁴¹ are each independently selected from H, hydroxy, C₁-C₆ alkyl, or acyl (e.g., t-butyloxycarbonyl). In particular embodiments, R⁴⁰ and R⁴¹ are each H.

In particular embodiments, R⁴⁰ and R⁴¹ are each independently selected from H; and R⁵ is C₁-C₆ alkyl, or amino (e.g., -N(alkyl)₂, -NH(alkyl), or -NH(acyl) (particularly -NH(acetamido)).

An illustrative example is:

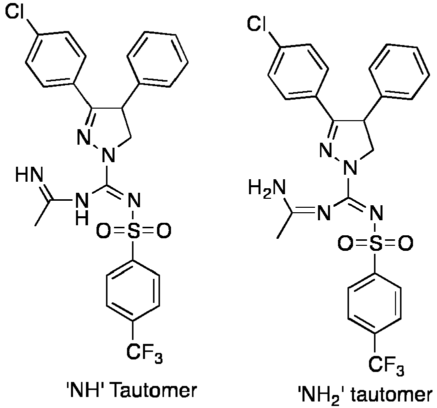

Also disclosed, but not within the literal scope of the claims, A may be a tautomer wherein the other isomer has a structure of 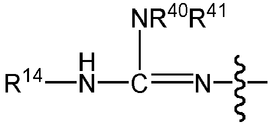 wherein R¹⁴ is H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R¹⁴ is H, acyl, or C₁-C₆ alkyl.

Also disclosed, but not within the literal scope of the claims, the amidino-containing moiety of A is a biguanidino-containing moiety having a structure of 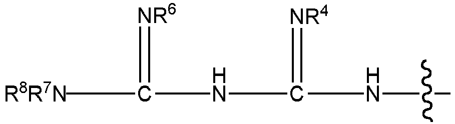 wherein R⁴, R⁶, R⁷, and R⁸ are each independently selected from H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R⁴ and R⁶ are each H. R⁷ and R⁸ are each independently selected from H or C₁-C₆ alkyl. R⁴ and R⁶ are each H, and R⁷ and R⁸ are each independently selected from H or C₁-C₆ alkyl.

Also disclosed, but not within the literal scope of the claims, A is a hydrazino-containing moiety has a structure of 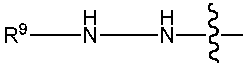 wherein R⁹ is H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R⁹ is H or C₁-C₆ alkyl.

In certain embodiments, a and c are each one, R¹ is halogen (particularly Cl), and R³ is halogen (particularly Cl). In certain embodiments, b is zero.

In certain embodiments, X is SO₂.

Also disclosed, each R²⁰ is independently H or C₁-C₆ alkyl.

Also disclosed, R¹⁰ and R¹¹ are each independently H or C₁-C₆ alkyl.

Also disclosed, R¹² is H, C₁-C₆ alkyl, aryl, or amino. Also disclosed, R¹³ is H or C₁-C₆ alkyl. Also disclosed, M is S.

In certain embodiments, A is 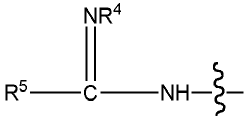 wherein R⁴ is H, and R⁵ is C₁-C₆ alkyl, C₃-C₆ cycloalkyl, amino (e.g., -N(alkyl)₂ or -NH(alkyl)), phenyl, heteroaryl (e.g., an N-heteroaryl), or heterocycloalkyl (e.g., an N-heterocycloalkyl); and X is SO₂.

More specific examples of compounds disclosed are listed below. 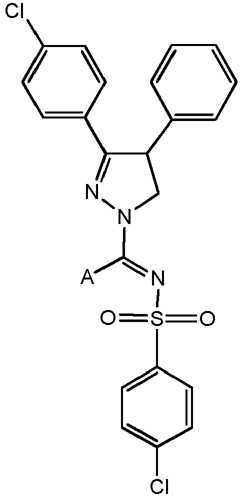 wherein A is as described herein; 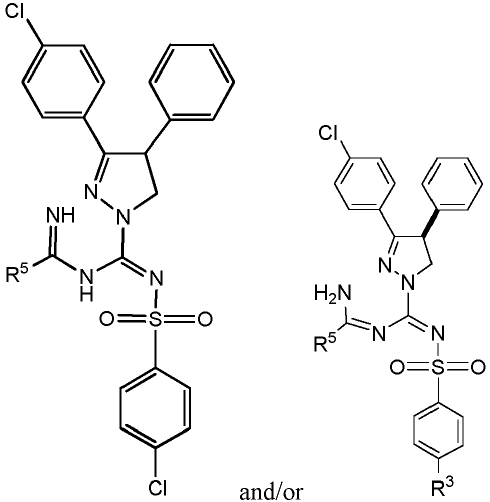 wherein R³ and R⁵ are as described herein. In certain embodiments, R⁵ is methyl, -S-methyl, - NH(acetamido), NH(Boc), or NH₂, and R³ is CF₃ or Cl.

Also disclosed, the compound is: 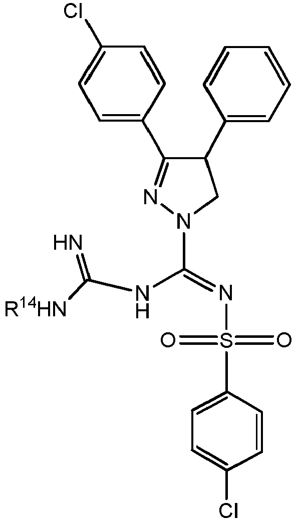 wherein R¹⁴ is H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R¹⁴ is H, acyl, or C₁-C₆ alkyl.

Also disclosed, the compound is:

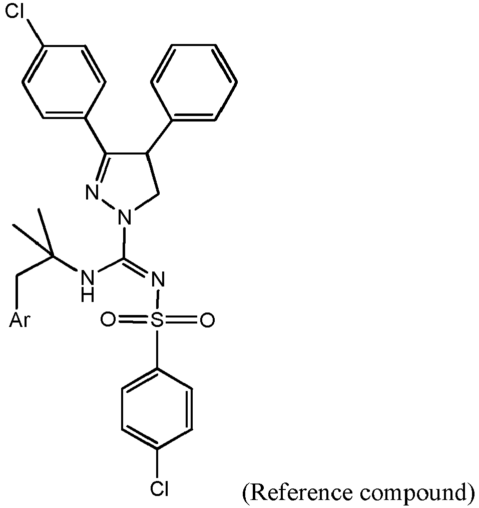

Also disclosed, the compound is: 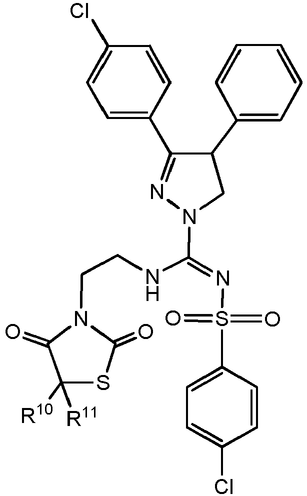 wherein R¹⁰ and R¹¹ are as described herein.

Also disclosed, the compound is: 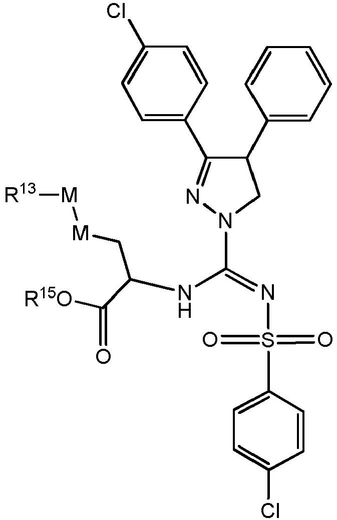 wherein R¹³ is as described herein, and R¹⁵ is H, optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino. R¹⁵ is H, C₁-C₆ alkyl, or amino.

Also disclosed, at least one of G or G' are hydroxy, optionally-substituted C₁-C₆ alkyl (e.g., phenyl-substituted C₁-C₆ alkyl), amino, or alkoxy-substituted thiol having a structure of S(CH₂)ₙOR, where R can be H, alkyl or aralkyl and n is 1 to 10.

Particular examples of the presently disclosed compounds include one or more asymmetric centers; thus these compounds can exist in different stereoisomeric forms. Accordingly, compounds and compositions may be provided as individual pure enantiomers or as stereoisomeric mixtures, including racemic mixtures. In certain embodiments the compounds disclosed herein are synthesized in or are purified to be in substantially enantiopure form, such as in a 90% enantiomeric excess, a 95% enantiomeric excess, a 97% enantiomeric excess or even in greater than a 99% enantiomeric excess, such as in enantiopure form.

The presently disclosed compounds can have at least one asymmetric center or geometric center, cis-trans center(C=C, C=N). All chiral, diasteromeric, racemic, meso, rotational and geometric isomers of the structures are intended unless otherwise specified. The compounds can be isolated as a single isomer or as mixture of isomers. All tautomers of the compounds are also considered part of the disclosure. The presently disclosed compounds also includes all isotopes of atoms present in the compounds, which can include, but are not limited to, deuterium, tritium, ¹⁸F, etc.

For example, compounds of formula I may be in the form of a stereoisomeric mixture or *cis*/*trans* isomers. In certain embodiments, the compounds of formula I may be provided as an S-enantiomer: 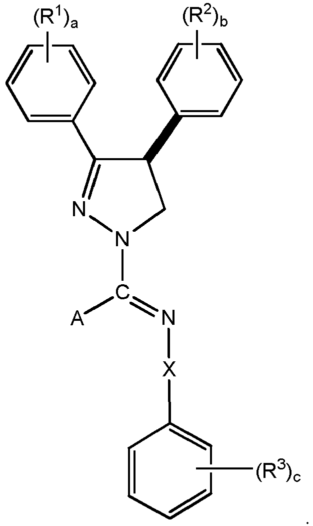 In certain embodiments, the compounds of formula I may be provided as an A-enantiomer:

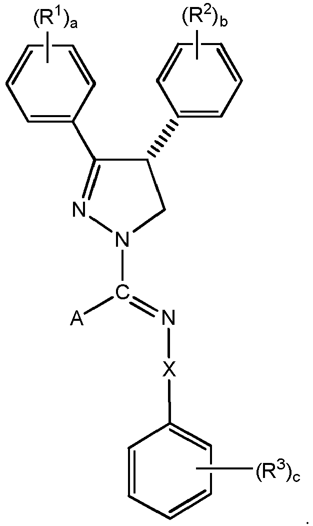

In certain embodiments, the S-enantiomer is preferred. In certain embodiments, the R-enantiomer is preferred.

In certain embodiments, the agents disclosed herein are hybrid compounds that include (i) a CB₁ receptor mediating scaffold (e.g., an inverse agonist or neutral antagonist) and (ii) a second therapeutic scaffold. The hybrid compounds may be 3-phenyl-N'-phenyl-N-imino-1H-pyrazole-1-carboximidiamide compounds. In certain embodiments, the "A" moiety in formula I constitutes at least a portion of the second therapeutic scaffold. In certain embodiments, the second therapeutic scaffold may undergo *in vivo* cleavage, thereby releasing the second therapeutic scaffold which may retain at least a portion of its therapeutic activity. For example, in the case of metformin as the second therapeutic scaffold, the resulting hybrid compound could have therapeutic efficacy not only due to its blockade of CB₁ receptors, but also due to the release of metformin, a widely used antidiabetic agent, during the *in vivo* metabolism of the compound. The *in vivo* cleavage may occur at any location in the body, but typically occurs in the liver, via the action of drug metabolizing enzymes, such as isoforms of cytochrome P450. In certain embodiments, the cleavage occurs at the bond between the "A" moiety and the C atom of the carboximidiamide portion of the compound.

Illustrative second therapeutic scaffolds include an antidiabetic agent, an anticancer agent, an antiobesity agent, and an antifibrotic agent.

The metformin scaffold is either implicit as shown below (compound 44, Table 1 and 2) or as an explicit attachment at the unsubstituted nitrogen end:

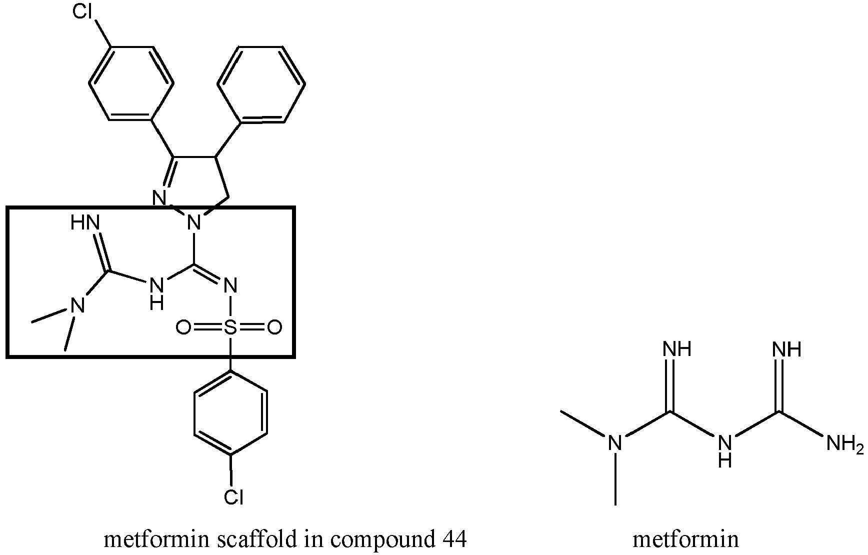

Also disclosed, a further illustrative antidiabetic scaffold is:

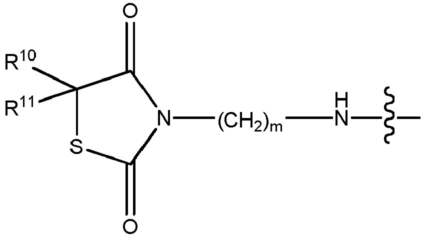

Also disclosed, an illustrative anticancer scaffold is:

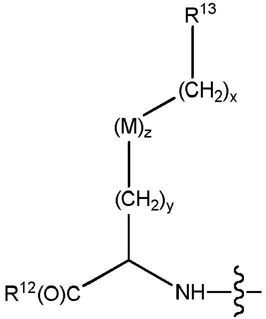

Also disclosed, an illustrative antiobesity scaffold is:

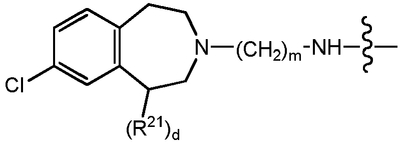

An illustrative iNOS inhibitor scaffold is 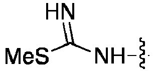 wherein R²¹ is optionally-substituted alkyl, optionally-substituted cycloalkyl, halogen, cyano, nitro, hydroxy, optionally-substituted alkoxy, amino, optionally-substituted sulfonyl, optionally-substituted aryl, optionally-substituted heteroaryl, optionally-substituted carboxyl, acyl, optionally-substituted alkenyl, optionally-substituted alkynyl, optionally-substituted phosphonyl, optionally-substituted phosphinyl, optionally-substituted boronate, optionally-substituted silyl, or imino; and d is 0 or 1.

In certain embodiments, the compounds disclosed herein have improved chemical stability resulting in a plasma half-life in the 1-16 hours range, more particularly 4-8 hours range.

In certain embodiments, the compounds disclosed herein have low or no cytochrome P450 activity meaning that the agents may result in few, if any, drug-to-drug interactions.

In certain embodiments, the compounds disclosed herein have a CB₁R binding affinity in the range of 0.1 to 20 nM, and CB₁/CB₂ selectivity of at least 20-fold, or more particularly 100-fold or greater.

Fig. 1 depicts a general synthesis method of making the compounds disclosed herein. The method involves chemically linking an amide via an imidoyl chloride to hydrazine-, amidine-, guanidine- or biguanide (e.g., metformin)-containing moieties.

### Compositions and Methods of Use

The peripherally restricted cannabinoid receptor mediating agents disclosed herein are unique in that they improve all aspects of the metabolic syndrome. They reduce food intake and body weight, reverse insulin and leptin resistance, reverse hepatic steatosis (fatty liver) and improve dyslipidemia. Also disclosed, but not within the literal scope of the claims, the compounds may be used for treating obesity, diabetes (e.g., type 2 diabetes), and non-alcoholic and alcoholic fatty liver disease (NAFLD/AFLD), the latter being a risk factor for insulin resistance, cirrhosis and liver cancer, dyslipidemias that predispose to arteriosclerotic heart disease, diabetic nephropathy, gout, and fibrosis. The compounds according to the claims are for use in the treatment of pulmonary fibrosis. The agents disclosed herein may be devoid of the psychiatric side effects that prevent the use of globally acting CB₁ antagonists.

The diabetes disorder may be Type 1 diabetes, Type 2 diabetes, inadequate glucose tolerance, and/or insulin resistance.

Also disclosed herein, but not within the literal scope of the claims, is a compound for use in a method for treating a co-morbidity of obesity. The co-morbidity may be selected from diabetes, Metabolic Syndrome, dementia, and heart disease. In further embodiments, the co-morbidity is selected from hypertension; gallbladder disease; gastrointestinal disorders; menstrual irregularities; degenerative arthritis; venous statis ulcers; pulmonary hypoventilation syndrome; sleep apnea; snoring; coronary artery disease; arterial sclerotic disease; pseudotumor cerebri; accident proneness; increased risks with surgeries; osteoarthritis; high cholesterol; and, increased incidence of malignancies of the liver, ovaries, cervix, uterus, breasts, prostrate, and gallbladder.

Also disclosed herein, but not within the literal scope of the claims, is a compound for use in a method of preventing or reversing the deposition of adipose tissue in a subject. By preventing or reversing the deposition of adipose tissue, the compounds disclosed herein are expected to reduce the incidence or severity of obesity, thereby reducing the incidence or severity of associated co-morbidities.

Also disclosed, but not within the literal scope of the claims, is a compound for use in treating scleroderma.

Further disclosed herein is a compound according to the claims, for use in a method for treating pulmonary fibrosis, particularly fibrosis resulting from Hermansky-Pudlak Syndrome. Endocannabinoids acting via CB₁R are profibrotic and proinflammatory. The brain-penetrant CB₁R antagonist, rimonabant, mitigates liver fibrosis in rodent models but it is no longer available for human use due to psychiatric side effects. In experiments, we found increased expression of endocannabinoids and CB₁R in lung samples (bronchoalveolar lavage fluid, lung tissue) from mice with bleomycin-induced pulmonary fibrosis (PF) and from patients with Hermansky-Pudlak Syndrome pulmonary fibrosis (HPSPF) and idiopathic pulmonary fibrosis (IPF). Rimonabant treatment ameliorated bleomycin-induced PF in mice, suggesting CB₁R involvement in pulmonary fibrogenesis. The activity of inducible nitric oxide synthase (iNOS) is also increased in PF, promoting lung inflammation and fibrosis progression. However the role of these targets has not yet been investigated in HPSPF, in which fibrosis is associated with alveolar inflammation.

Pirfenidone, a drug with anti-TGFβ and antioxidant properties, has recently been approved for the treatment of IPF. However, its efficacy in IPF is moderate and its efficacy in HPSPF is not known. The pathogenesis of HPSPF is complex, and targeting multiple pathways may offer improved therapeutic efficacy in this devastating disease. Disclosed herein are orally bioavailable, dual-target compounds that selectively block peripheral CB₁R due to their limited brain penetrance and also directly inhibit iNOS activity, resulting in improved efficacy relative to single target CB₁R inhibitors in rodent models of liver fibrosis.

Fibrosis results from excessive extracellular matrix deposition that accompanies chronic inflammation or wound healing, and is a key pathogenic process in many organs, including lung, liver and kidney. HPS is a rare autosomal recessive disorder affecting biogenesis in lysosome-related organelles that can arise through mutations in nine HPS genes. Patients with HPS-1, HPS-2, and HPS-4 genes are particularly susceptible to develop fatal PF with aging. Lung histopathology in HPS patients diagnosed with PF is similar to that in patients with IPF with additional features of foamy alveolar macrophages (AMs) and enlarged alveolar type II (ATII) cells. HPS-1 is the most common and severe form of HPS, and currently there is no effective treatment for HPSPF. The effects of pirfenidone, a drug approved for the treatment of IPF, are inconclusive in HPS-1 patients, and it is possible that targeting multiple pathways will be efficacious for the treatment for HPSPF.

For multifactorial chronic diseases, the conventional approach of `one disease/one target/one drug' may limit therapeutic efficacy and could be improved by simultaneously hitting multiple targets. Although this could be achieved by combination therapy, there are drawbacks to using multiple chemical entities, such as differences in pharmacokinetics, complicated dosing schedules, and a wider range of drug-drug interactions and adverse effect profiles. These issues could be mitigated by developing a single drug that addresses more than one target or biological pathway for therapeutic benefit. For instance, it has been shown that HPS patients exhibit alveolar inflammation with accumulation of activated alveolar macrophages (AM) even before fibrosis is evident. Thus, simultaneously targeting alveolar inflammation and the fibrotic process may result in improved therapeutic efficacy in HPS.

iNOS, an enzyme that catalyzes the generation of proinflammatory reactive nitrogen species involved in cell injury and apoptosis, is induced in fibrotic lung tissues in mice and humans, and the early proliferative response of human pulmonary fibroblasts to inflammatory stimuli is associated with increased iNOS gene expression. There is a strong association between iNOS protein levels and the degree of PF in human lung tissue, with low expression of iNOS being associated with reduced risk of death in PF. In a mouse model of HPS-2, iNOS expression was significantly elevated in ATII cells even before alveolar inflammation was evident, resulting in increased nitrosative stress. In the same study, increased nitrosative stress in human HPS-1 patients correlated with disease severity. In addition, generation of NO by iNOS in HPS-2 mice was much greater in alveolar macrophages than in peritoneal macrophages, marking iNOS as a key factor in AM hyper-responsiveness. Thus, iNOS is an important therapeutic target for HPSPF. iNOS inhibitors have antifibrotic activity in animal models of PF. However, iNOS inhibitors used to date in preclinical studies to inhibit fibrosis lack oral bioavailability, whereas the more recently developed, orally bioavailable iNOS inhibitors have had disappointingly low anti-inflammatory efficacy in recent clinical trials, probably due to low target organ engagement.

The endocannabinoid/CB₁R system may be another therapeutic target in PF. Endocannabinoids are lipid-signaling molecules that act on the same cannabinoid receptors - CB₁ and CB₂ - that recognize and mediate the effects of marijuana. Endocannabinoids acting via CB₁R promote fibrosis progression in multiple organs including liver, kidney, heart, and skin, and CB₁R have recently been linked to radiation-induced pulmonary fibrosis in mice. The prototype CB₁R antagonist/inverse agonist rimonabant mitigates fibrosis in animal models with moderate to low efficacy. However, it also causes neuropsychiatric side effects in human subjects due to blockade of CB₁R in the CNS, which halted the further therapeutic development of this class of compounds. Non brain-penetrant CB₁R antagonists have recently been reported to retain the metabolic benefit of globally acting compounds in experimental models of obesity and diabetes, but their therapeutic potential in different forms of fibrosis has not yet been fully evaluated. In the lung, functional CB₁R are expressed in ATII cells, AMs, and infiltrating immune cells. Injury of lung epithelial cells is believed to be critical for the initiation of the fibrotic process. Endoplasmic reticulum (ER) stress is a pro-fibrotic stimulus across different organs including the lung, and it has a role in age-related susceptibility to PF. Furthermore, ER stress and apoptosis in ATII cells contributes to fibrosis development in both IPF and HPSPF. In addition, the potential role of the NLRP3 inflammasome in pulmonary inflammation, macrophage apoptosis and fibrosis has been reported in mouse model of PF. We and others have shown that CB₁R-mediated ER stress has an obligatory role in multiple pathologies such as obesity, diabetes, cancer. In addition, we recently demonstrated that endocannabinoids acting via CB₁R activate the NLRP3-ASC inflammasome in macrophages infiltrating pancreatic islets where they trigger β-cell loss. Although CB₁R is a promising target for multiple organ fibrosis, the cellular mechanisms by which blockade of CB₁R in ATII cells and AM mitigates PF in HPS have not yet been explored and is the subject of the disclosure herein.

Disclosed herein are novel hybrid inhibitors of CB₁R and iNOS. These compounds have several features to optimize therapeutic efficacy and safety. First, the compounds have limited, or no, brain penetrance in order to minimize its potential to cause neuropsychiatric adverse effects, while retaining high CB₁R affinity and high CB₁R:CB₂R selectivity. Second, the compounds act as parent drug or pro-drug to directly inhibit iNOS activity, and the high uptake of the compound into liver, lung and kidney ensures adequate target organ exposure in fibrosis. This dual targeting strategy could result in improved antifibrotic efficacy over compounds that target only one of these molecules. These features should optimize the chance for therapeutic efficacy in HPSPF.

Also disclosed, but not within the literal scope of the claims, includes pharmaceutical compositions prepared for administration to a subject and which include a therapeutically effective amount of one or more of the compounds disclosed herein. The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Pharmaceutical compositions disclosed herein include those formed from pharmaceutically acceptable salts and/or solvates of the disclosed compounds. Pharmaceutically acceptable salts include those derived from pharmaceutically acceptable inorganic or organic bases and acids. Particular disclosed compounds possess at least one basic group that can form acid-base salts with acids. Examples of basic groups include, but are not limited to, amino and imino groups. Examples of inorganic acids that can form salts with such basic groups include, but are not limited to, mineral acids such as hydrochloric acid, hydrobromic acid, sulfuric acid or phosphoric acid. Basic groups also can form salts with organic carboxylic acids, sulfonic acids, sulfo acids or phospho acids or N-substituted sulfamic acid, for example acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, and, in addition, with amino acids, for example with α-amino acids, and also with methanesulfonic acid, ethanesulfonic acid, 2-hydroxymethanesulfonic acid, ethane-1,2-disulfonic acid, benzenedisulfonic acid, 4-methylbenzenesulfonic acid, naphthalene-2- sulfonic acid, 2- or 3-phosphoglycerate, glucose-6-phosphate or *N*-cyclohexylsulfamic acid (with formation of the cyclamates) or with other acidic organic compounds, such as ascorbic acid. In particular, suitable salts include those derived from alkali metals such as potassium and sodium, alkaline earth metals such as calcium and magnesium, among numerous other acids well known in the pharmaceutical art.

Certain compounds include at least one acidic group that can form an acid-base salt with an inorganic or organic base. Examples of salts formed from inorganic bases include salts of the presently disclosed compounds with alkali metals such as potassium and sodium, alkaline earth metals, including calcium and magnesium and the like. Similarly, salts of acidic compounds with an organic base, such as an amine (as used herein terms that refer to amines should be understood to include their conjugate acids unless the context clearly indicates that the free amine is intended) are contemplated, including salts formed with basic amino acids, aliphatic amines, heterocyclic amines, aromatic amines, pyridines, guanidines and amidines. Of the aliphatic amines, the acyclic aliphatic amines, and cyclic and acyclic di- and tri- alkyl amines are particularly suitable for use in the disclosed compounds. In addition, quaternary ammonium counterions also can be used.

Particular examples of suitable amine bases (and their corresponding ammonium ions) for use in the present compounds include, without limitation, pyridine, *N*,*N*-dimethylaminopyridine, diazabicyclononane, diazabicycloundecene, N-methyl-N-ethylamine, diethylamine, triethylamine, diisopropylethylamine, mono-, bis- or tris- (2-hydroxyethyl)amine, 2-hydroxy-*tert*-butylamine, tris(hydroxymethyl)methylamine, *N*;*N*-dimethyl-N-(2- hydroxyethyl)amine, tri-(2-hydroxyethyl)amine and *N*-methyl-D-glucamine. For additional examples of "pharmacologically acceptable salts," see Berge et al., J. Pharm. Sci. 66:1 (1977).

Compounds disclosed herein can be crystallized and can be provided in a single crystalline form or as a combination of different crystal polymorphs. As such, the compounds can be provided in one or more physical form, such as different crystal forms, crystalline, liquid crystalline or non-crystalline (amorphous) forms. Such different physical forms of the compounds can be prepared using, for example different solvents or different mixtures of solvents for recrystallization. Alternatively or additionally, different polymorphs can be prepared, for example, by performing recrystallizations at different temperatures and/or by altering cooling rates during recrystallization. The presence of polymorphs can be determined by X-ray crystallography, or in some cases by another spectroscopic technique, such as solid phase NMR spectroscopy, IR spectroscopy, or by differential scanning calorimetry.

The pharmaceutical compositions can be administered to subjects by a variety of mucosal administration modes, including by oral, rectal, intranasal, intrapulmonary, or transdermal delivery, or by topical delivery to other surfaces. Optionally, the compositions can be administered by non-mucosal routes, including by intramuscular, subcutaneous, intravenous, intra-arterial, intra-articular, intraperitoneal, intrathecal, intracerebroventricular, or parenteral routes. In other alternative embodiments, the compound can be administered *ex vivo* by direct exposure to cells, tissues or organs originating from a subject.

To formulate the pharmaceutical compositions, the compound can be combined with various pharmaceutically acceptable additives, as well as a base or vehicle for dispersion of the compound. Desired additives include, but are not limited to, pH control agents, such as arginine, sodium hydroxide, glycine, hydrochloric acid, citric acid, and the like. In addition, local anesthetics (for example, benzyl alcohol), isotonizing agents (for example, sodium chloride, mannitol, sorbitol), adsorption inhibitors (for example, Tween 80 or Miglyol 812), solubility enhancing agents (for example, cyclodextrins and derivatives thereof), stabilizers (for example, serum albumin), and reducing agents (for example, glutathione) can be included. Adjuvants, such as aluminum hydroxide (for example, Amphogel, Wyeth Laboratories, Madison, NJ), Freund's adjuvant, MPL^{™} (3-O-deacylated monophosphoryl lipid A; Corixa, Hamilton, IN) and IL-12 (Genetics Institute, Cambridge, MA), among many other suitable adjuvants well known in the art, can be included in the compositions. When the composition is a liquid, the tonicity of the formulation, as measured with reference to the tonicity of 0.9% (w/v) physiological saline solution taken as unity, is typically adjusted to a value at which no substantial, irreversible tissue damage will be induced at the site of administration. Generally, the tonicity of the solution is adjusted to a value of about 0.3 to about 3.0, such as about 0.5 to about 2.0, or about 0.8 to about 1.7.

The compound can be dispersed in a base or vehicle, which can include a hydrophilic compound having a capacity to disperse the compound, and any desired additives. The base can be selected from a wide range of suitable compounds, including but not limited to, copolymers of polycarboxylic acids or salts thereof, carboxylic anhydrides (for example, maleic anhydride) with other monomers (for example, methyl (meth)acrylate, acrylic acid and the like), hydrophilic vinyl polymers, such as polyvinyl acetate, polyvinyl alcohol, polyvinylpyrrolidone, cellulose derivatives, such as hydroxymethylcellulose, hydroxypropylcellulose and the like, and natural polymers, such as chitosan, collagen, sodium alginate, gelatin, hyaluronic acid, and nontoxic metal salts thereof. Often, a biodegradable polymer is selected as a base or vehicle, for example, polylactic acid, poly(lactic acid-glycolic acid) copolymer, polyhydroxybutyric acid, poly(hydroxybutyric acid-glycolic acid) copolymer and mixtures thereof. Alternatively or additionally, synthetic fatty acid esters such as polyglycerin fatty acid esters, sucrose fatty acid esters and the like can be employed as vehicles. Hydrophilic polymers and other vehicles can be used alone or in combination, and enhanced structural integrity can be imparted to the vehicle by partial crystallization, ionic bonding, crosslinking and the like. The vehicle can be provided in a variety of forms, including fluid or viscous solutions, gels, pastes, powders, microspheres and films for direct application to a mucosal surface.

The compound can be combined with the base or vehicle according to a variety of methods, and release of the compound can be by diffusion, disintegration of the vehicle, or associated formation of water channels. In some circumstances, the compound is dispersed in microcapsules (microspheres) or nanocapsules (nanospheres) prepared from a suitable polymer, for example, isobutyl 2-cyanoacrylate (see, for example, Michael et al., J. Pharmacy Pharmacol. 43:1-5, 1991), and dispersed in a biocompatible dispersing medium, which yields sustained delivery and biological activity over a protracted time.

The compositions of the disclosure can alternatively contain as pharmaceutically acceptable vehicles substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

Pharmaceutical compositions for administering the compound can also be formulated as a solution, microemulsion, or other ordered structure suitable for high concentration of active ingredients. The vehicle can be a solvent or dispersion medium containing, for example, water, ethanol, polyol (for example, glycerol, propylene glycol, liquid polyethylene glycol, and the like), and suitable mixtures thereof. Proper fluidity for solutions can be maintained, for example, by the use of a coating such as lecithin, by the maintenance of a desired particle size in the case of dispersible formulations, and by the use of surfactants. In many cases, it will be desirable to include isotonic agents, for example, sugars, polyalcohols, such as mannitol and sorbitol, or sodium chloride in the composition. Prolonged absorption of the compound can be brought about by including in the composition an agent which delays absorption, for example, monostearate salts and gelatin.

In certain embodiments, the compound can be administered in a time release formulation, for example in a composition which includes a slow release polymer. These compositions can be prepared with vehicles that will protect against rapid release, for example a controlled release vehicle such as a polymer, microencapsulated delivery system or bioadhesive gel. Prolonged delivery in various compositions of the disclosure can be brought about by including in the composition agents that delay absorption, for example, aluminum monostearate hydrogels and gelatin. When controlled release formulations are desired, controlled release binders suitable for use in accordance with the disclosure include any biocompatible controlled release material which is inert to the active agent and which is capable of incorporating the compound and/or other biologically active agent. Numerous such materials are known in the art. Useful controlled-release binders are materials that are metabolized slowly under physiological conditions following their delivery (for example, at a mucosal surface, or in the presence of bodily fluids). Appropriate binders include, but are not limited to, biocompatible polymers and copolymers well known in the art for use in sustained release formulations. Such biocompatible compounds are non-toxic and inert to surrounding tissues, and do not trigger significant adverse side effects, such as nasal irritation, immune response, inflammation, or the like. They are metabolized into metabolic products that are also biocompatible and easily eliminated from the body.

Exemplary polymeric materials for use in the present disclosure include, but are not limited to, polymeric matrices derived from copolymeric and homopolymeric polyesters having hydrolyzable ester linkages. A number of these are known in the art to be biodegradable and to lead to degradation products having no or low toxicity. Exemplary polymers include polyglycolic acids and polylactic acids, poly(DL-lactic acid-co-glycolic acid), poly(D-lactic acid-co-glycolic acid), and poly(L-lactic acid-co-glycolic acid). Other useful biodegradable or bioerodable polymers include, but are not limited to, such polymers as poly(epsilon-caprolactone), poly(epsilon-aprolactone-CO-lactic acid), poly(epsilon.-aprolactone-CO-glycolic acid), poly(beta-hydroxy butyric acid), poly(alkyl-2-cyanoacrilate), hydrogels, such as poly(hydroxyethyl methacrylate), polyamides, poly(amino acids) (for example, L-leucine, glutamic acid, L-aspartic acid and the like), poly(ester urea), poly(2-hydroxyethyl DL-aspartamide), polyacetal polymers, polyorthoesters, polycarbonate, polymaleamides, polysaccharides, and copolymers thereof. Many methods for preparing such formulations are well known to those skilled in the art (see, for example, Sustained and Controlled Release Drug Delivery Systems, J. R. Robinson, ed., Marcel Dekker, Inc., New York, 1978). Other useful formulations include controlled-release microcapsules (U.S. Patent Nos. 4,652,441 and 4,917,893), lactic acid-glycolic acid copolymers useful in making microcapsules and other formulations (U.S. Patent Nos. 4,677,191 and 4,728,721) and sustained-release compositions for water-soluble peptides (U.S. Patent No. 4,675,189).

The pharmaceutical compositions of the disclosure typically are sterile and stable under conditions of manufacture, storage and use. Sterile solutions can be prepared by incorporating the compound in the required amount in an appropriate solvent with one or a combination of ingredients enumerated herein, as required, followed by filtered sterilization. Generally, dispersions are prepared by incorporating the compound and/or other biologically active agent into a sterile vehicle that contains a basic dispersion medium and the required other ingredients from those enumerated herein. In the case of sterile powders, methods of preparation include vacuum drying and freeze-drying which yields a powder of the compound plus any additional desired ingredient from a previously sterile-filtered solution thereof. The prevention of the action of microorganisms can be accomplished by various antibacterial and antifungal agents, for example, parabens, chlorobutanol, phenol, sorbic acid, thimerosal, and the like.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

The administration of the compound of the disclosure can be for either prophylactic or therapeutic purpose. When provided prophylactically, the compound is provided in advance of any symptom. The prophylactic administration of the compound serves to prevent or ameliorate any subsequent disease process. When provided therapeutically, the compound is provided at (or shortly after) the onset of a symptom of disease or infection.

For prophylactic and therapeutic purposes, the compound can be administered to the subject by the oral route or in a single bolus delivery, via continuous delivery (for example, continuous transdermal, mucosal or intravenous delivery) over an extended time period, or in a repeated administration protocol (for example, by an hourly, daily or weekly, repeated administration protocol). The therapeutically effective dosage of the compound can be provided as repeated doses within a prolonged prophylaxis or treatment regimen that will yield clinically significant results to alleviate one or more symptoms or detectable conditions associated with a targeted disease or condition as set forth herein. Determination of effective dosages in this context is typically based on animal model studies followed up by human clinical trials and is guided by administration protocols that significantly reduce the occurrence or severity of targeted disease symptoms or conditions in the subject. Suitable models in this regard include, for example, murine, rat, avian, dog, sheep, porcine, feline, non-human primate, and other accepted animal model subjects known in the art. Alternatively, effective dosages can be determined using *in vitro* models. Using such models, only ordinary calculations and adjustments are required to determine an appropriate concentration and dose to administer a therapeutically effective amount of the compound (for example, amounts that are effective to alleviate one or more symptoms of a targeted disease). In alternative embodiments, an effective amount or effective dose of the compound may simply inhibit or enhance one or more selected biological activities correlated with a disease or condition, as set forth herein, for either therapeutic or diagnostic purposes.

The actual dosage of the compound will vary according to factors such as the disease indication and particular status of the subject (for example, the subject's age, size, fitness, extent of symptoms, susceptibility factors, and the like), time and route of administration, other drugs or treatments being administered concurrently, as well as the specific pharmacology of the compound for eliciting the desired activity or biological response in the subject. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. A nonlimiting range for a therapeutically effective amount of a compound and/or other biologically active agent within the methods and formulations of the disclosure is about 0.01 mg/kg body weight to about 20 mg/kg body weight, such as about 0.05 mg/kg to about 5 mg/kg body weight, or about 0.2 mg/kg to about 2 mg/kg body weight.

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, intraosseous, or intranasal delivery versus intravenous or subcutaneous or intramuscular delivery. Dosage can also be adjusted based on the release rate of the administered formulation, for example, of an intrapulmonary spray versus powder, sustained release oral versus injected particulate or transdermal delivery formulations, and so forth.

The compounds disclosed herein may also be co-administered with an additional therapeutic agent. Such agents include, but are not limited to, an antidiabetic agent, a cholesterol-lowering agent, an anti-inflammatory agent, an antimicrobial agent, a matrix metalloprotease inhibitor, a lipoxygenase inhibitor, a cytokine antagonist, an immunosuppressant, an anti-cancer agent, an antiviral agent, a cytokine, a growth factor, an immunomodulator, a prostaglandin or an anti-vascular hyperproliferation compound.

Also disclosed, but not within the literal scope of the claims, are kits, packages and multi-container units containing the herein described pharmaceutical compositions, active ingredients, and/or means for administering the same for use in the prevention and treatment of diseases and other conditions in mammalian subjects. Kits for diagnostic use are also disclosed. These kits include a container or formulation that contains one or more of the compounds described herein. In one example, this component is formulated in a pharmaceutical preparation for delivery to a subject. The compound is optionally contained in a bulk dispensing container or unit or multi-unit dosage form. Optional dispensing means can be provided, for example a pulmonary or intranasal spray applicator. Packaging materials optionally include a label or instruction indicating for what treatment purposes and/or in what manner the pharmaceutical agent packaged therewith can be used.

### Examples

Figs. 1A and 1B depict the general route to convert commercially available suitably substituted 2-phenylacetophenones to novel CB1-selective inverse agonist compounds bearing an A appendage as described herein. For example, 1-(4-chlorophenyl)-2-phenylethanone can be converted to 1-(4-chlorophenyl)-2-phenylprop-2-en-1-one using 37% formaldehyde containing piperidine and acetic acid (step a). Treatment of the acrylophenone with hydrazine hydrate in refluxing 2-propanol produces 3-(4-chlorophenyl)-4-phenyl-4,5-dihydro-1*H*-pyrazole (step b) (J. Agric. Food Chem. 1979, 27, 406). The pyrazoline was condensed with methyl (4-chlorophenyl)sulfonylcarbamate obtained from methyl chloroformate and 4-chlorobenzenesulfonamide to give the diarylpyrazoline acylsulfonamide (step c). Chlorination of this product with phosphorus pentachloride in refluxing chlorobenzene gave the imidoylchloride (step d) as previously described (J. Med CheM. 2004, 47, 627, and Che, Ber. 1966, 99, 2885). The imidoyl chloride was coupled with suitable amidine hydrochloride in (step e) the presence of triethylamine in a mixture of methanol and dichloromethane to yield dihydro-1*H*-pyrazole-1-carboximidamides. This compound can be subjected to preparative HPLC conditions using a chiral column to give *R* and *S* optically pure enantiomers. Alternatively, the racemic diarylpyrazoline acylsulfonamide can be separated on a chiral column to give optically pure enantiomeric acyl sulfonamides which can be individually subjected to further manipulations as shown in step d and e.

### Illustrative example 1:

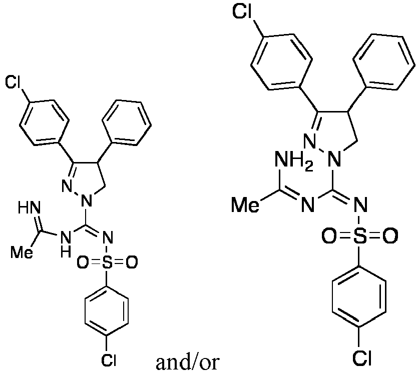

To the imidoyl chloride compound V (500 mg, 1.02 mmoles) in dichloromethane (10 mL) was added a pre-mixed mixture of acetamidine hydrochloride (3.06 mmoles) in methanol:dichloromethane:Et₃N (2:1:1) at -78°C (step e) dropwise and allowed to warm up to room temperature overnight. The reaction mixture was extracted in to dichloromethane washed with water and purified by flash chromatography using hexanes: EtOAC (6:4) to afford 3-(4-chlorophenyl)-*N*'-((4-chlorophenyl)sulfonyl)-N-(1-iminoethyl)-4-phenyl-4,5-dihydro-1*H*-pyrazole-1-carboximidamine in 30-40% yield. The racemic compound was further subjected to chiral preparatory chromatography using (R,R) WhelK-O1 to afford enantiomers E1 and E2. (Analytical: 3.2 min E1, 4.1 min E2 solvent (Hex:DCM:IPA) 40:40:20 + 0.1% TFA, Flow rate 1 ml/min)

Illustrative example 2:

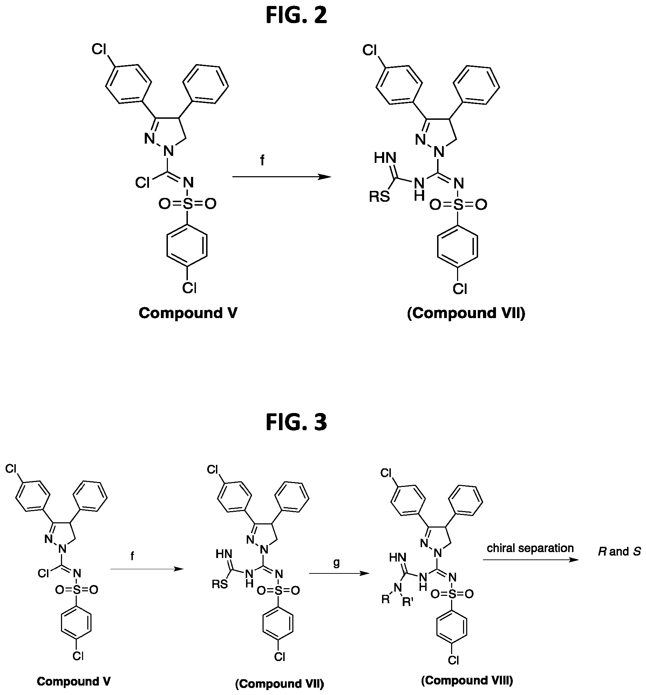

A further synthesis scheme is shown in Fig. 2. For instance, this scheme was used to synthesize

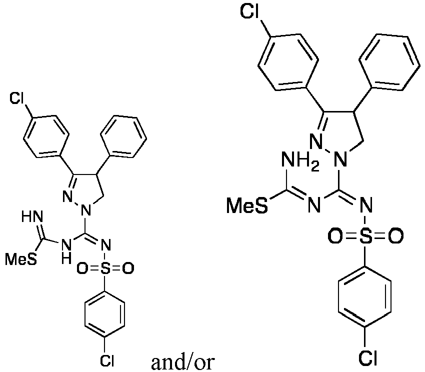

To the imidoyl chloride compound V (500 mg, 1.02 mmoles) in dichloromethane (10 mL) was added a pre-mixed mixture of S-methylisothiuronium iodide (2.04 mmoles) in methanol:dichloromethane:Et₃N (2:1:1) at -78°C (step f) dropwise and allowed to warm up to room temperature overnight. The reaction mixture was extracted in to dichloromethane washed with water and purified by flash chromatography using hexanes: EtOAC (6:4) to afford methyl-((3-(4-chlorophenyl)-4-phenyl-4,5-dihydro-1*H*-pyrazol-1yl)(((4-chlorophenyl)sulfonyl)imino)methyl)carbamimidothioate in 35-45% yield.

### Illustrative example 3:

A further synthesis scheme is shown in Fig. 3. For instance, this scheme was used to synthesize

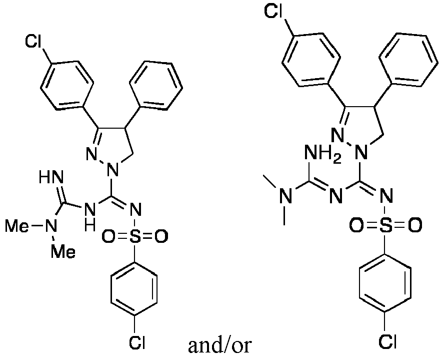

To the imidoyl chloride compound V (100 mg, 0.203 mmoles) in dichloromethane (10 mL) was added a pre-mixed mixture of S-methylisothiuronium iodide (2.04 mmoles) in methanol:dichloromethane:Et₃N (2:1:1) (step f) at -78°C dropwise and allowed to warm up to room temperature overnight. The reaction mixture was extracted in to dichloromethane washed with water and purified by flash chromatography using hexanes: EtOAC (6:4) to afford the S-methylamidino compound in 35-45% yield. To this compound in CH₃CN was added dimethyl amine and pyridine (step g) and heated at 75°C for 3h. The reaction mixture was cooled and solvent evaporated. The crude reaction mixture was washed with water extracted in to dichloromethane. The concentrated dichloromethane extract was then purified by Flash chromatography to yield 3-(4-chlorophenyl)-N'-((4-chlorophenyl)sulfonyl)-*N(N,N-*dimethylcarbamimidoyl)-4-phenyl-4,5-dihydro-1*H*-pyrazole-1-carboximidamide in 36% (40 mg) yield over two steps.

Table 1 below summarizes *in vitro* and *in vivo* data for several compounds based on the structure:

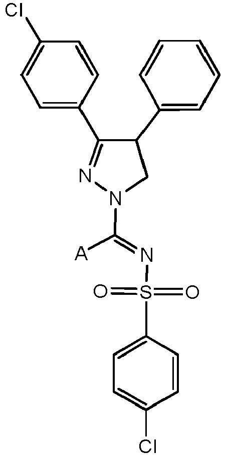

### Synthesized Compounds:

**Table 1**

| Data on synthesized compounds: | | | | | | | |
|---|---|---|---|---|---|---|---|
| Compound | A | Kᵢ CB₁ (nM) | cLogp^{a} | PSA^{a} | Brain/ Plasma | Functional GTPyS binding CB₁ IC₅₀(nM) and Eₘₐₓ (% basal) | In vivo Comments |
| 2 | 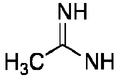 | 18 | 4.94 | 97.98 | 0.03 | 50 and -20 | -anti-diabetic |
| | | | | | | | -anti-obesity |
| 2E1 | 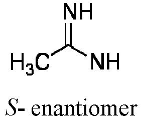 | 9 | 4.94 | 97.98 | NA | NA | NA |
| 2E2 | 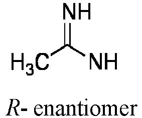 | 48 | 4.94 | 97.98 | NA | NA | NA |
| 8 | 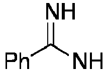 | 26 | 6.79 | 97.98 | NA | NA | NA |
| 41 | 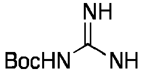 | 6.5 | 6.27 | 136.3 | NA | NA | NA |
| 73 | NH₂-NH | 33 | 4.65 | 100.1 | NA | NA | NA |

| | | | | | | | |
|---|---|---|---|---|---|---|---|
| ^{a} Theoretical values | | | | | | | |

### Additional compounds:

| | | | | | | | |
|---|---|---|---|---|---|---|---|
| 12 | 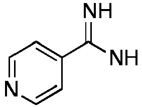 | 34 | 5.58 | 110.8 | NA | NA | NA |
| 4 | 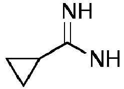 | 13 | 5.72 | 97.98 | NA | NA | NA |
| 3 | 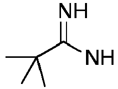 | 7 | 6.78 | 97.98 | NA | NA | NA |
| 52 | 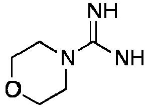 | 193 | 4.98 | 110.4 | NA | NA | NA |
| 55 | 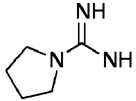 | 241 | 5.61 | 101.2 | NA | NA | NA |
| 44 | 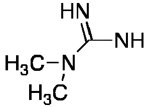 | 171 | 5.20 | 101.2 | NA | NA | NA |

Note that the A group in Table 1 may also exist as a tautomer as described above.

Compounds disclosed in Table 1 are also shown in Table 2, which includes additional compounds. They were analogously prepared as shown in Fig. 1, Fig. 2 or Fig 3:

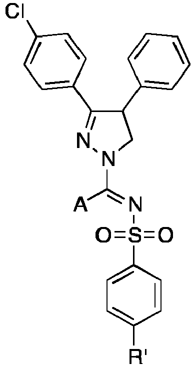

**Table 2:**

| Serial# | A | R' | Kᵢ CB₁ (nM) | ¹HNMR | [M+H]⁺ |
|---|---|---|---|---|---|
| 1 | 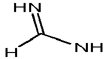 | Cl | 392 | (500 MHz, cDCl₃): δ 8.34 (s, 1H), 7.86 (d, *J* = 7.4 Hz, 2H), 7.52 (d, *J* = 7.7 Hz, 2H), 7.45 (d, *J* = 7.5 Hz, 2H), 7.41 (d, *J* = 7.6 Hz, 3H), 7.30 (d, *J* = 6.9 Hz, 2H), 7.10 (d, *J* = 7.2 Hz, 2H), 4.72 (dd, *J* = 11.5, 5.4 Hz, 1H), 4.39 (t, *J* = 11.8 Hz, 1H), 4.02-3.98 (m, 1H). | 500.1 |
| 2 | 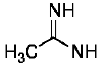 | Cl | 18 | (500 MHz, CDCl₃): δ 7.86 (d, *J* = 7.8 Hz, 2H), 7.49 (d, *J* = 7.4 Hz, 2H), 7.27 (d, *J* = 6.5 Hz, 2H), 7.20-7.18 (m, 3H), 7.07 *(d, J* = 6.7 Hz, 2H), 6.90-6.88 (m, 2H), 4.71 (dt, *J* = 6.3, 0.9 Hz, 1H), 4.49 (t, *J* = 12.0 Hz, 1H), 4.11 (t, *J* = 6.7 Hz, 1H), 2.04 (s, 3H). | 514.0 |
| 2E1 | 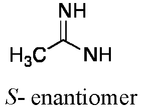 | Cl | 9 | - | 514.0 |
| 2E2 | 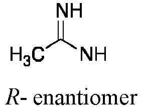 | Cl | 182 | - | 514.0 |
| 3 | 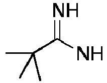 | Cl | 7 | 500 MHz, CDCl₃): δ 7.95 (d, *J* = 6.6 Hz, 2H), 7.48 (dd, *J* = 26.1, 8.3 Hz, 2H), 7.36 (d, *J* = 6.4 Hz, 2H), 7.32 (d, *J* = 7.1 Hz, 3H), 7.26 (d, *J* = 8.3 Hz, 2H), 7.20 (s, 2H), 5.52 (d, *J* = 0.4 Hz, 1H), 4.80 (s, 1H), 4.67 (s, 1H), 4.07 (dd, *J* = 12.2, 5.8 Hz, 1H), 1.32 (s, 9H). | 556.1 |
| 3E1 | 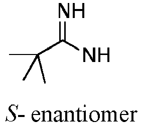 | Cl | 3.6 | - | 556. 1 |
| 4 | 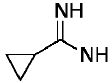 | Cl | 13 | (500 MHz, CDCl₃): δ 7.87 (d, *J* = 7.8 Hz, 2H), 7.39 (d, *J* = 8.4 Hz, 2H), 7.28 (d, *J* = 7.5 Hz, 3H), 7.24 (d, *J* = 7.4 Hz, 2H), 7.18 (d, *J* = 8.1 Hz, 2H), 7.10 (d, *J* = 6.7 Hz, 2H), 4.65 (t, *J* = 0.9 Hz, 1H), 4.48 (t, *J* = 11.6 Hz, 1H), 4.00 (dd, *J* = 10.9, 4.1 Hz, 1H), 1.25 (s, 2H), 0.98 (d, *J* = 3.6 Hz, 1H), 0.87 (t, *J* = 2.2 Hz, 2H). | 540.1 |
| 5 | 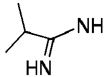 | Cl | 29 | (500 MHz, cDCl₃): δ 7.87-7.87 (m, 2H), 7.48 (d, *J* = 8.6 Hz, 2H), 7.40-7.38 (m, 2H), 7.31-7.29 (m, 3H), 7.22-7.20 (m, 2H), 7.10 (dd, *J* = 2.7, 1.1 Hz, 2H), 5.06 (s, 2H), 4.71 (dd, *J* = 2.3, 1.3 Hz, 1H), 4.50 (t, *J* = 11.9 Hz, 1H), 4.08 (d, *J* = 12.2 Hz, 1H), 2.58 (s, 1H), 1.28 (s, 6H). | 542.1 |
| 6 | 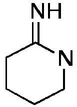 | Cl | 29 | (500 MHz, CDCl₃): δ 7.87 (d, *J* = 7.6 Hz, 2H), 7.49 (s, 2H), 7.40 (d*, J* = 8.1 Hz, 2H), 7.29 (d, *J* = 7.4 Hz, 3H), 7.20 (d, *J* = 8.3 Hz, 2H), 7.10-7.08 (m, 2H), 4.70 (s, 1H), 4.55-4.50 (m, 1H), 4.10 (dd, *J* = 12.5, 4.7 Hz, 1H), 3.41 (s, 2H), 2.46 (s, 2H), 1.77 (dd, *J* = 20.3, 0.5 Hz, 4H). | 554.1 |
| 7 | 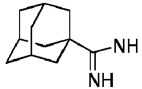 | Cl | 15 | (500 MHz, CDCl₃): δ 7.90 (d, *J* = 5.8 Hz, 2H), 7.45 (d, *J* = 8.6 Hz, 2H), 7.39 (d, *J* = 8.3 Hz, 2H), 7.30-7.26 (m, 3H), 7.20 (d, *J* = 8.3 Hz, 3H), 7.12 (s, 1H), 4.69 (t, *J* = 1.6 Hz, 1H), 4.54 (t, *J* = 11.9 Hz, 1H), 4.04 (dd, *J* = 12.3, 5.6 Hz, 1H), 2.08 (s, 4H), 1.88 (s, 5H), 1.78-1.69 (m, 5H), 1.62 (t, *J* = 0.5 Hz, 1H). | 634.2 |
| 8 | 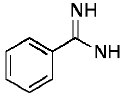 | Cl | 26 | (500 MHz, CDCl₃): δ 7.91 (d, *J* = 7.5 Hz, 2H), 7.77 (d, *J* = 7.7 Hz, 2H), 7.55 (t, *J* = 6.9 Hz, 2H), 7.43 (t, *J* = 7.4 Hz, 5H), 7.38 (d, *J* = 7.9 Hz, 2H), 7.19 (d, *J* = 8.0 Hz, 5H), 6.61 (s, 1H), 5.69 (s, 1H), 4.55-4.53 (m, 1H), 4.39-4.38 (m, 1H), 3.97-3.94 (m, 1H). | 576.04 |
| 9 | 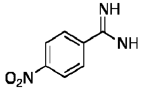 | Cl | 62 | - | 621.1 |
| 10 | 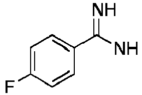 | Cl | 7.9 | (500 MHz, CDCl₃): δ 7.90 (d, *J* = 7.7 Hz, 2H), 7.80 (dd, *J* = 8.6, 5.2 Hz, 2H), 7.43-7.38 (m, 4H), 7.20 (t, *J* = 7.9 Hz, 3H), 7.10 (t, *J* = 8.5 Hz, 2H), 6.66 (s, 1H), 5.76 (s, 1H), 4.57 (d, *J* = 7.0 Hz, 1H), 4.39 (d, *J* = 3.6 Hz, 1H), 3.96 (s, 1H). | 594.04 |
| 10E1 | 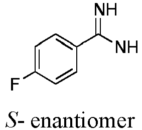 | Cl | 6.5 | - | 594.04 |
| 10E2 | 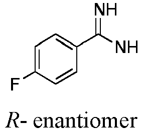 | Cl | 524 | - | 594.04 |
| 11 | 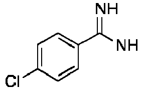 | Cl | 10 | (500 MHz, CDCl₃): δ 7.90 (d, *J* = 7.6 Hz, 2H), 7.72 (d, *J* = 8.0 Hz, 3H), 7.40-7.38 (m, 6H), 7.20 (t, *J* = 8.5 Hz, 6H), 6.61 (bs, 1H), 5.95 (s, 1H), 4.57-4.56 (m, 1H), 4.38 (t*, J* = 12.0 Hz, 1H), 3.93 (d, *J* = 3.0 Hz, 1H). | 610.9 |
| 11E1 | 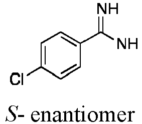 | Cl | 15 | - | 610.9 |
| 11E2 | 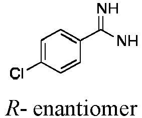 | Cl | >1000 | - | 610.9 |
| 12 | 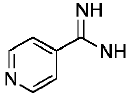 | Cl | 34 | (500 MHz, cDCl₃): δ 8.72 (s, 2H), 7.86 (d, *J* = 5.8 Hz, 2H), 7.65 (s, 2H), 7.41-7.38 (m, 6H), 7.20 (t, *J* = 7.6 Hz, 5H), 6.68 (s, 1H), 6.04 (bs, 1H), 4.60-4.58 (m, 1H), 4.42-4.38 (m, 1H), 3.95-3.93 (m, 1H). | 577.1 |
| 13 | 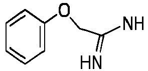 | Cl | 72 | (500 MHz, cDCl₃): δ 7.88-7.87 (m, 2H), 7.51 (d, *J* = 8.1 Hz, 2H), 7.39 (d, *J* = 8.4 Hz, 2H), 7.34-7.31 (m, 2H), 7.31-7.28 (m, 2H), 7.21 (d, *J* = 8.6 Hz, 2H), 7.07 (d, *J* = 15.4 Hz, 2H), 7.04 (t, *J =* 7.3 Hz, 2H), 6.93 (d, *J* = 8.0 Hz, 2H), 4.74 (dd, *J* = 10.1, 1.0 Hz, 3H), 4.54 (t, *J* = 12.0 Hz, 1H), 4.10 (dd, *J* = 12.4, 5.2 Hz, 1H). | 606.0 |
| 14 | 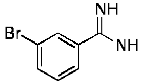 | Cl | 56 | (500 MHz, cDCl₃): δ 7.87-7.83 (m, 3H), 7.71 (d, *J* = 7.6 Hz, 1H), 7.65 (d, *J* = 8.0 Hz, 1H), 7.41 (t, *J* = 8.7 Hz, 3H), 7.29 (dd, *J* = 9.8, 6.1 Hz, 2H), 7.23 (s, 4H), 7.18 (d*, J* = 8.4 Hz, 3H), 6.82 (s, 1H), 5.84 (s, 1H), 4.64-4.62 (m, 1H), 4.47 (t, *J* = 10.4 Hz, 1H), 3.99 (d, *J* = 2.9 Hz, 1H). | 655.9 |
| 15 | 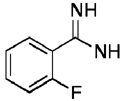 | Cl | 19 | (500 MHz, CDCl₃): δ 7.94-7.90 (m, 3H), 7.52-7.46 (m, 4H), 7.38 (d, *J* = 7.1 Hz, 3H), 7.21-7.18 (m, 5H), 7.13 (dd, *J* = 11.2, 8.4 Hz, 2H), 6.73 (s, 1H), 5.87 (s, 1H), 4.61-4.60 (m, 1H), 4.43-4.42 (m, 1H), 4.02-4.01 (m, 1H). | 594.0 |
| 16 | 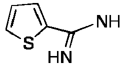 | Cl | 60 | - | 582.1 |
| 17 | 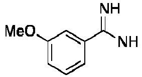 | Cl | 86 | (500 MHz, CDCl₃): δ 7.91 (d, *J* = 7.9 Hz, 2H), 7.38 (d, *J* = 8.4 Hz, 4H), 7.33-7.29 (m, 4H), 7.18 (d, *J* = 8.4 Hz, 5H), 7.06 (s, 2H), 6.61 (s, 1H), 5.87 (s, 1H), 4.52 (s, 1H), 4.37 (s, 1H), 3.95 (s, 1H), 3.75 (s, 3H). | 606.0 |
| 18 | 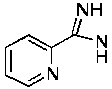 | Cl | 26 | (500 MHz, cDCl₃): δ 8.97 (s, 1H), 8.65 (d, *J* = 4.1 Hz, 2H), 8.34 (d, *J* = 7.8 Hz, 2H), 7.86 (d, *J* = 7.7 Hz, 3H), 7.51 (d, *J* = 5.7 Hz, 2H), 7.32 (t, *J* = 7.0 Hz, 3H), 7.16 (s, 4H), 4.73 (s, 1H), 4.64 (s, 1H), 4.20 (s, 1H). | 577.0 |
| 19 | 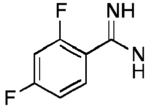 | Cl | 7.7 | (500 MHz, CDCl₃): δ 7.87 (d, *J* = 6.9 Hz, 2H), 7.45-7.44 (m, 2H), 7.37 (d, *J* = 7.6 Hz, 2H), 7.20 (d, *J* = 13.2 Hz, 8H), 6.94 (t, *J* = 7.3 Hz, 1H), 6.87 (d, *J* = 10.8 Hz, 1H), 6.76 (s, 1H), 6.21 (s, 1H), 4.63 (d, *J* = 6.3 Hz, 1H), 4.48 (d, *J* = 8.5 Hz, 1H), 3.98-3.96 (m, 1H). | 612.08 |
| 20 | 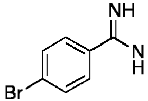 | Cl | 27 | (500 MHz, CDCl₃): δ 7.90-7.89 (m, 2H), 7.65 (d, *J* = 8.1 Hz, 3H), 7.56 (d, *J* = 8.0 Hz, 3H), 7.40 (s, 5H), 7.20 (d, *J* = 8.4 Hz, 3H), 6.63 (s, 1H), 5.77 (s, 1H), 4.55 (s, 1H), 4.37 (s, 1H), 3.94 (s, 1H). | 655.9 |
| 21 | 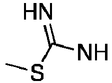 | Cl | 67 | (500 MHz, CDCl₃): δ 7.88 (d, *J* = 6.3 Hz, 2H), 7.46 (d, *J* = 8.4 Hz, 2H), 7.39 (d, *J=* 8.4 Hz, 2H), 7.27 (t, *J* = 12.4 Hz, 3H), 7.19 (d, *J* = 8.5 Hz, 2H), 7.13 (s, 2H), 4.70 (t*, J* = 0.6 Hz, 1H), 4.55 (t, *J* = 11.8 Hz, 1H), 4.05 (dd, *J* = 12.2, 5.3 Hz, 1H), 2.28 (s, 3H). | 546.1 |
| 22 | 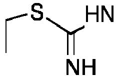 | Cl | 67 | (500 MHz, cDCl₃): δ 7.90-7.88 (m, 2H), 7.48 (d, *J* = 8.5 Hz, 2H), 7.40 (d, *J* = 8.3 Hz, 2H), 7.30 (d, *J* = 7.4 Hz, 2H), 7.26 (d, *J* = 7.0 Hz, 1H), 7.21 (d, *J* = 8.5 Hz, 2H), 7.14 (t, *J* = 0.5 Hz, 2H), 4.70 (d, *J* = 10.3 Hz, 1H), 4.54 (t, *J* = 11.8 Hz, 1H), 4.09 (dd, *J* = 12.2, 5.1 Hz, 1H), 2.83 (s, 2H), 1.31 (t, *J* = 7.1 Hz, 4H). | 560.07 |
| 23 | 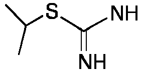 | Cl | 166 | (500 MHz, CDCl₃): δ 7.90-7.89 (m, 2H), 7.48 (d, *J* = 7.6 Hz, 2H), 7.41 (d, *J* = 7.6 Hz, 2H), 7.31 (d, *J* = 6.8 Hz, 2H), 7.21 (d, *J* = 7.7 Hz, 2H), 7.12 (s, 3H), 5.12 (bs, 1H), 4.70-4.68 (m, 1H), 4.52-4.50 (m, 1H), 4.13-4.09 (m, 1H), 3.23 (m, 1H), (1.37-1.33 (m, 6H). | 574.0 |
| 24 | 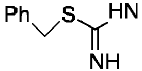 | Cl | 127 | (500 MHz, CDCl₃): δ 7.88 (t, *J* = 1.1 Hz, 2H), 7.38 (d, *J* = 8.1 Hz, 4H), 7.31 (s, 5H), 7.17 (d, *J* = 8.4 Hz, 4H), 7.10 (s, 3H), 4.71 (s, 1H), 4.57-4.55 (m, 1H), 4.06-3.99 (m, 3H). | 622.08 |
| 25 | 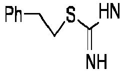 | Cl | 287 | (500 MHz, CDCl₃): δ 7.89 (d, *J* = 1.6 Hz, 2H), 7.47 (d, *J* = 7.8 Hz, 2H), 7.37 (d, *J* = 6.9 Hz, 2H), 7.30 (d, *J* = 6.8 Hz, 3H), 7.23-7.22 (m, 3H), 7.18 (d, *J* = 8.1 Hz, 3H), 7.11 (s, 3H), 4.70 (s, 1H), 4.54 (t, *J* = 11.6 Hz, 1H), 4.11-4.08 (m, 1H), 3.03 (s, 2H), 2.97 (s, 2H). | 636.1 |
| 26 | 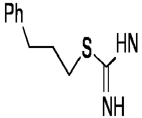 | Cl | 190 | (500 MHz, cDCl₃): δ 7.86 (s, 2H), 7.39 (d, *J* = 18.6 Hz, 3H), 7.29 (s, 3H), 7.20 (d, *J* = 6.6 Hz, 2H), 7.17 (d, *J* = 6.4 Hz, 2H), 7.10 (s, 6H), 4.71 (s, 1H), 4.56-4.51 (m, 1H), 4.06 (s, 1H), 2.72 (s, 1H), 2.64 (d, *J* = 5.9 Hz, 2H), 1.97 (s, 3H). | 650.1 |
| 27 | 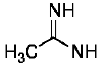 | F | 60 | (500 MHz, cDCl₃): δ 7.95-7.93 (m, 2H), 7.51 (d, *J* = 8.0 Hz, 2H), 7.29 (d, *J* = 7.0 Hz, 2H), 7.21 (d, *J* = 8.3 Hz, 3H), 7.09 (t, *J* = 7.3 Hz, 4H), 5.29 (bs, 2H), 4.71 (dd, *J* = 11.4, 4.9 Hz, 1H), 4.49 (t, *J* = 11.9 Hz, 1H), 4.11-4.09 (m, 1H), 2.08 (s, 3H). | 498.1 |
| 28 | 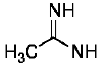 | Br | 18 | (500 MHz, CDCl₃): δ 7.80 (d, *J* = 7.5 Hz, 2H), 7.55 (d*, J* = 8.3 Hz, 2H), 7.51 (d*, J* = 8.5 Hz, 2H), 7.30 (d, *J* = 7.6 Hz, 3H), 7.22 (d, *J* = 8.5 Hz, 2H), 7.09-7.08 (m, 2H), 5.11 (bs, 2H), 4.71 (dt, *J* = 6.3, 0.9 Hz, 1H), 4.49 (t, *J* = 12.0 Hz, 1H), 4.11 (t, *J =* 6.7 Hz, 1H), 2.08 (s, 3H). | 558.03 |
| 29 | 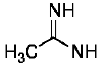 | I | 10 | 500 MHz, CDCl₃): δ 7.77 (d, *J* = 8.0 Hz, 2H), 7.67-7.64 (m, 2H), 7.51 (d, *J* = 7.7 Hz, 2H), 7.30 (s, 3H), 7.23-7.21 (m, 2H), 7.09-7.08 (m, 2H), 5.14 (s, 1H), 4.71 (d, *J* = 8.0 Hz, 1H), 4.51-4.46 (m, 1H), 4.10 (d, *J* = 10.6 Hz, 1H), 2.08 (s, 3H). | 606.1 |
| 30 | 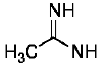 | H | 114 | (500 MHz, CDCl₃): δ 7.94 (d, *J* = 7.1 Hz, 2H), 7.50 (d, *J* = 8.3 Hz, 2H), 7.46 (d, *J* = 6.7 Hz, 2H), 7.42 (t, *J* = 7.2 Hz, 2H), 7.30 (t, *J* = 7.1 Hz, 2H), 7.21 (d, *J* = 8.4 Hz, 2H), 7.09-7.08 (m, 2H), 5.19 (bs, 1H), 4.72-4.69 (m, 1H), 4.50 (t, *J* = 12.0 Hz, 1H), 4.13-4.10 (m, 1H), 2.05 (s, 3H). | 480.1 |
| 31 | 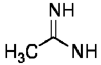 | CH₃ | 87 | (500 MHz, CDCl₃): δ 7.81 (d, *J* = 7.8 Hz, 2H), 7.47 (d, *J* = 8.5 Hz, 2H), 7.25-7.22 (m, 3H), 7.18 (dd, *J* = 12.0, 8.4 Hz, 4H), 7.06 (d, *J* = 7.0 Hz, 2H), 5.82 (s, 1H), 4.68 (dd, *J* = 11.2, 4.8 Hz, 1H), 4.49 (t, *J* = 11.3 Hz, 1H), 4.06 (t, *J =* 6.6 Hz, 1H), 2.35 (s, 3H), 2.00 (s, 3H). | 494.1 |
| 32 | 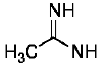 | OCH₃ | 182 | (500 MHz, CDCl₃): δ 7.86 (d, *J* = 7.8 Hz, 2H), 7.49 (d, *J* = 7.4 Hz, 2H), 7.27 (d, *J* = 6.5 Hz, 3H), 7.20-7.18 (m, 2H), 7.07 *(d, J* = 6.7 Hz, 2H), 6.90-6.88 (m, 2H), 4.70-4.67 (m, 1H), 4.51-4.46 (m, 1H), 4.07 (s, 1H), 3.82 (s, 3H), 2.03 (s, 3H). | 510.1 |
| 33 | 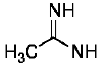 | - (C₄H₈)- | 8 | (500 MHz, CDCl₃): δ 8.48 (s, 1H), 7.96 (d, *J* = 8.4 Hz, 1H), 7.92-7.91 (m, 1H), 7.87 (t, *J* = 7.4 Hz, 2H), 7.58-7.52 (m, 2H), 7.48 (d, *J* = 8.4 Hz, 3H), 7.28 (s, 2H), 7.18 (d, *J* = 7.7 Hz, 2H), 7.08 (d, *J* = 6.9 Hz, 2H), 5.22 (s, 1H), 4.70-4.69 (m, 1H), 4.51 (t, *J* = 12.0 Hz, 1H), 4.14-4.11 (m, 1H), 2.04 (s, 3H). | 530.1 |
| 34 | 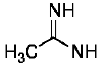 | CF₃ | 5.7 | (500 MHz, CDCl₃): δ 8.05 (d, *J* = 5.7 Hz, 2H), 7.69-7.68 (m, 2H), 7.50 (d, *J* = 8.0 Hz, 2H), 7.29 (d, *J* = 7.2 Hz, 2H), 7.21 (d, *J* = 8.0 Hz, 3H), 7.09-7.08 (m, 2H), 4.73-4.71 (m, 1H), 4.51 (t, *J* = 11.5 Hz, 1H), 3.87 (d, *J* = 5.1 Hz, 1H), 2.09 (s, 3H). | 548.1 |
| 35 | 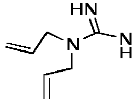 | Cl | 263 | (500 MHz, CDCl₃): δ 7.93 (d, *J* = 8.4 Hz, 2H), 7.40 (d, *J* = 8.4 Hz, 2H), 7.31 (t, *J* = 7.5 Hz, 2H), 7.24 (s, 3H), 7.18 (d, *J* = 7.4 Hz, 2H), 7.14 (d*, J* = 8.5 Hz, 2H), 5.76 (td, *J* = 11.1, 5.6 Hz, 2H), 5.23-5.20 (m, 4H), 4.56 (dd, *J* = 11.3, 4.6 Hz, 1H), 4.48 (t, *J* = 11.5 Hz, 1H), 4.04 (dd, *J* = 11.8, 4.6 Hz, 1H), 3.98-3.89 (m, 4H). | 595.1 |
| 36 | 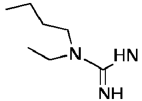 | Cl | 535 | (500 MHz, CDCl₃): δ 7.94 (d, *J* = 8.3 Hz, 2H), 7.40 (d*, J* = 8.4 Hz, 2H), 7.31-7.29 (m, 3H), 7.19 (d, *J* = 0.9 Hz, 3H), 7.13 (d, *J* = 8.5 Hz, 3H), 4.56-4.53 (m, 1H), 4.50-4.47 (m, 1H), 4.05-4.02 (m, 1H), 3.40-3.29 (m, 2H), 3.38-3.29 (m, 2H), 1.84 (m, 4H), 0.91 (s, 3H), 0.88 (s, 3H). | 599.1 |
| 37 | 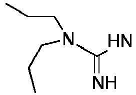 | Cl | 138 | (500 MHz, cDCl₃): δ 7.95-7.93 (m, 2H), 7.41-7.39 (m, 2H), 7.30 (dd, *J* = 5.0, 1.9 Hz, 3H), 7.20-7.18 (m, 4H), 7.13 (d, *J* = 6.8 Hz, 2H), 4.54 (d, *J* = 11.5 Hz, 1H), 4.47 (t*, J* = 11.5 Hz, 1H), 4.05-4.02 (m, 1H), 3.30 (m, 2H), 3.18 (m, 2H), 1.30-1.28 (m, 2H), 1.17 (d, *J* = 24.7 Hz, 2H), 0.89-0.88 (m, 6H). | 599.1 |
| 38 | 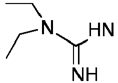 | Cl | 49 | (500 MHz, CDCl₃): δ 7.93 (d, *J* = 8.3 Hz, 2H), 7.40-7.38 (m, 2H), 7.29 (d, *J* = 7.4 Hz, 2H), 7.24-7.23 (m, 2H), 7.17 (d, *J* = 7.8 Hz, 2H), 7.11 (d, *J* = 8.2 Hz, 3H), 4.55-4.52 (m, 1H), 4.46 (t, *J* = 11.4 Hz, 1H), 4.03-4.00 (m, 1H), 3.30-3.27 (m, 2H), 3.20-3.16 (m, 2H), 0.86 (t, *J* = 5.7 Hz, 6H). | 571.1 |
| 39 | 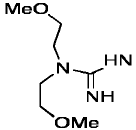 | Cl | 425 | (500 MHz, CDCl₃): δ 7.93 (d, *J* = 8.5 Hz, 2H), 7.39 (d, *J* = 8.5 Hz, 2H), 7.30 (d, *J* = 7.3 Hz, 4H), 7.25 (q, *J* = 8.4 Hz, 2H), 7.15 (dd, *J* = 11.0, 8.4 Hz, 3H), 4.56 (dd, *J* = 11.2, 4.7 Hz, 1H), 4.45 (t, *J* = 11.6 Hz, 1H), 4.04-4.01 (m, 1H), 3.54 (d, *J* = 3.5 Hz, 8H), 3.30 (s, 6H). | 631.1 |
| 40 | 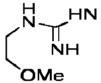 | Cl | 848 | (500 MHz, CDCl₃): δ 7.93 (d, *J* = 8.4 Hz, 2H), 7.40 (d, *J* = 8.4 Hz, 2H), 7.29 (t, *J* = 7.4 Hz, 3H), 7.24 (d, *J* = 7.2 Hz, 1H), 7.18 (d, *J* = 0.6 Hz, 2H), 7.13 (t, *J* = 8.0 Hz, 3H), 4.55 (dd, *J* = 11.3, 4.5 Hz, 1H), 4.44 (t, *J =* 11.7 Hz, 1H), 4.03 (dd, *J* = 12.0, 4.6 Hz, 1H), 3.44 (d, *J* = 4.4 Hz, 2H), 3.34 (d, *J* = 7.8 Hz, 2H), 3.31 (s, 3H). | 573.1 |
| 41 | 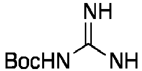 | Cl | 6.5 | (500 MHz, CDCl₃): δ 8.02 (d, *J* = 8.4 Hz, 2H), 7.60 (d, *J* = 8.6 Hz, 2H), 7.49 (d, *J* = 8.5 Hz, 2H), 7.38 (d, *J* = 7.6 Hz, 3H), 7.34-7.31 (m, 2H), 7.26 (dd, *J* = 14.0, 8.0 Hz, 2H), 4.71 (dd, *J* = 11.2, 4.9 Hz, 1H), 4.55 (t, *J* = 11.7 Hz, 1H), 4.16 (dd, *J* = 12.2, 4.9 Hz, 1H), 1.54 (s, 9H). | 615.07 |
| 42 | 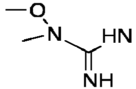 | Cl | 23 | (500 MHz, CDCl₃): δ 7.93 (d, *J* = 8.4 Hz, 2H), 7.40 (d, *J* = 8.4 Hz, 2H), 7.31 (t, *J* = 7.5 Hz, 2H), 7.24 (s, 2H), 7.18 (d, *J* = 7.4 Hz, 3H), 7.14 (d*, J* = 8.5 Hz, 2H), 4.56 (dd, *J* = 11.3, 4.6 Hz, 1H), 4.48 (t, *J* = 11.5 Hz, 1H), 4.04 (dd, *J* = 11.8, 4.6 Hz, 1H), 3.59 (s, 3H), 3.02 (s, 3H). | 559.4 |
| 43 | 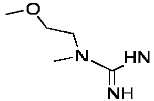 | Cl | 908 | (500 MHz, cDCl₃): δ 7.95-7.93 (m, 2H), 7.39 (d, *J* = 8.1 Hz, 2H), 7.31-7.29 (m, 3H), 7.18-7.14 (m, 6H), 4.56-4.54 (m, 1H), 4.49-4.45 (m, 1H), 4.05 (d, *J* = 7.2 Hz, 1H), 3.54 (s, 2H), 3.49-3.47 (m, 2H), 3.34 (s, 3H), 3.00 (s, 3H). | 587.1 |
| 44 | 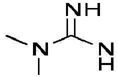 | Cl | 171 | (500 MHz, CDCl₃): δ 7.92 (d, *J* = 8.4 Hz, 2H), 7.40 (d, *J* = 8.4 Hz, 2H), 7.29 (t, *J* = 7.4 Hz, 2H), 7.23 (d, *J* = 7.2 Hz, 1H), 7.15 (d, *J* = 7.3 Hz, 3H), 7.10 (s, 4H), 4.53 (dd, *J* = 11.3, 4.5 Hz, 1H), 4.46 (t, *J* = 11.5 Hz, 1H), 4.02 (dd, *J* = 11.7, 4.5 Hz, 1H), 2.98 (s, 6H). | 543.1 |
| 45 | 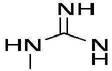 | Cl | 327 | (500 MHz, CDCl₃): δ 7.90 (d, *J* = 8.3 Hz, 2H), 7.40 (d, *J* = 8.5 Hz, 2H), 7.29 (d, *J* = 7.0 Hz, 2H), 7.23 (d, *J* = 7.3 Hz, 1H), 7.13-7.09 (m, 6H), 4.54 (dd, *J* = 11.2, 4.6 Hz, 1H), 4.44 (t, *J* = 11.7 Hz, 1H), 4.01 (dd, *J* = 11.9, 4.6 Hz, 1H), 2.73 (d, *J* = 2.4 Hz, 3H). | 529.1 |
| 46 | 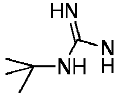 | Cl | 49 | (500 MHz, CDCl₃): δ 7.92 (d, *J* = 8.2 Hz, 2H), 7.41 (d, *J* = 8.1 Hz, 2H), 7.30 (t, *J* = 7.2 Hz, 3H), 7.14 (d, *J* = 7.4 Hz, 3H), 7.10 (s, 3H), 4.54 (t, *J* = 5.5 Hz, 1H), 4.49 (t, *J* = 11.3 Hz, 1H), 4.05-4.02 (m, 1H), 1.30 (s, 9H). | 571.1 |
| 47 | 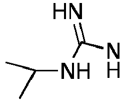 | Cl | 516 | (500 MHz, CDCl₃): δ 7.92 (d, *J* = 8.4 Hz, 2H), 7.41 (d, *J* = 8.4 Hz, 2H), 7.31-7.22 (m, 3H), 7.14 (d, *J* = 7.3 Hz, 3H), 7.10 (d, *J* = 7.2 Hz, 3H), 4.54 (dd, *J* = 11.2, 4.5 Hz, 1H), 4.45 (t, *J* = 11.6 Hz, 1H), 4.03 (dd, *J* = 11.9, 4.6 Hz, 1H), 3.82 (s, 1H), 1.11 (dd, *J* = 9.8, 6.6 Hz, 6H). | 557.0 |
| 48 | 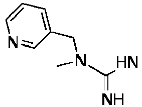 | Cl | 147 | (500 MHz, CDCl₃): δ 8.54-8.51 (m, 3H), 7.89 (d, *J* = 8.4 Hz, 2H), 7.67 (s, 1H), 7.38-7.32 (m, 3H), 7.23 (d, *J* = 8.6 Hz, 5H), 7.10 (d, *J* = 25.6 Hz, 3H), 4.67-4.66 (m, 3H), 4.44 (s, 1H), 4.10 (d, *J* = 8.4 Hz, 1H), 3.10 (s, 3H). | 620.1 |
| 49 | 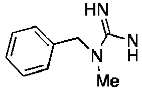 | Cl | 343 | (500 MHz, CDCl₃): δ 7.93-7.91 (m, 2H), 7.36 (d, *J* = 7.0 Hz, 2H), 7.27 (d, *J* = 11.6 Hz, 7H), 7.20 (d, *J* = 7.7 Hz, 4H), 7.13 (t, *J* = 8.1 Hz, 3H), 4.74 (d, *J* = 15.2 Hz, 1H), 4.51 (d, *J* = 11.5 Hz, 1H), 4.45 (dd, *J* = 20.1, 7.6 Hz, 2H), 4.01-3.98 (m, 1H), 2.94 (s, 3H). | 619.1 |
| 50 | 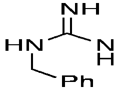 | Cl | 505 | (500 MHz, CDCl₃): 7.81 (d, *J* = 8.3 Hz, 2H), 7.32-7.22 (m, 14H), 7.09 (d, *J* = 7.1 Hz, 2H), 4.51-4.28 (m, 4H), 3.97-3.94 (m, 1H) | 605.1 |
| 51 | 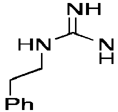 | Cl | 931 | (500 MHz, CDCl₃): δ 7.89 (d, *J* = 7.7 Hz, 2H), 7.40 (d, *J* = 7.5 Hz, 2H), 7.29 (d, *J* = 6.9 Hz, 2H), 7.20 (d, *J* = 6.3 Hz, 5H), 7.14-7.07 (m, 7H), 4.55-4.53 (m, 1H), 4.43 (t*, J* = 11.4 Hz, 1H), 4.03-4.00 (m, 1H), 3.41-3.39 (m, 2H), 2.77 (d, *J* = 6.8 Hz, 2H). | 619.1 |
| 52 | 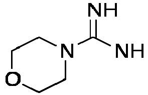 | Cl | 193 | (500 MHz, CDCl₃): δ 7.90 (d, *J* = 8.5 Hz, 2H), 7.41 (d, *J* = 8.5 Hz, 2H), 7.30 (t, J= 7.4 Hz, 2H), 7.23 (t, *J* = 7.4 Hz, 2H), 7.14-7.07 (m, 7H), 4.53 (dd, *J* = 11.2, 4.6 Hz, 1H), 4.45 (t, *J* = 11.5 Hz, 1H), 4.01 (dd, *J* = 11.7, 4.6 Hz, 1H), 3.66 (t, *J* = 4.5 Hz, 4H), 3.51 (d, *J* = 4.4 Hz, 4H). | 585.1 |
| 53 | 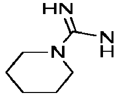 | Cl | 276 | (500 MHz, CDCl₃): δ 7.92 (d, *J* = 8.2 Hz, 2H), 7.38 (d, *J* = 8.2 Hz, 2H), 7.29-7.26 (m, 4H), 7.22 (t, *J* = 7.3 Hz, 2H), 7.13 (t, *J* = 9.5 Hz, 3H), 4.54 (t, *J* = 5.6 Hz, 1H), 4.47 (t, *J* = 11.5 Hz, 1H), 4.02 (dd, *J* = 11.7, 4.6 Hz, 1H), 3.43 (s, 4H), 1.62 (d, *J* = 4.3 Hz, 2H), 1.55 (s, 4H). | 583.1 |
| 54 | 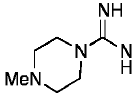 | Cl | 309 | (500 MHz, CDCl₃): δ 7.91 (d, *J* = 8.2 Hz, 2H), 7.40 (d, *J* = 8.2 Hz, 2H), 7.30 (t, *J* = 7.4 Hz, 3H), 7.25 (s, 1H), 7.14 (dd, *J=* 13.8, 8.2 Hz, 5H), 4.55 (dd, *J* = 11.2, 4.7 Hz, 1H), 4.47 (t, *J* = 11.5 Hz, 1H), 4.02 (dd, *J* = 11.7, 4.7 Hz, 1H), 3.51 (s, 4H), 2.39 (s, 4H), 2.30 (s, 3H). | 598.1 |
| 55 | 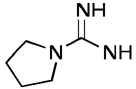 | Cl | 241 | (500 MHz, CDCl₃): δ 7.93 (d, *J* = 8.2 Hz, 2H), 7.39 (d, *J* = 7.8 Hz, 2H), 7.28-7.22 (d, *J* = 7.0 Hz, 3H), 7.15 (d, *J* = 7.5 Hz, 2H), 7.09 (s, 4H), 4.52 (dd, *J* = 11.2, 4.5 Hz, 1H), 4.44 (t, *J* = 11.6 Hz, 1H), 4.01 (dd, *J* = 11.9, 4.5 Hz, 1H), 3.36 (m, 4H), 1.89 (m, 4H). | 569.1 |
| 56 | 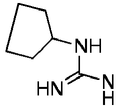 | Cl | 459 | (500 MHz, CDCl₃): δ 7.94 (d, *J* = 8.5 Hz, 2H), 7.41 (d, *J* = 8.5 Hz, 2H), 7.29 (d, *J* = 7.6 Hz, 3H), 7.23 (s, 1H), 7.16 (d, *J* = 7.3 Hz, 3H), 7.13 (d*, J* = 8.3 Hz, 2H), 4.56-4.53 (m, 1H), 4.45 (t, *J* = 11.5 Hz, 1H), 4.06-4.03 (m, 1H), 3.78 (m, 1H), 1.88-1.86 (m, 2H), 1.63 (s, 2H), 1.53 (dd, *J* = 4.3, 1.1 Hz, 2H), 1.41-1.38 (m, 2H). | 583.1 |
| 57 | 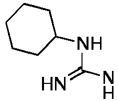 | Cl | 419 | (500 MHz, CDCl₃): δ 7.93 (d, *J* = 8.3 Hz, 2H), 7.41 (d, *J* = 8.3 Hz, 2H), 7.30 (t, *J* = 7.2 Hz, 3H), 7.23 (d, *J* = 7.0 Hz, 1H), 7.15 (d, *J* = 7.5 Hz, 3H), 7.13-7.11 (m, 2H), 4.56-4.53 (m, 1H), 4.44 (t, *J* = 11.5 Hz, 1H), 4.05-4.02 (m, 1H), 3.45 (m, 1H), 1.85-1.83 (m, 2H), 1.66-1.65 (m, 2H), 1.58 (m, 4H), 1.13-1.11 (m, 2H). | 597.1 |
| 58 | 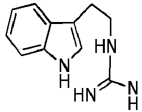 | Cl | 585 | (500 MHz, CDCl₃): δ 8.10 (s, 1H), 7.84 (d, *J* = 7.9 Hz, 2H), 7.49 (d, *J* = 7.7 Hz, 1H), 7.30 (dt, *J* = 13.2, 8.0 Hz, 7H), 7.18-7.14 (m, 2H), 7.14-7.11 (m, 3H), 7.04 (d, *J* = 9.2 Hz, 2H), 4.49-4.47 (m, 1H), 4.36-4.32 (m, 1H), 3.99-3.96 (m, 1H), 3.46-3.42 (m, 2H), 2.94-2.91 (m, 2H). | 658.1 |
| 59 | 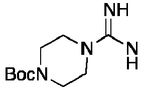 | Cl | 387 | (500 MHz, CDCl₃): δ 7.92 (d, *J* = 8.3 Hz, 2H), 7.41 (s, 2H), 7.30 (d, *J* = 7.3 Hz, 3H), 7.16 (t, *J* = 8.3 Hz, 6H), 4.59-4.56 (m, 1H), 4.49-4.44 (m, 1H), 4.03 (dd, *J* = 11.9, 4.7 Hz, 1H), 3.49-3.48 (m, 8H), 1.46 (s, 9H). | 684.1 |
| 60 | 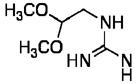 | Cl | 443 | 500 MHz, CDCl₃): δ 7.92 (d, *J* = 7.3 Hz, 2H), 7.41-7.40 (m, 2H), 7.31-7.28 (m, 3H), 7.24 (s, 2H), 7.14 (d, *J* = 4.6 Hz, 4H), 4.57-4.55 (m, 1H), 4.45 (t, *J* = 11.6 Hz, 1H), 4.40 (s, 1H), 4.04 (dd, *J* = 11.5, 4.0 Hz, 1H), 3.37 (d, *J* = 6.5 Hz, 2H), 3.32 (s, 6H). | 603.1 |
| 61 | 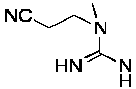 | Cl | 203 | (500 MHz, CDCl₃): δ 7.90 (d, *J* = 8.3 Hz, 2H), 7.41 (d, *J* = 8.4 Hz, 2H), 7.30 (d, *J* = 7.4 Hz, 2H), 7.16 (d, *J* = 17.8 Hz, 3H), 7.14 (d, *J* = 7.7 Hz, 4H), 4.58 (dd, *J* = 10.9, 4.5 Hz, 1H), 4.47 (t, *J* = 11.5 Hz, 1H), 4.35 (m, 1H), 3.99 (dd, *J* = 11.8, 4.8 Hz, 1H), 3.5 (m, 1H), 3.13 (s, 3H), 2.66 (d, *J* = 5.7 Hz, 2H). | 582.1 |
| 62 | 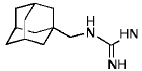 | Cl | >1000 | (500 MHz, CDCl₃): δ 7.93 (d, J= 8.5 Hz, 2H), 7.41 (d, *J* = 8.4 Hz, 2H), 7.30-7.28 (m, 4H), 7.16 (d, *J* = 7.1 Hz, 2H), 7.12 (d, *J* = 7.1 Hz, 3H), 4.56-4.52 (m, 1H), 4.47-4.42 (m, 1H), 4.07-4.04 (m, 1H), 2.83-2.75 (m, 1H), 2.74 (d, *J* = 8.9 Hz, 1H), 1.95 (s, 4H), 1.70-1.68 (m, 5H), 1.60 (s, 3H), 1.30-1.25 (m, 3H). | 663.2 |
| 63 | 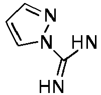 | Cl | 11 | (500 MHz, CDCl₃): δ 7.91 (s, 2H), 7.68 (s, 1H), 7.47-7.43 (m, 4H), 7.40-7.30 (m, 4H), 7.17 (s, 5H), 6.43 (s, 1H), 4.75 (s, 2H), 4.14 (d, *J* = 16.7 Hz, 1H). | 566.0 |
| 64 | 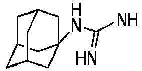 | Cl | >1000 | (500 MHz, CDCl₃): δ 7.94 (d, *J* = 7.8 Hz, 2H), 7.41 (d, *J* = 7.9 Hz, 2H), 7.32-7.28 (m, 4H), 7.16 (dd, *J* = 14.3, 7.8 Hz, 5H), 4.58-4.55 (m, 1H), 4.49-4.45 (m, 1H), 4.09-4.05 (m, 1H), 2.04 (s, 6H), 1.83 (m, 5H), 1.66-1.64 (m, 4H). | 649.2 |
| 65 | 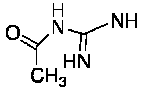 | Cl | 9 | (500 MHz, CDCl₃): δ 7.92 (d, *J* = 7.2 Hz, 2H), 7.47 (d, *J* = 7.9 Hz, 2H), 7.38 (d, *J* = 7.6 Hz, 2H), 7.30 (d, *J* = 21.1 Hz, 3H), 7.19 (d, *J* = 8.1 Hz, 2H), 7.11-7.10 (m, 2H), 4.56-4.50 (m, 2H), 4.04-4.01 (m, 1H), 1.86 (s, 3H). | 557.0 |
| 66 | 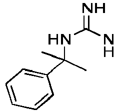 | Cl | 112 | (500 MHz, CDCl₃): δ 7.96 (d, *J* = 7.5 Hz, 2H), 7.40 (t, *J* = 9.3 Hz, 7H), 7.30-7.29 (m, 4H), 7.15 (d, *J* = 7.8 Hz, 5H), 4.52 (s, 2H), 4.40-4.34 (m, 1H), 4.01-3.98 (m, 1H), 1.51 (s, 11H). | 633.2 |
| 67 | 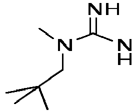 | Cl | 2390 | (500 MHz, CDCl₃): δ 7.94 (d, *J* = 8.4 Hz, 2H), 7.40 (d, *J* = 8.4 Hz, 2H), 7.33-7.30 (m, 3H), 7.19 (d, *J* = 7.2 Hz, 3H), 7.14 (d, *J* = 8.5 Hz, 3H), 4.57-4.54 (m, 1H), 4.48 (t, *J* = 11.4 Hz, 1H), 4.07-4.04 (m, 1H), 3.10 (s, 1H), 3.07 (s, 1H), 3.02 (s, 3H), 0.97-0.94 (m, 9H). | 599.2 |
| 68 | 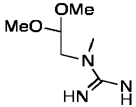 | Cl | 443 | (500 MHz, CDCl₃): δ 7.94 (d, *J* = 8.2 Hz, 2H), 7.40 (d*, J* = 8.3 Hz, 2H), 7.30-7.28 (m, 5H), 7.18-7.14 (m, 4H), 4.58-4.55 (m, 1H), 4.49-4.46 (m, 2H), 4.06-4.04 (m, 1H), 3.47 (s, 1H), 3.40 (s, 6H), 3.02 (s, 3H). | 617.1 |
| 69 | 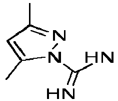 | Cl | 10 | (500 MHz, CDCl₃): δ 7.91 (dd, *J* = 1.5, 0.5 Hz, 2H), 7.51-7.50 (m, 2H), 7.39 (s, 2H), 7.35-7.29 (m, 4H), 7.20 (d, *J* = 8.0 Hz, 3H), 5.99 (s, 1H), 4.71-4.69 (m, 1H), 4.56 (t*, J* = 11.5 Hz, 1H), 4.12 (dd, *J* = 11.8, 4.4 Hz, 1H), 2.43 (s, 3H), 2.23 (s, 3H). | 594.1 |
| 70 | 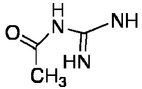 | I | 3.2 | (500 MHz, CDCl₃): δ 7.80 (d, *J* = 8.1 Hz, 2H), 7.73 (d, *J* = 8.0 Hz, 2H), 7.43 (d, *J* = 8.3 Hz, 2H), 7.33 (t, *J* = 7.3 Hz, 2H), 7.28 (s, 1H), 7.20 (d, *J* = 8.5 Hz, 2H), 7.15 (d, *J* = 7.0 Hz, 2H), 4.61-4.59 (m, 1H), 4.49 (t, *J* = 11.6 Hz, 1H), 4.07 (dd, *J* = 12.9, 4.9 Hz, 1H), 1.95 (s, 3H). | 637.0 |
| 71 | 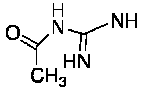 | - (C₄H₈)- | 16 | (400 MHz, CDCl₃): δ 8.54 (s, 1H), 8.04 (d, *J* = 8.0 Hz, 1H), 7.92-7.89 (m, 3H), 7.42 (m, 2H), 7.48 (d, *J* = 8.4 Hz, 2H), 7.29 (m, 3H), 7.15 (d, *J* = 7.7 Hz, 2H), 7.05 (d, *J* = 6.9 Hz, 2H), 4.59-4.57 (m, 1H), 4.51 (t, *J* = 12.0 Hz, 1H), 4.04-3.99 (m, 1H), 1.78 (s, 3H). | 562.1 |
| 72 | 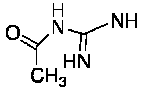 | CF₃ | 2.7 | (400 MHz, CDCl₃): δ 8.14 (d, *J* = 8.0 Hz, 2H), 7.73 (d, *J* = 8.0 Hz, 2H), 7.38 (d, *J* = 8.3 Hz, 2H), 7.33 (m, 3H), 7.15 (d, *J* = 8.0 Hz, 4H), 4.63 (dd, *J* = 12, 5.0 Hz, 1H), 4.50 (t, *J* = 12.0 Hz, 1H), 4.09 (dd, J= 11.8,4.8 Hz, 1H), 1.94 (s, 3H). | 580.0 |
| 73 | NH₂NH | Cl | 33 | - | 488.1 |
| 74 | 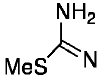 | CF₃ | 59 | - | 579.08 |
| 75 | 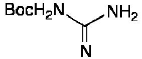 | CF₃ | - | - | 648.1 |
| 76 | 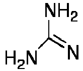 | CF₃ | - | - | 548.1 |
| 77 | 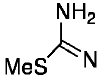 | I | 6 | - | 636.1 |
| 78 | 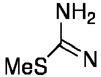 | - (C₄H₈)- | 29 | - | 561.1 |

Note that the A group in Table 2 may also exist as a tautomer as described above.

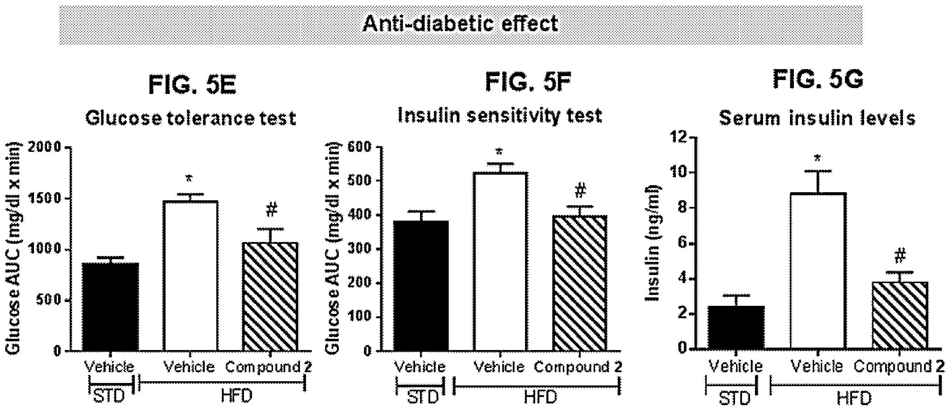

Using radioligand displacement assay in mouse brain membranes, the Ki of one of the enantiomers of compound 2 (compound 2E1) is 9 nM. Measuring its tissue levels 1 hour after administration of 10 mg/kg to mice, plasma levels were comparable after oral or i.p. administration (indicating good oral bioavailability), and brain tissue level was < 2% of plasma level, indicating low brain penetrance/peripheral selectivity. 1 h after oral administration of compound 2 at 10 mg/kg dose in mice, the metabolite generation in plasma was monitored by LC-MS/MS. As expected, this compound underwent in vivo metabolism to liberate the amidine moiety and a metabolite (structure 4IV in the Fig. 1). Besides CB₁R antagonism, both intact compound and its metabolically cleaved amidine moiety were able to inhibit iNOS activity about 48% and 37% at 1 µM concentration in lung homogenates from LPS-treated mouse (Fig 4 ). Mice with diet-induced obesity (DIO) mice were orally treated for 14 days with the compound 2 (10 mg/kg/day). The results are shown in Figs. 5A-5G. The compound 2 reduced body weight (Fig. 5A), food intake (Fig. 5B), hyperleptinemia (Fig. 5C), hepatic TG (Fig. 5D) and abrogated HFD-induced glucose intolerance (Fig. 5E), insulin resistance (Fig. 5F), and hyperinsulinemia (Fig. 5G). Data represent mean ± SEM from 5-6 mice per group. **(P<0.05),* indicate significant difference from (STD) diet control. ^{#} indicates significant treatment effect *(P<0.05)* relative to vehicle-treated HFD group. Food intake was reduced by ~ 20% during the first week and body weight was progressively reduced by ~10% relative to vehicle-treated DIO mice, but remained significantly higher than the weight of lean mice on regular diet. Glucose tolerance and insulin sensitivity were determined on the last 2 days of treatment, using i.p. glucose tolerance and insulin sensitivity tests. DIO mice show glucose intolerance (blood glucose following an i.p. glucose load of 1.5 g/kg rises higher and takes longer to return to baseline than in lean mice) and insulin resistance (reduction of blood glucose by insulin is attenuated). In compound 2-treated DIO mice, both of these parameters were nearly completely normalized.

The *Zucker diabetic fatty* (ZDF) rat is a commonly used animal model of type 2 diabetes with progressive β-cell loss resulting in extreme hyperglycemia. Recently, we showed that peripheral CB₁R antagonism prevents β-cell loss by blocking CB₁R in infiltrating, proinflammatory macrophages. (Jourdan et al, Nature Med 2013, 19-(9):1132-1140). This highlights the therapeutic potential of peripheral CB₁R antagonists in type 2 diabetes. The compound 2 prevented the increase in blood glucose, and the parallel decline in insulin and c-peptide levels in ZDF rats (Fig 6) which indicates prevention of β-cell loss as reported recently (Jourdan et al, Nature Med 2013, 19-(9): 1132-1140).

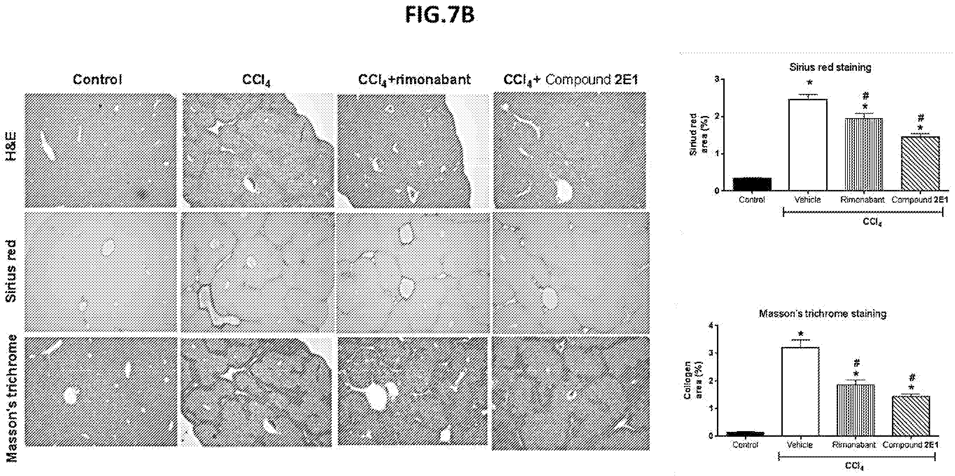

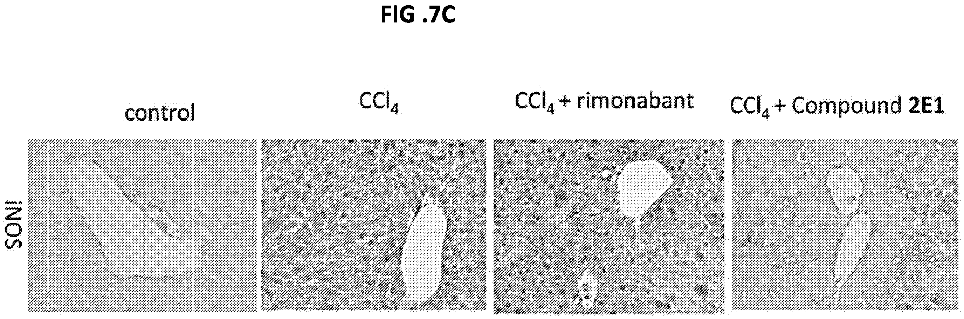

Fibrosis results from excessive extracellular matrix deposition by myofibroblasts accompanying chronic inflammation and wound healing, and is a key pathogenic process in many organs, including kidneys, lung, and liver. Since the prototype CB₁R antagonist rimonabant was reported to have an anti-fibrotic effect in mouse models of liver fibrosis (Teixeira-Clerc, Nature Med 2006 12(6);671-676) we also used CCl₄-induced liver fibrosis model in mice to assess the in vivo efficacy of compound 2. In order to compare its efficacy with that of rimonabant, we treated mice either with rimonabant or compound 2E1 (Fig 7). Compound 2E1 was more effective than rimonabant in reducing CCl₄-induced collagen deposition in liver as shown by Sirius red and Masson's trichrome stainings (Fig 7B,D). Importantly, CCl₄-induced elevation of iNOS immunostaining was dramatically attenuated by the compound 2E1 but not by rimonabant (Fig 7C). This may indicate that dual targeting on CB₁R and iNOS by compound 2E1 results in higher in vivo efficacy. Overall, compound 2E1 showed higher anti-fibrotic efficacy than rimonabant in liver fibrosis.

Activators of AMP-activated protein kinase (AMPK), such as guanidines of biguanides such as metformin are useful for the treatment of diabetes owing to their AMPK activating property (Hardie, et al., Chem & Biol 2012, 19(10); 1222-1236). Guanidine analogues of certain embodiments were screened for AMPK activation by using a recombinant AMPK activity assay. The compounds activated AMPK to various extents, whereas rimonabant had no effect on AMPK activity at 1 µM (Fig 8).

### Increased endocannabinoid AEA levels in BALFs from patients with IPF and HPSPF

Endocannabinoid levels are increased locally in certain organs and/or in the systemic circulation in pathologies such as obesity, diabetes, liver fibrosis. Eventually, this results in overactivity of the endocannabinoid/CB₁R system that contributes to pathogenesis. However, this phenomenon has not yet been investigated in lung fibrosis. Human specimens collected from healthy volunteers (NV), patients with either IPF or HPSPF, and HPS patients without PF were analyzed. The levels of the endocannabinoids arachidonoyl ethanolamide (AEA) and 2-arachidonyl glycerol (2AG) in BALF and plasma of these subjects were measured by using Liquid Chromatography coupled Tandem Mass Spectrometry (LC-MS/MS), with the person conducting the analyses being blinded to the identity of the samples. In BALF, AEA was significantly elevated in both IPF and HPSPF relative to NV, whereas there was no change in AEA level in HPS without PF. Increased AEA level in BALF was negatively correlated with pulmonary function tests (PFT) in these patients. On the other hand, neither AEA nor 2AG levels changed in plasma samples of IPF patients.

### Overexpression of CB₁R protein in fibrotic lung tissues removed during lung transplantation

CB₁R protein levels were evaluated by immunohistochemistry in lung tissue samples from IPF and HPSPF patients removed during lung transplantation, and it was found that CB₁R protein is dramatically increased in both IPF and HPSPF lungs. These results, taken together with increased AEA levels (Fig. 10), suggest an overactive endocannabinoid/CB₁R system in human PF.

### Overactivity of endocannabinoid/CB₁R system and iNOS in bleomycin-induced PF in mice.

The endocannabinoid/CB₁R system in a murine model of fibrosis was analyzed using bleomycin-induced PF in C57BL/6J mice. After intratracheal installation of bleomycin (1U/kg), lung fibrosis was evident histologically on day 7 post-bleomycin (Fig. 12A,B), and further progression was evident by day 14 and 18 post-bleomycin, with increased mortality rate (Fig. 12).

BALF was collected from these mice for endocannabinoid measurements and gene expression analyses. AEA but not 2AG was significantly and progressively increased in both BALF and lung tissue from mice with bleomycin-induced PF (Fig. 13A, B), similar to our findings in human IPF and HPSPF (Fig. 10A).

mRNA levels for both CB₁R and iNOS were determined by real-time PCR in lung tissue and in cells isolated from BALF (Fig. 13 C, D). CB₁R and iNOS mRNA were significantly increased both in lung and BALF cells at day 18 post-bleomycin (Fig. 13C, D). Interestingly, at the earlier 7-day time point, CB₁R mRNA was increased in BALF cells but not in lung, whereas an early increase in iNOS was evident in lung but not in BALF cells. Assuming that the majority of the infiltrating immune cells were collected and represented in BALF cells, these findings are compatible with the idea that infiltrating pro-inflammatory cells induce increased CB₁R expression in lung tissue to promote PF. The progressive increase of CB₁R protein in bleomycin-induced PF in mice is similar to finding in human IPF lung, and support the translational relevance of this mouse model. Furthermore, these data are compatible with the involvement of CB₁R and iNOS activity in the pathogenesis of PF, with distinct roles of different cell populations, and justify the dual targeting of CB₁R and iNOS as anti-fibrotic strategy in PF.

### CB₁R^{-/}⁻ mice is protected against bleomycin-induced pulmonary fibrosis

In a recent study, pharmacological inhibition or genetic deletion of CB₁R was found to decrease radiation-induced pulmonary fibrosis in mice and improved survival of mice following thoracic irradiation . Genetic deletion of the CB₁R gene resulted in improved survival rate following bleomycin treatment (1U/kg). Although sample size was very low (n:4), mortality in CB₁R^{-/-} mice up to 14 days was not observed(Fig. 14). Bleomycin-induction of TGFβ1 mRNA expression was dramatically attenuated in CB₁R^{-/-} mice. These preliminary data are compatible with the postulated deleterious role of CB₁R overactivation in lung fibrosis, and further justifies testing CB₁R antagonists for the treatment of PF.

### Localization of CB₁R on macrophages in bleomycin-induced PF

In a recent study, CB₁R antagonism significantly reduced immune cell infiltration and proinflammatory cytokine expression in lung tissue in a radiation-induced PF mouse model. Our data on bleomycin-induced fibrosis also support the evidence and further suggest that CB₁R on infiltrating immune cells have a pro-inflammatory role since CB₁R and endocannabinoid levels are progressively elevated in infiltrating cells and BALF (Fig. 13). Alveolar macrophages may contribute to the development of PF. Therefore, we analyzed the co-localization of CB₁R and CD68 (macrophage marker) (Fig. 15). Both CB₁R and CD68⁺macrophages are significantly elevated in bleomycin-induced PF, with significant co-localization being evident. CB₁R expressed in alveolar macrophages may thus contribute to lung inflammation and fibrogenesis. The role of CB₁R in pro-inflammatory macrophages in beta-cell failure in type-2 diabetes via activating the Nlrp3 inflammasome was previously demonstrated. There is also evidence that activated Nlrp3 inflammasome in alveolar macrophages contributes to lung inflammation and the initiation and progression of PF. We measured *Nlrp3* mRNA after 14 days oral treatment with rimonabant (3mg/kg/day). Interestingly, CB₁R antagonism almost completely attenuated the bleomycin-induced increase of *Nlrp3* expression in lung tissue.

### Survival rate is significantly increased by dual inhibition of peripheral CB₁R and iNOS in mice with bleomycin-induced PF

Mice instilled with bleomycin were subjected to one of 2 treatment paradigms: oral treatment with an equipotent CB₁R inhibitory dose of rimonabant or MRI-1867 (3 mg/kg/day) was started either simultaneously with or 7 days after bleomycin instillation, to asses prevention or regression of PF, respectively, as illustrated in Fig. 16

In the prevention paradigm, both rimonabant and MRI-1867 significantly increased survival rate, with MRI-1867 treated mice having higher survival rate (85%) compared to the rimonabant treated group (64%). In the fibrosis regression paradigm, only MRI-1867 caused a significant increase in survival rate (67%, compared 25% in the vehicle group and 45% in the rimonabant group.

MRI-1867 is the S -(-)-enantiomer of the compound in Table 2, Serial #34. The structure based on X-ray is as below:

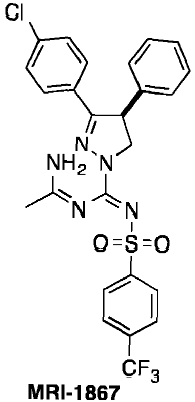

## Claims

1. A compound, or a tautomer thereof, or a pharmaceutically acceptable salt or ester thereof, for use in the treatment of pulmonary fibrosis in a subject, wherein the compound is of the formula: 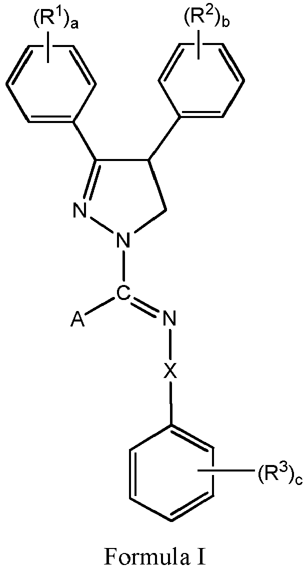 wherein
X is SO₂;
b is zero;
a and c are each independently 0, 1, 2, 3, 4 or 5;
R¹ and R³ are each independently selected from C₁-C₆ alkyl, substituted C₁-C₆ alkyl, halogen, C₁-C₈ alkoxy and substituted C₁-C₈ alkoxy;
A is an amidino-containing moiety having the structure of: 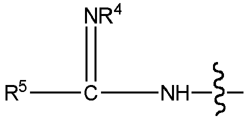
wherein
R⁴is H;
R⁵ is C₁-C₆ alkyl, C₃-C₆ cycloalkyl, -NH₂, -N(C₁-C₆ alkyl)₂, -NH(C₁-C₆ alkyl), phenyl, a monocyclic or bicyclic heteroaryl that includes at least one nitrogen heteroatom, or a monocyclic or bicyclic heterocycloalkyl that includes at least one nitrogen heteroatom; or
R⁵ is C₁-C₆ alkyl, -N(C₁-C₆ alkyl)₂, -NH(C₁-C₆ alkyl), or -NHC(O)CH₃; or
R⁵ is an amino of the formula -NR³⁰R³¹, where R³⁰ and R³¹ are, independently, hydrogen or an optionally-substituted C₁-C₆ alkyl, provided that at least one of R³⁰ and R³¹ is optionally substituted C₁-C₆ alkyl; or
R⁵ is a thiol substituted with a C₁-C₆ alkyl or a substituted C₁-C₆ alkyl;
wherein substituted C₁-C₆ alkyl is a C₁-C₆ alkyl wherein one of more hydrogen atoms are substituted with halogen, cycloalkyl, C₁-C₈ alkoxy, -NH₂, hydroxyl, aryl, alkenyl with up to 6 carbon atoms or carboxyl; and
substituted C₁-C₈ alkoxy is C₁-C₈ alkoxy substituted with alkenyl with up to 6 carbon atoms, alkynyl with up to 6 carbon atoms, aryl, aryl-C₁-C₆ alkyl, cycloalkyl, halogenated C₁-C₆ alkyl, or C₁-C₈ alkoxy.

2. The compound for the use of claim 1, wherein R⁵ is C₁-C₆ alkyl, C₃-C₆ cycloalkyl, - N(C₁-C₆ alkyl)₂, -NH(C₁-C₆ alkyl)), phenyl, a monocyclic or bicyclic heteroaryl that includes at least one nitrogen heteroatom, or a monocyclic or bicyclic heterocycloalkyl that includes at least one nitrogen heteroatom.

3. The compound for the use of claim 1, wherein R⁵ is an amino of the formula - NR³⁰R³¹, where R³⁰ and R³¹ are, independently, hydrogen or an optionally-substituted C₁-C₆ alkyl, provided that at least one of R³⁰ and R³¹ is optionally substituted C₁-C₆ alkyl.

4. The compound for the use of claim 1, wherein, a and c are each one, R¹ is halogen, and R³ is halogen.

5. The compound for the use of claim 1, wherein the compound is of the formula: 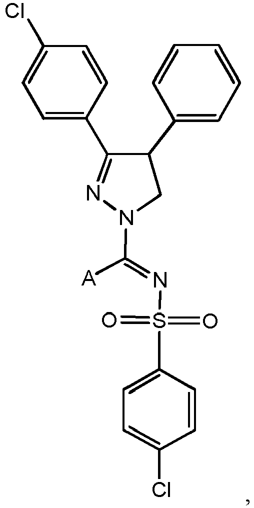 or a tautomer thereof, or a pharmaceutically acceptable salt or ester thereof.

6. The compound for the use of claim 1, wherein the compound is of the formula: 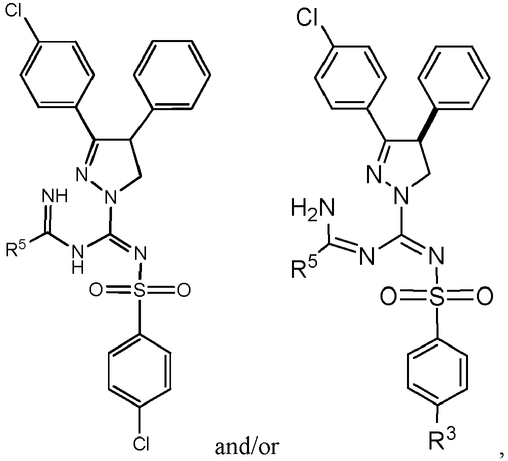 or a tautomer thereof, or a pharmaceutically acceptable salt or ester thereof.

7. The compound for the use of claim 1, wherein the compound is of the formula: 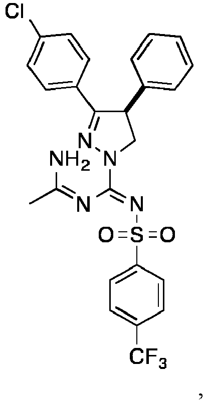 or a tautomer thereof, or a pharmaceutically acceptable salt thereof.

8. The compound for the use of claim 1, wherein the compound is of the formula: 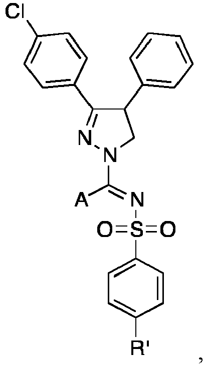 or a tautomer thereof, or a pharmaceutically acceptable salt thereof,
wherein A is 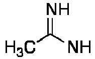 and R' is Cl.

9. The compound for the use of claim 1, wherein the compound is of the formula: 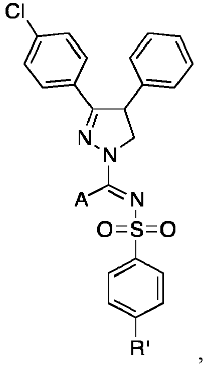 or a tautomer thereof, or a pharmaceutically acceptable salt thereof,
wherein A is 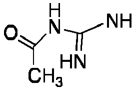 and R' is Cl.

10. The compound for the use of claim 1, wherein the compound is of the formula: 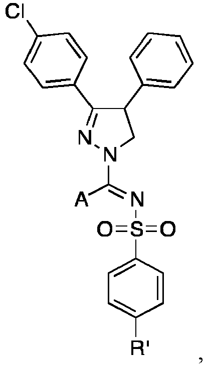 or a tautomer thereof, or a pharmaceutically acceptable salt thereof,
wherein A is 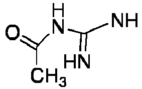 and R' is CF₃.

11. A compound or a tautomer thereof, or a pharmaceutically acceptable salt thereof, for use in the treatment of pulmonary fibrosis in a subject, wherein the compound is of the formula: 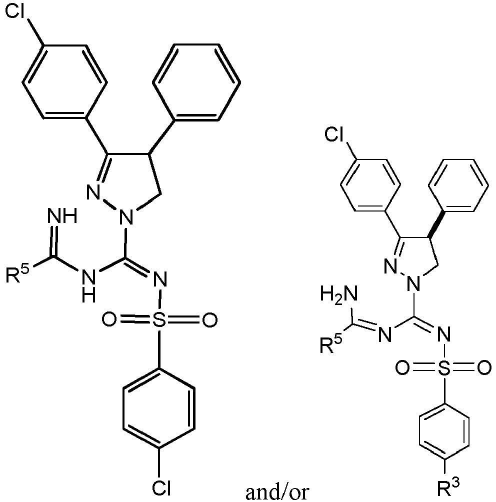
wherein R⁵ is methyl, -S-methyl, -NHC(O)CH₃, NH(Boc), or NH₂; and
R³ is CF₃ or Cl.

12. A compound or a tautomer thereof, or a pharmaceutically acceptable salt thereof, for use in the treatment of pulmonary fibrosis in a subject, wherein the compound is of the formula: 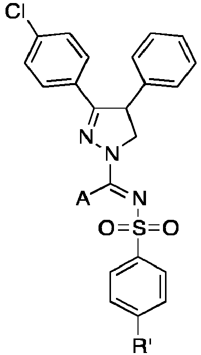 wherein the compound has an A and R' group as indicated in the table below:
| Serial# | A | R' |
|---|---|---|
| 1 | 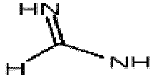 | Cl |
| 2E1 | 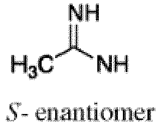 | Cl |
| 2E2 | 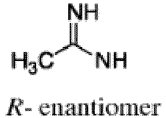 | Cl |
| 3 | 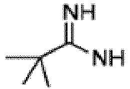 | Cl |
| 3E1 | 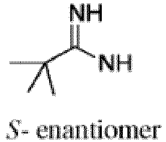 | Cl |
| 4 | 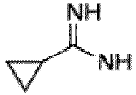 | Cl |
| 5 | 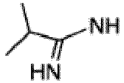 | Cl |
| 6 | 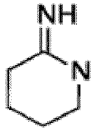 | Cl |
| 7 | 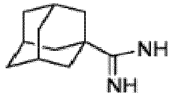 | Cl |
| 8 | 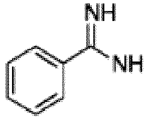 | Cl |
| 9 | 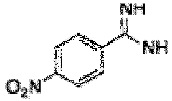 | Cl |
| 10 | 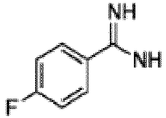 | Cl |
| 10E1 | 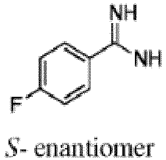 | Cl |
| 10E2 | 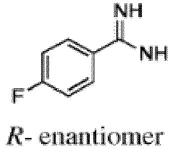 | Cl |
| 11 | 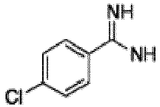 | Cl |
| 11E1 | 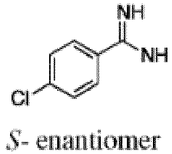 | Cl |
| 11E2 | 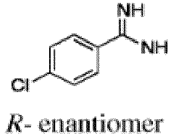 | Cl |
| 12 | 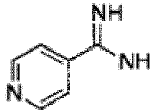 | Cl |
| 13 | 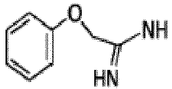 | Cl |
| 14 | 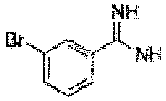 | Cl |
| 15 | 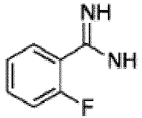 | Cl |
| 16 | 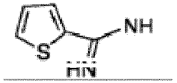 | Cl |
| 17 | 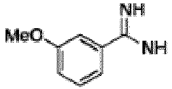 | Cl |
| 18 | 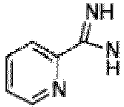 | Cl |
| 19 | 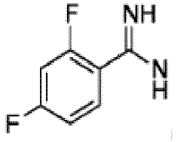 | Cl |
| 20 | 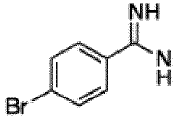 | Cl |
| 21 | 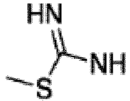 | Cl |
| 22 | 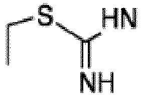 | Cl |
| 23 | 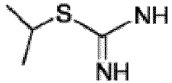 | Cl |
| 24 | 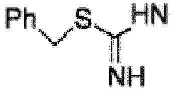 | Cl |
| 25 | 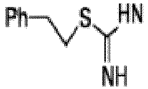 | Cl |
| 26 | 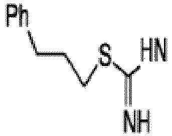 | Cl |
| 27 | 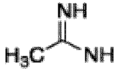 | F |
| 28 | 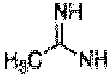 | Br |
| 29 | 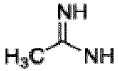 | I |
| 30 | 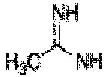 | H |
| 31 | 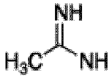 | CH₃ |
| 32 | 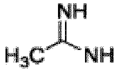 | OCH₃ |
| 33 | 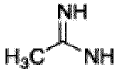 | (C₄H₈)- |
| 34 | 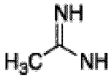 | CF₃ |
| 35 | 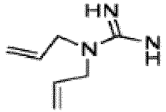 | Cl |
| 36 | 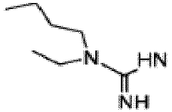 | Cl |
| 37 | 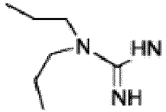 | Cl |
| 38 | 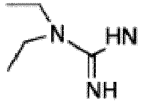 | Cl |
| 39 | 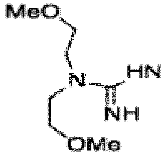 | Cl |
| 40 | 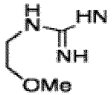 | Cl |
| 41 | 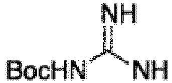 | Cl |
| 42 | 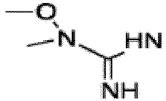 | Cl |
| 43 | 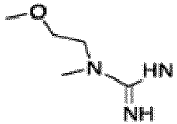 | Cl |
| 44 | 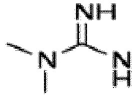 | Cl |
| 45 | 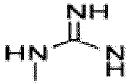 | Cl |
| 46 | 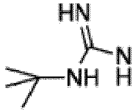 | Cl |
| 47 | 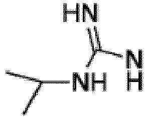 | Cl |
| 48 | 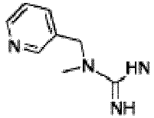 | Cl |
| 49 | 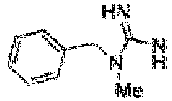 | Cl |
| 50 | 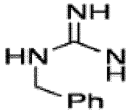 | Cl |
| 51 | 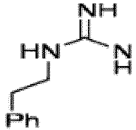 | Cl |
| 52 | 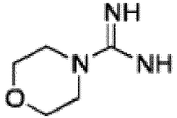 | Cl |
| 53 | 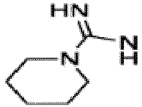 | Cl |
| 54 | 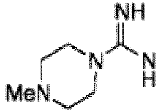 | Cl |
| 55 | 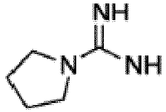 | Cl |
| 56 | 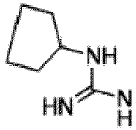 | Cl |
| 57 | 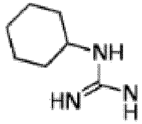 | Cl |
| 58 | 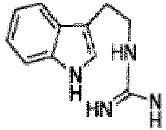 | Cl |
| 59 | 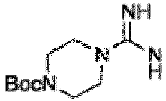 | Cl |
| 60 | 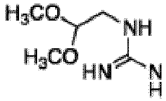 | Cl |
| 61 | 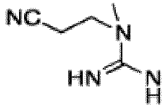 | Cl |
| 62 | 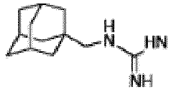 | Cl |
| 63 | 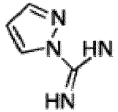 | Cl |
| 64 | 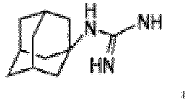 | Cl |
| 66 | 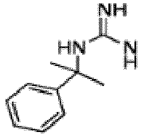 | Cl |
| 67 | 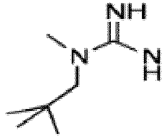 | Cl |
| 68 | 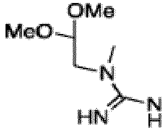 | Cl |
| 69 | 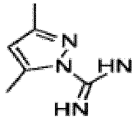 | Cl |
| 70 | 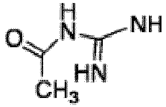 | I |
| 71 | 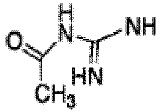 | C₄H₈ |
| 73 | NH₂NH | Cl |

13. The compound for the use of any one of claims 1 to 12, wherein the pulmonary fibrosis is induced by Hermansky-Pudlak Syndrome.

14. The compound for the use any one of claims 1 to 12, wherein the pulmonary fibrosis is idiopathic pulmonary fibrosis.

15. The compound for the use of any one of claims 1 to 14, wherein the pulmonary fibrosis is associated with alveolar inflammation.

## Patentansprüche

1. Verbindung oder Tautomer davon oder pharmazeutisch annehmbares Salz oder pharmazeutisch annehmbarer Ester davon zur Verwendung bei der Behandlung von Lungenfibrose bei einem Subjekt, wobei die Verbindung von der folgenden Formel ist: 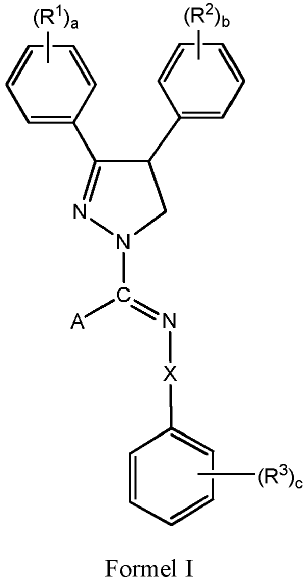 wobei
X SO₂ ist;
b null ist;
a und c jeweils unabhängig 0, 1, 2, 3, 4 oder 5 sind;
R¹ und R³ jeweils unabhängig ausgewählt sind aus C₁-C₆-Alkyl, substituiertem C₁-C₆-Alkyl, Halogen, C₁-C₈-Alkoxy und substituiertem C₁-C₈-Alkoxy;
A eine amidinohaltige Einheit mit der folgenden Struktur ist: 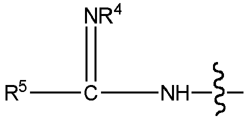
wobei
R⁴ H ist;
R⁵ C₁-C₆-Alkyl, C₃-C₆-Cycloalkyl, -NH₂, -N(C₁-C₆-Alkyl)₂, -NH(C₁-C₆-Alkyl), Phenyl, ein monocyclisches oder bicyclisches Heteroaryl, das zumindest ein Stickstoffheteroatom beinhaltet, oder ein monocyclisches oder bicyclisches Heterocycloalkyl, das zumindest ein Stickstoffheteroatom beinhaltet, ist; oder
R⁵ C₁-C₆-Alkyl, -N(C₁-C₆-Alkyl)₂, -NH(C₁-C₆-Alkyl) oder-NHC(O)CH₃ ist; oder
R⁵ ein Amino der Formel -NR³⁰R³¹ ist, wobei R³⁰ und R³¹ unabhängig Wasserstoff oder ein optional substituiertes C₁-C₆-Alkyl sind, vorausgesetzt, dass zumindest eines von R³⁰ und R³¹ optional substituiertes C₁-C₆-Alkyl ist; oder
R⁵ ein Thiol substituiert mit einem C₁-C₆-Alkyl oder einem substituierten C₁-C₆-Alkyl ist;
wobei substituiertes C₁-C₆-Alkyl ein C₁-C₆-Alkyl ist, wobei ein oder mehrere Wasserstoffatome mit Halogen, Cycloalkyl, C₁-C₈-Alkoxy, -NH₂, Hydroxyl, Aryl, Alkenyl mit bis zu 6 Kohlenstoffatomen oder Carboxyl substituiert sind; und
substituiertes C₁-C₈-Alkoxy C₁-C₈-Alkoxy substituiert mit Alkenyl mit bis zu 6 Kohlenstoffatomen, Alkinyl mit bis zu 6 Kohlenstoffatomen, Aryl, Aryl-C₁-C₆-alkyl, Cycloalkyl, halogeniertem C₁-C₆-Alkyl oder C₁-C₈-Alkoxy ist.

2. Verbindung zur Verwendung nach Anspruch 1, wobei R⁵ C₁-C₆-Alkyl, C₃-C₆-Cycloalkyl, -N(C₁-C₆-Alkyl)₂, -NH(C₁-C₆-Alkyl)), Phenyl, ein monocyclisches oder bicyclisches Heteroaryl, das zumindest ein Stickstoffheteroatom beinhaltet, oder ein monocyclisches oder bicyclisches Heterocycloalkyl, das zumindest ein Stickstoffheteroatom beinhaltet, ist.

3. Verbindung zur Verwendung nach Anspruch 1, wobei R⁵ ein Amino der Formel -NR³⁰R³¹ ist, wobei R³⁰ und R³¹ unabhängig Wasserstoff oder ein optional substituiertes C₁-C₆-Alkyl sind, vorausgesetzt, dass zumindest eines von R³⁰ und R³¹ optional substituiertes C₁-C₆-Alkyl ist.

4. Verbindung zur Verwendung nach Anspruch 1, wobei a und c jeweils eins sind, R¹ Halogen ist und R³ Halogen ist.

5. Verbindung zur Verwendung nach Anspruch 1, wobei die Verbindung von der folgenden Formel ist: 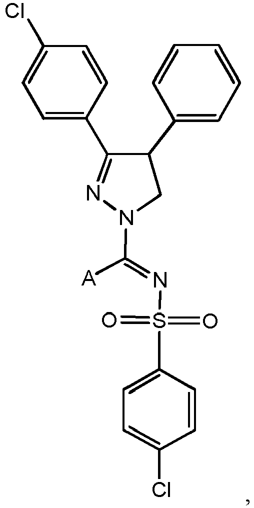 oder Tautomer davon oder pharmazeutisch annehmbares Salz oder pharmazeutisch annehmbarer Ester davon.

6. Verbindung zur Verwendung nach Anspruch 1, wobei die Verbindung von der folgenden Formel ist: 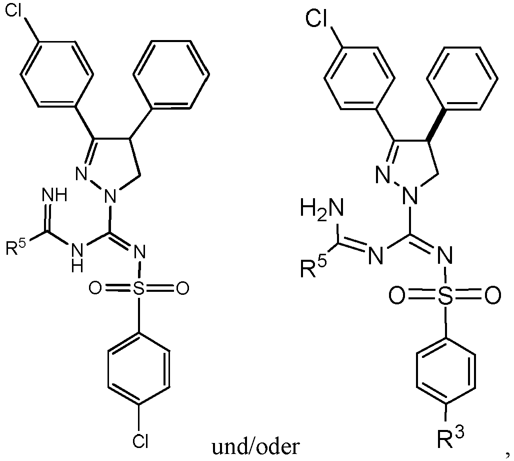 oder Tautomer davon oder pharmazeutisch annehmbares Salz oder pharmazeutisch annehmbarer Ester davon.

7. Verbindung zur Verwendung nach Anspruch 1, wobei die Verbindung von der folgenden Formel ist: 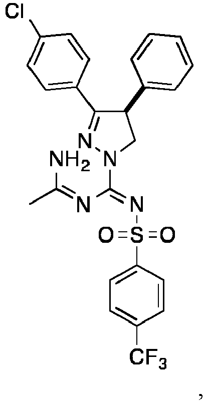 oder Tautomer davon oder pharmazeutisch annehmbares Salz davon.

8. Verbindung zur Verwendung nach Anspruch 1, wobei die Verbindung von der folgenden Formel ist: 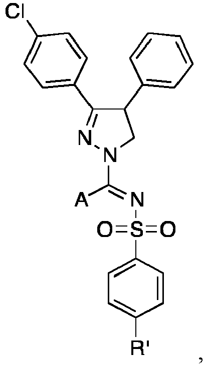 oder Tautomer davon oder pharmazeutisch annehmbares Salz davon,
wobei A 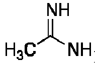 ist und R' Cl ist.

9. Verbindung zur Verwendung nach Anspruch 1, wobei die Verbindung von der folgenden Formel ist: 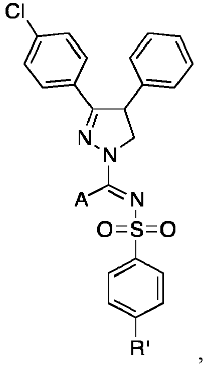 oder Tautomer davon oder pharmazeutisch annehmbares Salz davon,
wobei A 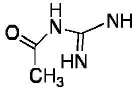 ist und R' Cl ist.

10. Verbindung zur Verwendung nach Anspruch 1, wobei die Verbindung von der folgenden Formel ist: 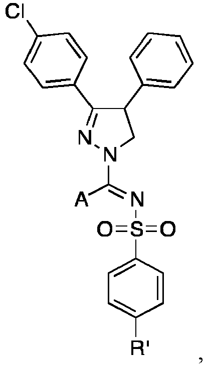 oder Tautomer davon oder pharmazeutisch annehmbares Salz davon,
wobei A 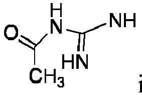 ist und R' CF₃ ist.

11. Verbindung oder Tautomer davon oder pharmazeutisch annehmbares Salz davon zur Verwendung bei der Behandlung von Lungenfibrose bei einem Subjekt, wobei die Verbindung von der folgenden Formel ist: 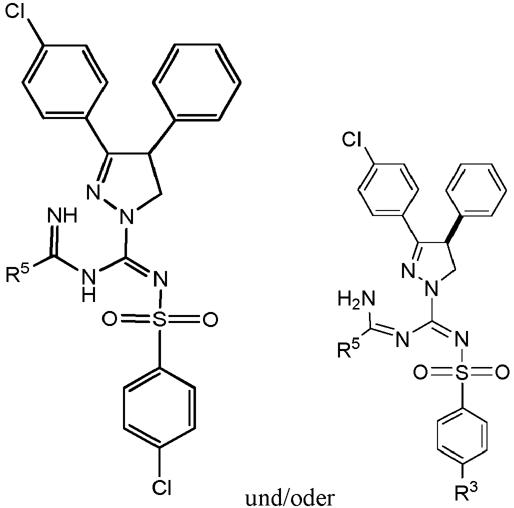
wobei R⁵ Methyl, -S-Methyl, -NHC(O)CH₃, NH(Boc) oder NH₂ ist; und
R³ CF₃ oder Cl ist.

12. Verbindung oder Tautomer davon oder pharmazeutisch annehmbares Salz davon zur Verwendung bei der Behandlung von Lungenfibrose bei einem Subjekt, wobei die Verbindung von der folgenden Formel ist: 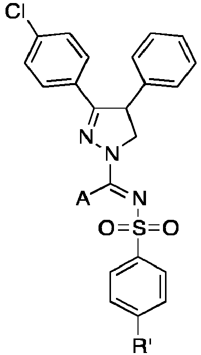 wobei die Verbindung eine A- und R'-Gruppe wie in der Tabelle unten angegeben aufweist:
| Serien-# | A | R' |
|---|---|---|
| 1 | 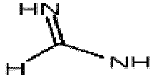 | Cl |
| 2E1 | 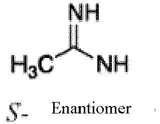 | Cl |
| 2E2 | 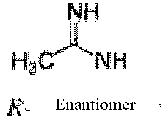 | Cl |
| 3 | 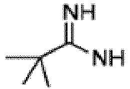 | Cl |
| 3E1 | 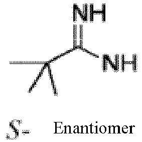 | Cl |
| 4 | 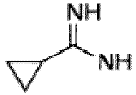 | Cl |
| 5 | 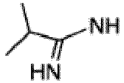 | Cl |
| 6 | 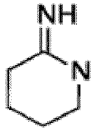 | Cl |
| 7 | 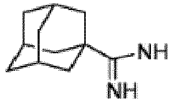 | Cl |
| 8 | 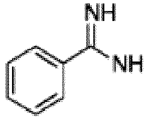 | Cl |
| 9 | 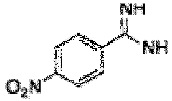 | Cl |
| 10 | 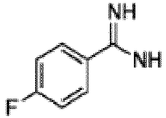 | Cl |
| 10E1 | 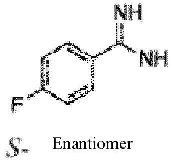 | Cl |
| 10E2 | 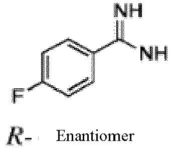 | Cl |
| 11 | 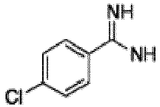 | Cl |
| 11E1 | 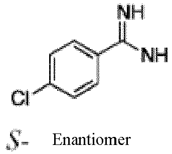 | Cl |
| 11E2 | 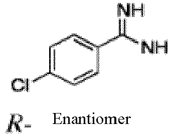 | Cl |
| 12 | 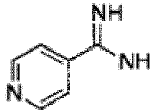 | Cl |
| 13 | 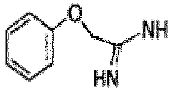 | Cl |
| 14 | 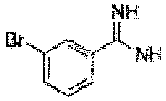 | Cl |
| 15 | 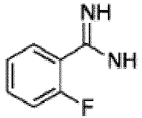 | Cl |
| 16 | 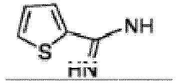 | Cl |
| 17 | 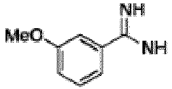 | Cl |
| 18 | 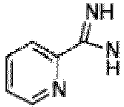 | Cl |
| 19 | 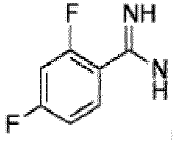 | Cl |
| 20 | 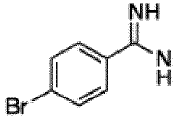 | Cl |
| 21 | 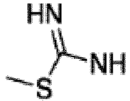 | Cl |
| 22 | 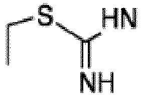 | Cl |
| 23 | 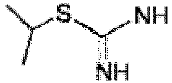 | Cl |
| 24 | 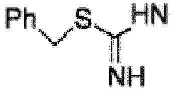 | Cl |
| 25 | 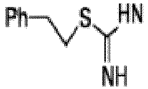 | Cl |
| 26 | 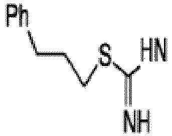 | Cl |
| 27 | 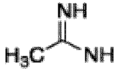 | F |
| 28 | 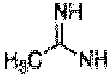 | Br |
| 29 | 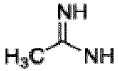 | 1 |
| 30 | 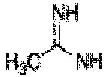 | H |
| 31 | 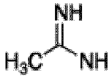 | CH₃ |
| 32 | 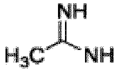 | OCH₃ |
| 33 | 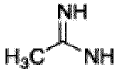 | (C₄H₈)- |
| 34 | 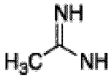 | CF₃ |
| 35 | 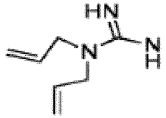 | Cl |
| 36 | 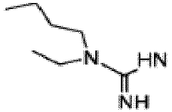 | Cl |
| 37 | 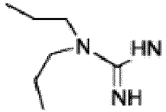 | Cl |
| 38 | 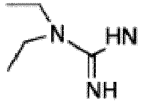 | Cl |
| 39 | 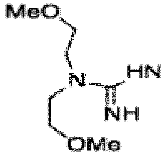 | Cl |
| 40 | 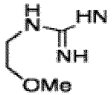 | Cl |
| 41 | 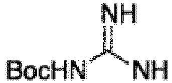 | Cl |
| 42 | 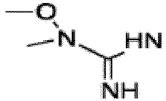 | Cl |
| 43 | 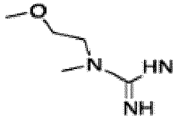 | Cl |
| 44 | 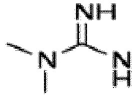 | Cl |
| 45 | 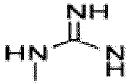 | Cl |
| 46 | 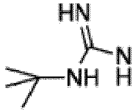 | Cl |
| 47 | 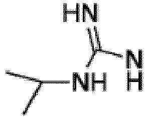 | Cl |
| 48 | 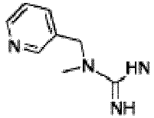 | Cl |
| 49 | 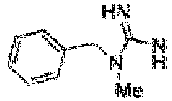 | Cl |
| 50 | 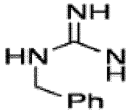 | Cl |
| 51 | 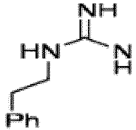 | Cl |
| 52 | 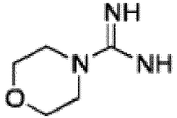 | Cl |
| 53 | 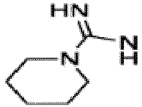 | Cl |
| 54 | 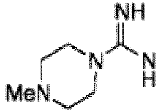 | Cl |
| 55 | 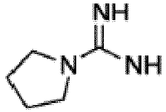 | Cl |
| 56 | 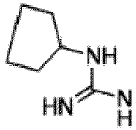 | Cl |
| 57 | 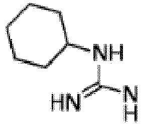 | Cl |
| 58 | 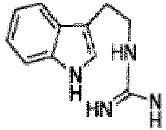 | Cl |
| 59 | 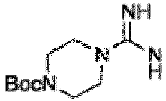 | Cl |
| 60 | 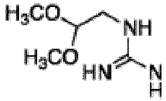 | Cl |
| 61 | 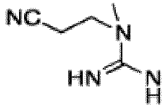 | Cl |
| 62 | 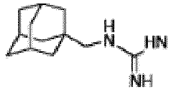 | Cl |
| 63 | 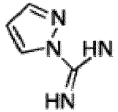 | Cl |
| 64 | 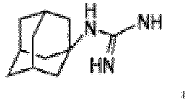 | Cl |
| 66 | 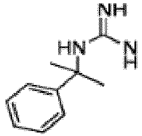 | Cl |
| 67 | 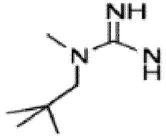 | Cl |
| 68 | 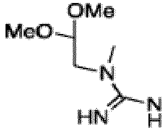 | Cl |
| 69 | 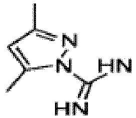 | Cl |
| 70 | 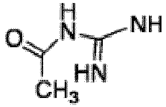 | 1 |
| 71 | 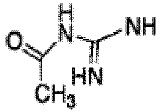 | C₄H₈ |
| 73 | NH₂NH | Cl |

13. Verbindung zur Verwendung nach einem der Ansprüche 1 bis 12, wobei die Lungenfibrose durch Hermansky-Pudlak-Syndrom induziert wird.

14. Verbindung zur Verwendung nach einem der Ansprüche 1 bis 12, wobei die Lungenfibrose idiopathische Lungenfibrose ist.

15. Verbindung zur Verwendung nach einem der Ansprüche 1 bis 14, wobei die Lungenfibrose mit alveolärer Entzündung assoziiert ist.

## Revendications

1. Composé, ou tautomère de celui-ci, ou sel pharmaceutiquement acceptable ou ester de celui-ci, destiné à être utilisé dans le traitement de la fibrose pulmonaire chez un sujet, ledit composé répondant à la formule : 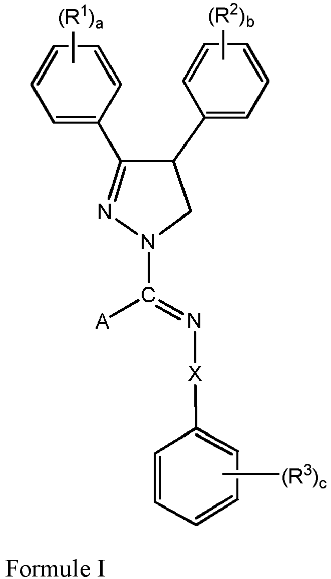 dans lequel
X est SO₂ ;
b est zéro ;
a et c sont chacun indépendamment 0, 1, 2, 3, 4 ou 5 ;
R¹ et R³ sont chacun indépendamment choisis parmi C₁-C₆ alkyle, C₁-C₆ alkyle substitué, halogène, C₁-C₈ alkoxy et C₁-C₈ alkoxy substitué ;
A est une fraction contenant un amidino comportant la structure : 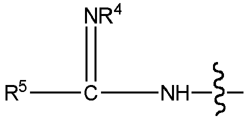
dans lequel
R⁴ est H ;
R⁵ est C₁-C₆ alkyle, C₃-C₆ cycloalkyle, -NH₂, -N(C₁-C₆ alkyl)₂, -NH(C₁-C₆ alkyl), phényle, un hétéroaryle monocyclique ou bicyclique qui comprend au moins un hétéroatome d'azote, ou un hétérocycloalkyle monocyclique ou bicyclique qui comprend au moins un hétéroatome d'azote ;
ou
R⁵ est C₁-C₆ alkyle, -N(C₁-C₆alkyl)₂, -NH(C₁-C₆ alkyl) ou -NHC(O)CH₃ ; ou
R⁵ est un amino de formule -NR³⁰R³¹, dans lequel R³⁰ et R³¹ sont, indépendamment, hydrogène ou un C₁-C₆ alkyle éventuellement substitué, à condition qu'au moins l'un de R³⁰ et R³¹ soit éventuellement C₁-C₆ alkyle substitué ; ou
R⁵ est un thiol substitué par un C₁-C₆ alkyle ou un C₁-C₆ alkyle substitué ;
dans lequel un C₁-C₆ alkyle substitué est un C₁-C₆ alkyle dans lequel au moins un atome d'hydrogène est substitué par halogène, cycloalkyle, C₁-C₈ alkoxy, -NH₂, hydroxyle, aryle, alcényle avec jusqu'à 6 atomes de carbone ou carboxyle ; et
C₁-C₈ alkoxy substitué est C₁-C₈ alkoxy substitué par alcényle avec jusqu'à 6 atomes de carbone, alkynyle avec jusqu'à 6 atomes de carbone, aryle, aryl-C₁-C₆ alkyle, cycloalkyle, C₁-C₆ alkyle halogéné ou C₁-C₈ alkoxy.

2. Composé destiné à être utilisé selon la revendication 1, dans lequel R⁵ est C₁-C₆ alkyle, C₃-C₆ cycloalkyle, -N(C₁-C₆ alkyl)₂, -NH(C₁-C₆ alkyl)), phényle, un hétéroaryle monocyclique ou bicyclique qui comprend au moins un hétéroatome d'azote, ou un hétérocycloalkyle monocyclique ou bicyclique qui comprend au moins un hétéroatome d'azote.

3. Composé destiné à être utilisé selon la revendication 1, dans lequel R⁵ est un amino de formule -NR³⁰R³¹, dans lequel R³⁰ et R³¹ sont, indépendamment, hydrogène ou un C₁-C₆ alkyle éventuellement substitué, à condition qu'au moins l'un de R³⁰ et R³¹ soit éventuellement C₁-C₆ alkyle substitué.

4. Composé destiné à être utilisé selon la revendication 1, dans lequel a et c sont chacun un, R¹ est halogène et R³ est halogène.

5. Composé destiné à être utilisé selon la revendication 1, ledit composé répondant à la formule : 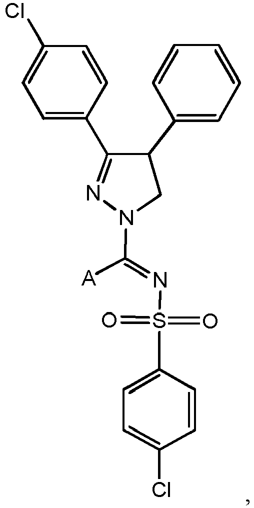 ou tautomère de celui, ou sel pharmaceutiquement acceptable ou ester de celui-ci.

6. Composé destiné à être utilisé selon la revendication 1, ledit composé répondant à la formule : 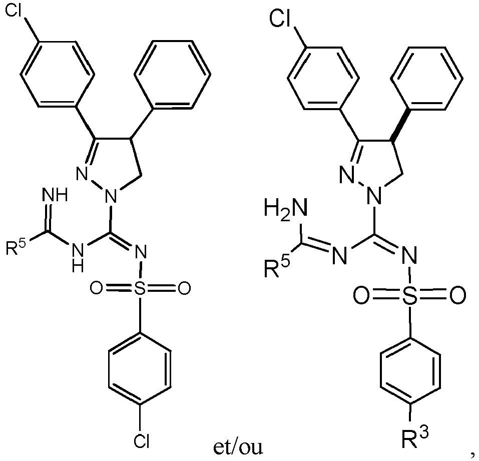 ou tautomère de celui, ou sel pharmaceutiquement acceptable ou ester de celui-ci.

7. Composé destiné à être utilisé selon la revendication 1, ledit composé répondant à la formule : 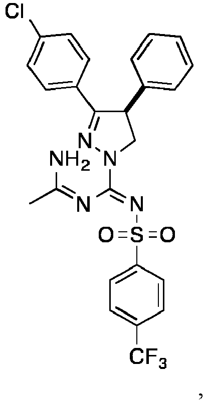 ou tautomère de celui, ou sel pharmaceutiquement acceptable de celui-ci.

8. Composé destiné à être utilisé selon la revendication 1, ledit composé répondant à la formule : 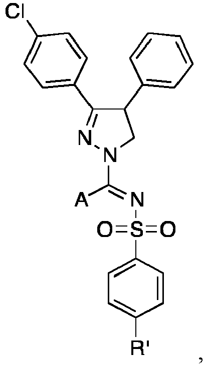 ou tautomère de celui, ou sel pharmaceutiquement acceptable de celui-ci,
dans lequel A est 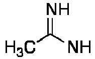 et R' est Cl.

9. Composé destiné à être utilisé selon la revendication 1, ledit composé répondant à la formule : 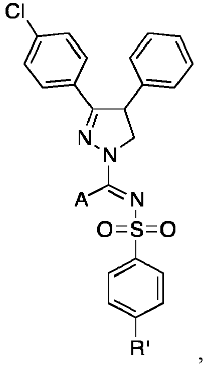 ou tautomère de celui, ou sel pharmaceutiquement acceptable de celui-ci,
dans lequel A est 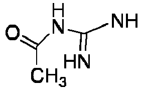 et R' est Cl.

10. Composé destiné à être utilisé selon la revendication 1, ledit composé répondant à la formule : 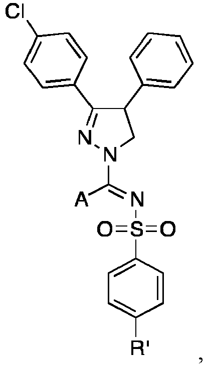 ou tautomère de celui, ou sel pharmaceutiquement acceptable de celui-ci,
dans lequel A est 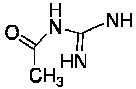 et R' est CF₃.

11. Composé, ou tautomère de celui-ci, ou sel pharmaceutiquement acceptable de celui-ci, destiné à être utilisé dans le traitement de la fibrose pulmonaire chez un sujet, ledit composé répondant à la formule : 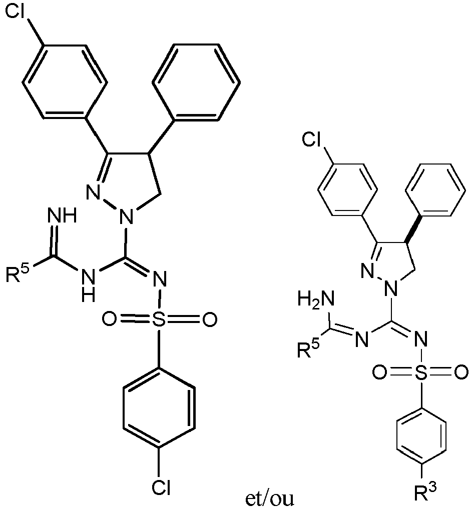
dans lequel R⁵ est méthyle, -S-méthyle, -NHC(O)CH₃, NH(Boc) ou NH₂ ; et
R³ est CF₃ ou Cl.

12. Composé, ou tautomère de celui-ci, ou sel pharmaceutiquement acceptable de celui-ci, destiné à être utilisé dans le traitement de la fibrose pulmonaire chez un sujet, ledit composé répondant à la formule : 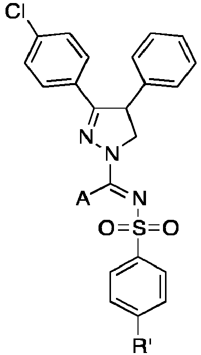 dans lequel le composé comporte un groupe A et R' comme indiqué dans le tableau ci-dessous :
| Serial# | A | R' |
|---|---|---|
| 1 | 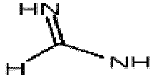 | Cl |
| 2E1 | 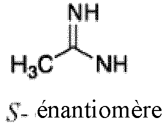 | Cl |
| 2E2 | 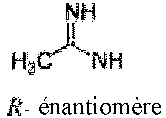 | Cl |
| 3 | 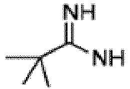 | Cl |
| 3E1 | 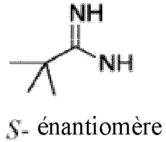 | Cl |
| 4 | 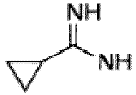 | Cl |
| 5 | 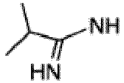 | Cl |
| 6 | 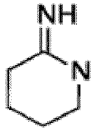 | Cl |
| 7 | 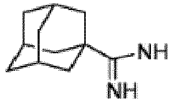 | Cl |
| 8 | 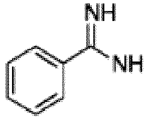 | Cl |
| 9 | 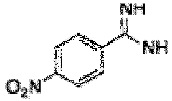 | Cl |
| 10 | 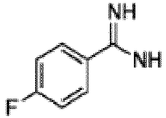 | Cl |
| 10E1 | 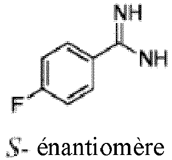 | Cl |
| 10E2 | 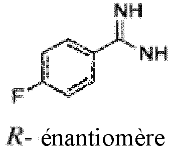 | Cl |
| 11 | 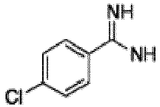 | Cl |
| 11E1 | 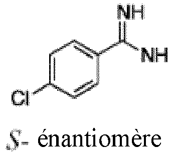 | Cl |
| 11E2 | 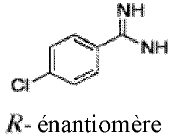 | Cl |
| 12 | 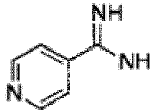 | Cl |
| 13 | 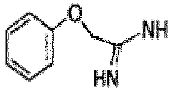 | Cl |
| 14 | 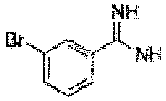 | Cl |
| 15 | 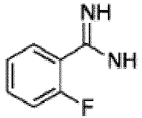 | Cl |
| 16 | 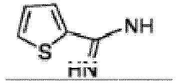 | Cl |
| 17 | 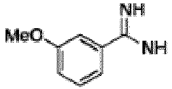 | Cl |
| 18 | 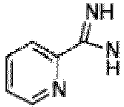 | Cl |
| 19 | 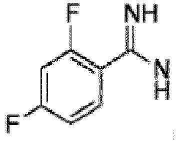 | Cl |
| 20 | 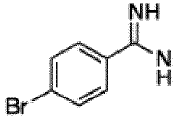 | Cl |
| 21 | 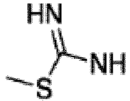 | Cl |
| 22 | 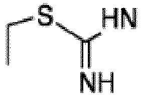 | Cl |
| 23 | 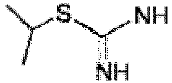 | Cl |
| 24 | 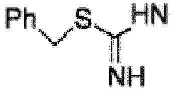 | Cl |
| 25 | 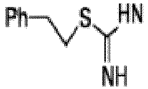 | Cl |
| 26 | 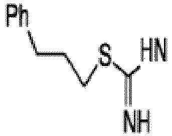 | Cl |
| 27 | 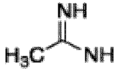 | F |
| 28 | 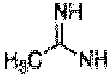 | Br |
| 29 | 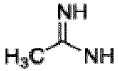 | 1 |
| 30 | 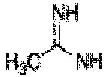 | H |
| 31 | 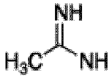 | CH₃ |
| 32 | 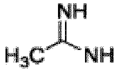 | OCH₃ |
| 33 | 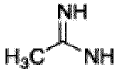 | (C₄H₈)- |
| 34 | 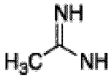 | CF₃ |
| 35 | 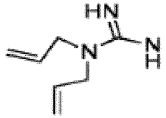 | Cl |
| 36 | 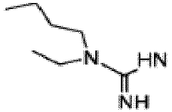 | Cl |
| 37 | 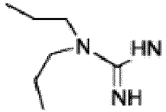 | Cl |
| 38 | 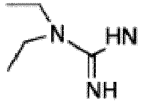 | Cl |
| 39 | 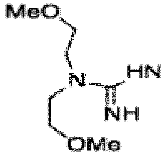 | Cl |
| 40 | 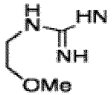 | Cl |
| 41 | 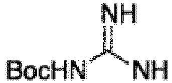 | Cl |
| 42 | 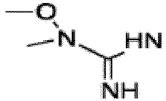 | Cl |
| 43 | 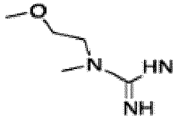 | Cl |
| 44 | 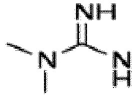 | Cl |
| 45 | 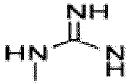 | Cl |
| 46 | 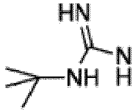 | Cl |
| 47 | 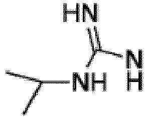 | Cl |
| 48 | 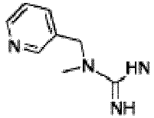 | Cl |
| 49 | 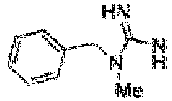 | Cl |
| 50 | 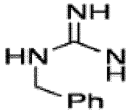 | Cl |
| 51 | 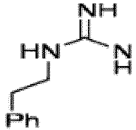 | Cl |
| 52 | 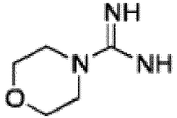 | Cl |
| 53 | 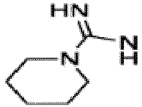 | Cl |
| 54 | 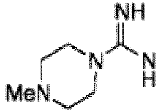 | Cl |
| 55 | 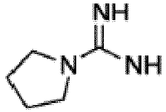 | Cl |
| 56 | 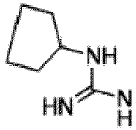 | Cl |
| 57 | 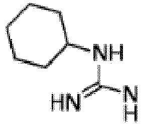 | Cl |
| 58 | 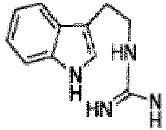 | Cl |
| 59 | 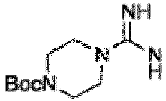 | Cl |
| 60 | 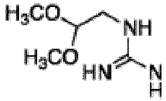 | Cl |
| 61 | 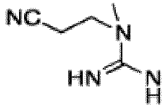 | Cl |
| 62 | 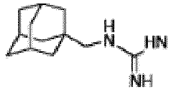 | Cl |
| 63 | 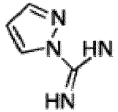 | Cl |
| 64 | 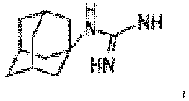 | Cl |
| 66 | 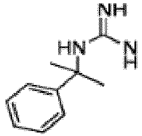 | Cl |
| 67 | 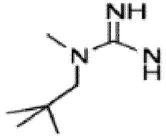 | Cl |
| 68 | 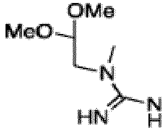 | Cl |
| 69 | 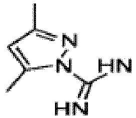 | Cl |
| 70 | 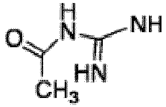 | 1 |
| 71 | 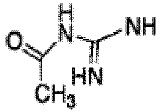 | C₄H₈ |
| 73 | NH₂NH | Cl |

13. Composé destiné à être utilisé selon l'une quelconque des revendications 1 à 12, dans lequel la fibrose pulmonaire est induite par le syndrome Hermansky-Pudlak.

14. Composé destiné à être utilisé selon l'une quelconque des revendications 1 à 12, dans lequel la fibrose pulmonaire est la fibrose pulmonaire idiopathique.

15. Composé destiné à être utilisé selon l'une quelconque des revendications 1 à 14, dans lequel la fibrose pulmonaire est associée à une inflammation alvéolaire.
